# EUROPÄISCHE PATENTSCHRIFT

(11) **EP 3 257 093 B1**
(45) Veröffentlichungstag und Bekanntmachung des Hinweises auf die Patenterteilung: **07.07.2021**
(21) Anmeldenummer: 16700144.5
(22) Anmeldetag: 11.01.2016
(51) Int. Cl.: H01L 51/54, C07D 403/10

(54) **ELEKTRONISCHE VORRICHTUNG ENTHALTEND CYCLISCHE LACTAME**
ELECTRONIC DEVICE CONTAINING CYCLIC LACTAMS
DISPOSITIF ÉLECTRONIQUE CONTENANT DES LACTAMES CYCLIQUES

(30) Priorität: 09.02.2015 EP 15000375
(43) Veröffentlichungstag der Anmeldung: 20.12.2017
(73) Patentinhaber: Merck Patent GmbH, 64293 Darmstadt (DE)
(72) Erfinder: STOESSEL, Philipp, 60389 Frankfurt am Main (DE); PARHAM, Amir Hossain, 60486 Frankfurt am Main (DE); PFLUMM, Christof, 64291 Darmstadt (DE); JATSCH, Anja, 60489 Frankfurt am Main (DE); EBERLE, Thomas, 76829 Landau (DE); KROEBER, Jonas, Valentin, 60311 Frankfurt am Main (DE)
(86) Internationale Anmeldenummer: PCT/EP2016/000034
(87) Internationale Veröffentlichungsnummer: WO 2016/128103

(56) Entgegenhaltungen:
- WO-A1-2013/071217
- WO-A1-2014/056567
- MAJUMDER ARPI ET AL: "Air-stable palladium(0) phosphine sulfide catalysts for Ullmann-type C-N and C-O coupling r", JOURNAL OF ORGANOMETALLIC CHEMISTRY, Bd. 781, 14. Januar 2015 (2015-01-14), Seiten 23-34, XP029200459, ISSN: 0022-328X, DOI: 10.1016/J.JORGANCHEM.2014.11.018
- KUETHE J T ET AL: "Synthesis of disubstituted imidazo[4,5-b]pyridin-2-ones", THE JOURNAL OF ORGANIC CHEMISTRY, AMERICAN CHEMICAL SOCIETY, US, Bd. 69, Nr. 22, 1. Januar 2004 (2004-01-01), Seiten 7752-7754, XP002605487, ISSN: 0022-3263, DOI: 10.1021/JO048887V [gefunden am 2004-09-25]

## Beschreibung

Die vorliegende Erfindung betrifft elektronische Vorrichtungen enthaltend spezielle cyclische Lactame, insbesondere organische Elektrolumineszenzvorrichtungen, sowie spezielle cyclische Lactame für die Verwendung in elektronischen Vorrichtungen, insbesondere in organischen Elektrolumineszenzvorrichtungen.

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Als emittierende Materialien werden hierbei zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Quanten- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Dies gilt insbesondere auch für OLEDs, welche im kürzerwelligen Bereich, beispielsweise grün, emittieren.

Die Eigenschaften phosphoreszierender OLEDs werden nicht nur von den eingesetzten Triplettemittern bestimmt. Hier sind insbesondere auch die anderen verwendeten Materialien, wie Matrixmaterialien, Lochblockiermaterialien, Elektronentransportmaterialien, etc. von besonderer Bedeutung. Verbesserungen dieser Materialien können somit auch zu deutlichen Verbesserungen der OLED-Eigenschaften führen. Auch für fluoreszierende OLEDs gibt es bei diesen Materialien noch Verbesserungsbedarf.

Gemäß dem Stand der Technik werden unter anderem Lactame, z. B. gemäß WO 2011/116865, WO 2011/137951 oder WO 2014/056567, als Matrixmaterialien für phosphoreszierende Emitter in organischen Elektrolumineszenzvorrichtungen eingesetzt. Generell sind hier weitere Verbesserungen wünschenswert, insbesondere in Bezug auf die Effizienz, die Lebensdauer und die thermische Stabilität der Materialien.

Dihydropteridinon-Verbindungen sind aus der WO 2013/071217 A1 bekannt. Pyrido[2,3-6]pyrazinedion-Verbindungen wurden bereits von KUETHE J T et al beschrieben: "Synthesis of disubstituted imidazo[4,5-b]pyridin-2-ones",THE JOURNAL OF ORGANIC CHEMISTRY, Bd. 69, Nr. 22, (2004-01-01), Seiten 7752-7754.

Aufgabe der vorliegenden Erfindung ist die Bereitstellung von elektronischen Vorrichtungen enthaltend Verbindungen, welche sich für den Einsatz als Matrixmaterial oder als Elektronentransport- bzw. Lochblockiermaterial eignen. Insbesondere ist es die Aufgabe der vorliegenden Erfindung, grün, gelb und rot und gegebenenfalls auch blau phosphoreszierende OLEDs bereitzustellen bzw. spezielle Matrixmaterialien bereitzustellen, welche sich für grün und rot und gegebenenfalls auch blau phosphoreszierende OLEDs eignen.

Überraschend wurde gefunden, dass die unten näher beschriebenen Verbindungen der Formel (1) diese Aufgabe lösen und zu Verbesserungen der organischen Elektrolumineszenzvorrichtung führen, insbesondere hinsichtlich der Lebensdauer, der Effizienz und/oder der Betriebsspannung. Dies gilt insbesondere für rot und grün phosphoreszierende Elektrolumineszenzvorrichtungen, vor allem bei Einsatz der Verbindungen der Formel (1) oder der erfindungsgemäßen Verbindungen als Matrixmaterial. Die Materialien zeichnen sich weiterhin durch eine hohe Temperaturstabilität aus. Elektronische Vorrichtungen, insbesondere organische Elektrolumineszenzvorrichtungen, welche derartige Verbindungen enthalten, sind daher der Gegenstand der vorliegenden Erfindung.

Gegenstand der vorliegenden Erfindung ist eine elektronische Vorrichtung enthaltend mindestens eine Verbindung der Formel (1), 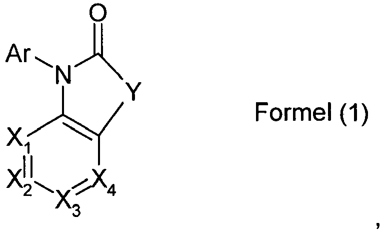 oder mindestens zwei Verbindungen der Formel (1), die über mindestens ein gemeinsames aromatisches oder heteroaromatisches Ringsystem Ar verbunden sind oder mindestens zwei Verbindungen der Formel (1), die eine gemeinsame Struktureinheit 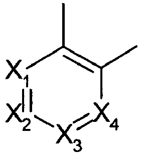 haben,
wobei für die verwendeten Symbole gilt:
X₁, X₂, X₃, X₄ sind jeweils unabhängig voneinander CR oder N;
Y ist bei jedem Auftreten 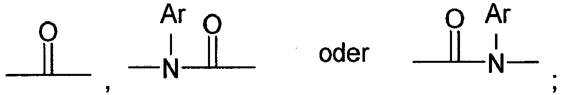
Ar ist bei jedem Auftreten gleich oder verschieden ein aromatisches oder heteroaromatisches Ringsystem mit 5-60 aromatischen Ringatomen, das mit einem oder mehreren Resten R¹ substituiert sein kann;
Ar¹ ist bei jedem Auftreten gleich oder verschieden ein aromatisches oder heteroaromatisches Ringsystem mit 5-30 aromatischen Ringatomen, das mit einem oder mehreren Resten R¹ substituiert sein kann; dabei können zwei Reste Ar¹, welche an dasselbe N-Atom oder P-Atom binden, auch durch eine Einfachbindung oder eine Brücke, ausgewählt aus N(R¹), C(R¹)₂ oder O, miteinander verbrückt sein;
R ist bei jedem Auftreten gleich oder verschieden ausgewählt aus der Gruppe bestehend aus H, D, F, Cl, Br, I, CN, CHO, NO₂, Si(R²)₃, B(OR²)₂, N(Ar¹)₂, N(R¹)₂, C(=O)Ar¹, C(=O)R¹, P(=O)(Ar¹)₂, S(=O)Ar¹, S(=O)(Ar¹)₂, CR²=CR²Ar¹, C≡CAr¹, OSO₂R¹;
einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, wobei die genannten Kohlenwasserstoffgruppen jeweils mit einem oder mehreren Resten R¹ substituiert sein können und wobei eine oder mehrere nicht-benachbarte CH₂-Gruppen durch R¹C=CR¹, -C≡C-, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können;
einem aromatischen oder heteroaromatischen Ringsystem mit 5 bis 60 aromatischen Ringatomen, das jeweils mit einem oder mehreren Resten R¹ substituiert sein kann,
einer Aryloxy- oder Heteroaryloxygruppe mit 5 bis 60 aromatischen Ringatomen, die mit einem oder mehreren Resten R¹ substituiert sein kann, einer Aralkyl- oder Heteroaralkylgruppe mit 5 bis 60 aromatischen Ringatomen, die mit einem oder mehreren Resten R¹ substituiert sein kann, oder einer Kombination dieser Systeme,
wobei zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann oder
wobei der Substituent R von X₁ und/oder der Substituent R von X₄ zusammen mit dem jeweils benachbarten N-Ar ein monocyclisches oder polycyclisches, aliphatisches, aromatisches oder heteroaromatisches Ringsystem bilden können, das mit einem oder mehreren Resten R¹ substituiert sein kann;
R¹ ist jeweils unabhängig ausgewählt aus der Gruppe bestehend aus H, D, F, CN, einer geradkettigen oder verzweigten Alkylgruppe mit 1 bis 20 C-Atomen, einer geradkettigen oder verzweigten Alkenylgruppe mit 2 bis 20 C-Atomen, einem aromatischem oder heteroaromatischem Ringsystem mit 5 bis 30 aromatischen Ringatomen, in dem ein oder mehrere H-Atome durch D, F, Cl, Br, I, CN oder eine geradkettige oder verzweigte Alkylgruppe mit 1 bis 10 C-Atomen oder eine geradkettige oder verzweigte Alkenylgruppe mit 2 bis 10 C-Atomen ersetzt sein können,
wobei zwei oder mehr benachbarte Substituenten R¹ miteinander ein mono- oder polycyclisches, aliphatisches, aromatisches oder heteroaromatisches Ringsystem bilden können und
R² ist jeweils unabhängig ausgewählt aus der Gruppe bestehend aus H, D oder ein aliphatischer, aromatischer und/oder heteroaromatischer Kohlenwasserstoffrest mit 1 bis 20 C-Atomen, wobei auch zwei oder mehrere Reste R² miteinander ein Ringsystem bilden können.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoffsensibilisierten Solarzellen (O-DSSC), Solarzellen enthaltend Perovskit, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs) und besonders bevorzugt phosphoreszierenden OLEDs.

Ein weiterer Gegenstand der Erfindung ist demzufolge die elektronische Vorrichtung enthaltend Verbindungen der Formel (1), wie zuvor und nachfolgend bevorzugt beschrieben, ausgewählt aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoffsensibilisierten Solarzellen, Solarzellen enthaltend Perovskit, organischen optischen Detektoren, organischen Photorezeptoren, organischen Feld-Quench-Devices, lichtemittierenden elektrochemischen Zellen, organischen Laserdioden und Organic Plasmon Emitting Devices.

Die elektronische Vorrichtung, bevorzugt die organische Elektrolumineszenzvorrichtung, enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Weiterhin lässt sich weiße Emission bevorzugt erzeugen durch Verwendung einer blauen Emissionsschicht und einer Emissionsschicht, die rot und grün emittiert, wobei diese beiden Emissionsschichten durch eine Ladungserzeugungsschicht voneinander separiert sein können.

Die Verbindung gemäß Formel (1), wie zuvor beschrieben oder bevorzugt nachfolgend beschrieben, kann dabei in unterschiedlichen Schichten der erfindungsgemäßen elektronischen Vorrichtung eingesetzt werden, je nach genauer Struktur. Bevorzugt ist eine organische Elektrolumineszenzvorrichtung, enthaltend mindestens eine Verbindung gemäß Formel (1) bzw. die nachfolgend ausgeführten bevorzugten Ausführungsformen oder der nachfolgend beschriebenen Verbindungen der Formeln (2), (2a), (3), (3a) bis (3j), (4), (4a), (5) und (5a), (6) bis (11), (6*) bis (11*), (12) bis (33), (34) bis (41) und (42 bis (57) als Matrixmaterial für fluoreszierende oder phosphoreszierende Emitter, insbesondere für phosphoreszierende Emitter, und/oder in einer Lochblockierschicht und/oder in einer Elektronentransportschicht und/oder in einer elektronenblockierenden bzw. exzitonenblockierenden Schicht und/oder in einer Lochtransportschicht, je nach genauer Substitution.

Ein weiterer Gegenstand der Erfindung ist die elektronische Vorrichtung dadurch gekennzeichnet, dass die Verbindung der Formel (1) bzw. die nachfolgend ausgeführten bevorzugten Ausführungsformen der Formel (1) oder der nachfolgend beschriebenen Verbindungen der Formeln (2), (2a), (3), (3a) bis (3j), (4), (4a), (5) und (5a), (6) bis (11), (6*) bis (11*), (12) bis (33), (34) bis (41) und (42 bis (57), als Matrixmaterial für einen fluoreszierenden oder phosphoreszierenden Emitter und/oder in einer Lochblockierschicht und/oder in einer Elektronentransportschicht und/oder in einer elektronenblockierenden bzw. exzitonenblockierenden Schicht und/oder in einer Lochtransportschicht eingesetzt wird, je nach genauer Substitution.

Ein weiterer Gegenstand der Erfindung ist die elektronische Vorrichtung dadurch gekennzeichnet, dass die Verbindung der Formel (1) bzw. die nachfolgend ausgeführten bevorzugten Ausführungsformen der Formel (1) oder der nachfolgend beschriebenen Verbindungen der Formeln (2), (2a), (3), (3a) bis (3j), (4), (4a), (5) und (5a), (6) bis (11), (6*) bis (11*), (12) bis (33), (34) bis (41) und (42 bis (57), als Matrixmaterial für einen fluoreszierenden oder phosphoreszierenden Emitter und/oder in einer Lochblockierschicht und/oder in einer Elektronentransportschicht eingesetzt wird.

Die erfindungsgemäßen organischen Elektrolumineszenzvorrichtungen und lichtemittierenden elektrochemischen Zellen können für verschiedene Anwendungen eingesetzt werden, beispielsweise für einfarbige oder mehrfarbige Displays, für Beleuchtungsanwendungen oder für medizinische und/oder kosmetische Anwendungen, beispielsweise in der Phototherapie.

Eine Arylgruppe im Sinne dieser Erfindung enthält 6 bis 60 C-Atome; eine Heteroarylgruppe im Sinne dieser Erfindung enthält 2 bis 60 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Phenyl, bzw. ein einfacher heteroaromatischer Cyclus, beispielsweise Pyridinyl, Pyrimidinyl, Thiophenyl, etc., oder eine kondensierte (anellierte) Aryl- oder Heteroarylgruppe, beispielsweise Naphthyl, Anthracenyl, Phenanthrenyl, Chinolinyl oder Isochinolinyl, verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 5-60 C-Atome im Ringsystem. Ein heteroaromatisches Ringsystem im Sinne dieser Erfindung enthält 3-60 C-Atome und mindestens ein Heteroatom im Ringsystem, mit der Maßgabe, dass die Summe aus C-Atomen und Heteroatomen mindestens 4 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein B-, Si-,C-, N- oder O-Atom, verbunden sein können. So sollen beispielsweise auch Systeme abgeleitet von Fluoren, 9,9'-Spirobifluoren, 9,9-Diarylfluoren, Triarylamin, Diarylether oder Stilben als aromatische Ringsysteme im Sinne dieser Erfindung verstanden werden, und ebenso Systeme, in denen zwei oder mehrere Arylgruppen beispielsweise durch eine kurze Alkylgruppe verbunden sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden.
Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.
Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten R¹ substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Benzanthracen, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombination dieser Systeme.
Ein bevorzugtes aromatisches oder heteroaromatisches Ringsystem mit 5 bis 24 aromatischen Ringatomen ist abgeleitet von Benzol, Naphthalin, Anthracen, Phenanthren, Biphenyl, Terphenyl, Quaterphenyl, Fluoren, Spirobifluoren, Pyridin, Pyrimidin, Thiophen, Furan, Pyrrol, Triazin, Carbazol, Benzofuran, Benzothiophen, Dibenzofuran, Dibenzothiophen, Chinolin, Isochinolin, Phenanthridin, Phenanthrolin, Azacarbazol, Imidazol, Benzimidazol, Indenocarbazol, Indolocarbazol, Triphenylamin oder Kombinationen aus zwei oder drei dieser Gruppen.

R¹ wird jeweils unabhängig ausgewählt aus der Gruppe bestehend aus H, D, F, CN, einer geradkettigen oder verzweigten Alkylgruppe mit 1 bis 20 C-Atomen, einer geradkettigen oder verzweigten Alkenylgruppe mit 2 bis 20 C-Atomen, einem aromatischem oder heteroaromatischem Ringsystem mit 5 bis 30 aromatischen Ringatomen, in dem ein oder mehrere H-Atome durch D, F, Cl, Br, I, CN oder eine geradkettige oder verzweigte Alkylgruppe mit 1 bis 10 C-Atomen oder eine geradkettige oder verzweigte Alkenylgruppe mit 2 bis 10 C-Atomen ersetzt sein können,
wobei zwei oder mehr benachbarte Substituenten R¹ miteinander ein mono- oder polycyclisches, aliphatisches, aromatisches oder heteroaromatisches Ringsystem bilden können.

R¹ bedeutet bevorzugt H, D, F, CN oder eine geradkettige oder verzweigte Alkylgruppe mit 1 bis 12 C-Atomen.

R¹ bedeutet besonders bevorzugt H, F, CN, eine geradkettige oder verzweigte Alkylgruppe mit 1 bis 4 C-Atomen.

R² wird jeweils unabhängig ausgewählt aus der Gruppe bestehend aus H, D oder ein aliphatischer, aromatischer und/oder heteroaromatischer Kohlenwasserstoffrest mit 1 bis 20 C-Atomen, wobei auch zwei oder mehrere Reste R² miteinander ein Ringsystem bilden können.

R² bedeutet bevorzugt H, D oder eine Alkylgruppe mit 1 bis 12 C-Atomen. R² bedeutet besonders bevorzugt H.

In einer bevorzugten Ausführungsform der erfindungsgemäßen elektronischen Vorrichtung sind Verbindungen der Formel (1) enthalten, in denen Y für 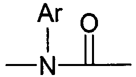 steht und Ar jeweils unabhängig voneinander eine zuvor genannte Bedeutung hat.

Ein weiterer Gegenstand der Erfindung ist demzufolge die erfindungsgemäße elektronische Vorrichtung, wie zuvor beschrieben oder als bevorzugt beschrieben, wobei Y in Formel (1) 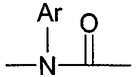 bedeutet und Ar jeweils unabhängig voneinander eine zuvor genannte Bedeutung hat.

In einer bevorzugten Ausführungsform der erfindungsgemäßen elektronischen Vorrichtung sind Verbindungen der Formel (1) enthalten, in denen Y für 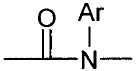 steht und Ar jeweils unabhängig voneinander eine zuvor genannte Bedeutung hat.

Ein weiterer Gegenstand der Erfindung ist demzufolge die erfindungsgemäße elektronische Vorrichtung, wie zuvor beschrieben oder als bevorzugt beschrieben, wobei Y in Formel (1) 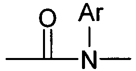 bedeutet und Ar jeweils unabhängig voneinander eine zuvor genannte Bedeutung hat.

In einer bevorzugten Ausführungsform der erfindungsgemäßen elektronischen Vorrichtung sind Verbindungen der Formel (1) enthalten, in denen Y für 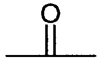 steht.

Ein weiterer Gegenstand der Erfindung ist demzufolge die erfindungsgemäße elektronische Vorrichtung, wie zuvor beschrieben oder als bevorzugt beschrieben, wobei Y in Formel (1) 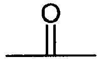 bedeutet.

In einer bevorzugten Ausführungsform der erfindungsgemäßen elektronischen Vorrichtung sind Verbindungen der Formel (1) enthalten, in denen mindestens eine Variable der Gruppe X₁, X₂, X₃ oder X₄ N bedeutet und die restlichen Variablen CR bedeuten und R jeweils unabhängig voneinander eine der in Formel (1) genannten Bedeutungen hat.

Ein weiterer Gegenstand der Erfindung ist demzufolge die erfindungsgemäße elektronische Vorrichtung, wie zuvor beschrieben oder als bevorzugt beschrieben, wobei in Formel (1) mindestens eine Variable der Gruppe X₁, X₂, X₃ oder X₄ N bedeutet und die restlichen Variablen CR bedeuten und R jeweils unabhängig voneinander eine der in Formel (1) genannten Bedeutungen hat.

In einer bevorzugten Ausführungsform der erfindungsgemäßen elektronischen Vorrichtung sind Verbindungen der Formel (1) enthalten, in denen die Variablen X₁, X₂, X₃ und X₄ CR bedeuten und R jeweils unabhängig voneinander eine der in Formel (1) genannten Bedeutungen hat.

Ein weiterer Gegenstand der Erfindung ist demzufolge die erfindungsgemäße elektronische Vorrichtung, wie zuvor beschrieben oder als bevorzugt beschrieben, wobei in Formel (1) die Variablen X₁, X₂, X₃ und X₄ CR bedeuten und R jeweils unabhängig voneinander eine der in Formel (1) genannten Bedeutungen hat.

Verbindungen der Formel (1), wie zuvor beschrieben oder bevorzugt beschrieben, können nach Syntheseverfahren hergestellt werden, die dem Fachmann auf dem Gebiet der organischen Synthese bekannt sind.

Ein weiterer Gegenstand, welcher nicht Teil dieser Erfindung ist, ist ein Verfahren zur Herstellung einer Verbindung gemäß Formel (1), wie zuvor beschrieben oder bevorzugt beschrieben, durch eine Kupplungsreaktion an der N-H-Gruppe von Verbindungen der Formel A, 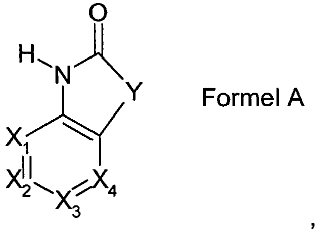 wobei X₁, X₂, X₃ und X₄ eine bei Formel (1) oder den nachfolgenden bevorzugten Ausführungsformen der Verbindungen der Formel (1) angegebene Bedeutung haben und Y bei jedem Auftreten 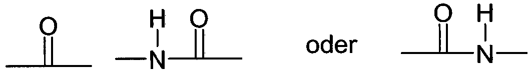 bedeutet;
mit Verbindungen der Formel B,

Ar-L Formel B,

wobei Ar eine in Formel (1) angegebene Bedeutung hat und L einer Abgangsgruppe entspricht, die für die Kupplungsreaktion geeignet ist.

Das Verfahren zur Herstellung gilt insbesondere für die Herstellung von Verbindungen der Formeln (42), (47) bis (51), (56) und (57), wobei die Bedeutungen von X₁, X₂, X₃ und X₄, sowie von Y und Ar diesen Verbindungen angepasst wird.

Es sind sehr viele Kupplungsreaktionen bekannt. Geeignet ist beispielsweise die Ullmann-Reaktion. Wird dieser Reaktionstyp gewählt, so ist L bevorzugt I oder Br. Eine Ullmann-Reaktion beschreibt allgemein eine Kupfer-katalysierte Kupplung eines Nucleophils mit einem Arylhalogenid. Geeignet ist beispielsweise die Hartwig-Buchwald-Reaktion. Wird dieser Reaktionstyp gewählt, so ist L bevorzugt Br. Eine Hartwig-Buchwald-Reaktion beschreibt allgemein eine Palladium-katalysierte Kupplung eines Nucleophils mit einem Arylhalogenid.

Weitere Verfahrensvarianten zur Herstellung von Verbindungen der Formel (1) werden nachfolgend bei den bevorzugten Ausführungsformen beschrieben.

Verbindungen der Formel (1), in denen Y für 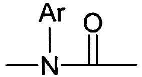 steht, können synonym durch die Formel (2) beschrieben werden, 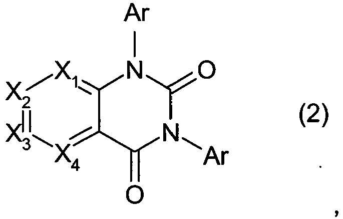 wobei X₁, X₂, X₃, X₄, Ar, R, R¹ und R² jeweils unabhängig voneinander eine zuvor genannte Bedeutung oder eine bevorzugt genannte Bedeutung haben.

In dieser Ausführungsform der erfindungsgemäßen elektronischen Vorrichtung, in der mindestens eine Verbindung der Formel (2) enthalten ist oder mindestens zwei Verbindungen der Formel (2) enthalten sind, die über mindestens ein gemeinsames aromatisches oder heteroaromatisches Ringsystem Ar verbunden sind oder mindestens zwei Verbindungen der Formel (2), die eine gemeinsame Struktureinheit 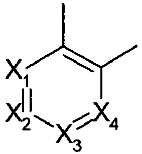 haben, wie zuvor beschrieben, ist es bevorzugt, wenn X₁, X₂, X₃ und X₄ CR bedeuten oder Teil der zweiten Verbindung der Formel (2) sind, wobei Ar, R, R¹ und R² jeweils unabhängig voneinander eine zuvor genannte Bedeutung oder eine nachfolgend angegebene bevorzugte Bedeutung haben.

Eine bevorzugte Ausführungsform der genannten Verbindungen der Formel (2) enthaltend in der erfindungsgemäßen elektronischen Vorrichtung entspricht der Formel (2a). Die Formel (2a) beschreibt demzufolge Verbindungen der Formel (2), wobei X₁, X₂, X₃ und X₄ CR bedeuten und wobei der Substituent R von X₁ zusammen mit dem jeweils benachbarten N-Ar ein monocyclisches oder polycyclisches, aliphatisches, aromatisches oder heteroaromatisches Ringsystem bildet, das mit einem oder mehreren Resten R¹ substituiert sein kann, 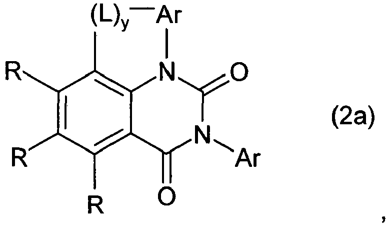
wobei y 0 oder 1 bedeutet,
L für C(=O) oder -O- steht und
wobei Ar, R, R¹ und R² jeweils unabhängig voneinander eine zuvor genannte Bedeutung oder eine nachfolgend angegebene bevorzugte Bedeutung haben.

Bevorzugte Ausführungsformen der genannten Verbindungen der Formel (2) in der erfindungsgemäßen Vorrichtung sind die Verbindungen der Formeln (3), (4) und (5), 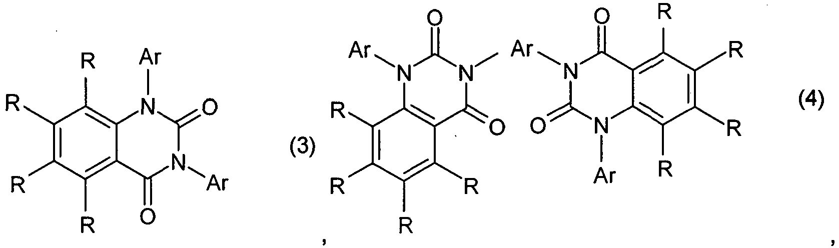 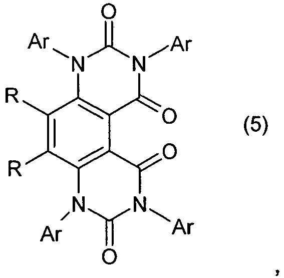 wobei Ar, R, R¹ und R² jeweils unabhängig voneinander eine zuvor genannte Bedeutung oder eine nachfolgend angegebene bevorzugte Bedeutung haben.

In Verbindungen der Formel (3) ist R jeweils unabhängig voneinander H oder ein aromatisches oder heteroaromatisches Ringsystem mit 5 bis 60 aromatischen Ringatomen, das jeweils mit einem oder mehreren Resten R¹ substituiert sein kann, wobei zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann oder wobei einer der Substituenten R, mit dem benachbarten N-Ar ein monocyclisches oder polycyclisches, aliphatisches, aromatisches oder heteroaromatisches Ringsystem bildet, das mit einem oder mehreren Resten R¹ substituiert sein kann. Derartige bevorzugte Verbindungen der Formel (3) können durch die Formeln (3a) bis (3j) beschrieben werden.

Die Formel (3a) beschreibt demzufolge Verbindungen der Formel (3), in denen alle Substituenten R für H stehen, 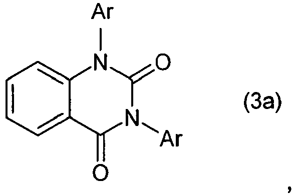 wobei Ar, R¹ und R² jeweils unabhängig voneinander eine zuvor genannte Bedeutung oder eine nachfolgend angegebene bevorzugte Bedeutung haben.

Die Formeln (3b), (3c), (3d), (3e) oder (3f) beschreiben demzufolge Verbindungen der Formel (3), in denen zwei oder drei Substituenten R für H stehen und zwei Substituenten oder ein Substituent R für ein aromatisches oder heteroaromatisches Ringsystem mit 5 bis 60 aromatischen Ringatomen stehen/steht, die/das jeweils mit einem oder mehreren Resten R¹ substituiert sein kann, 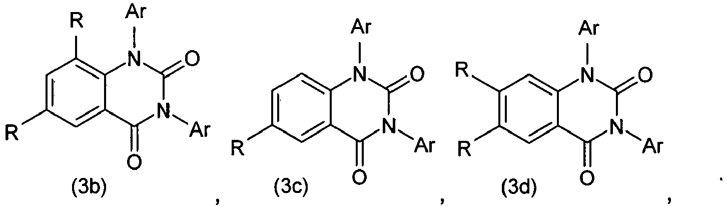 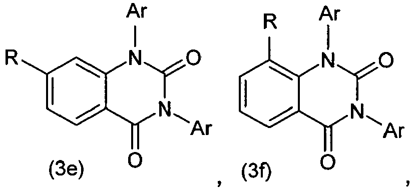 wobei Ar, R, R¹ und R² jeweils unabhängig voneinander eine zuvor genannte Bedeutung oder eine nachfolgend angegebene bevorzugte Bedeutung haben.

Die Formeln (3g), (3h), (3i) und (3j) beschreiben demzufolge Verbindungen der Formel (3), wobei zwei oder mehr benachbarte Substituenten R ein monocyclisches oder polycyclisches, aliphatisches, aromatisches oder heteroaromatisches Ringsystem bilden, welches durch das Symbol "(A" gekennzeichnet ist und die restlichen Substituenten R für H stehen, 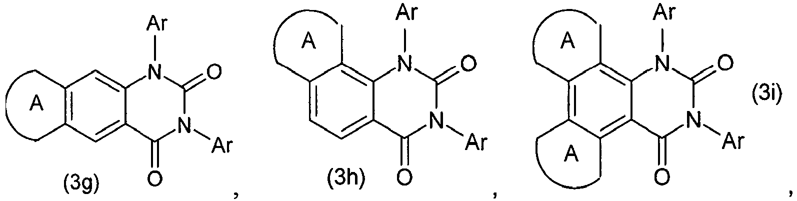 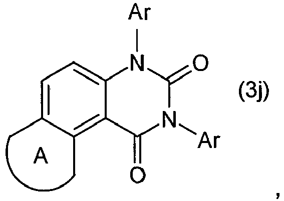 wobei Ar, R¹ und R² jeweils unabhängig voneinander eine zuvor genannte Bedeutung oder eine nachfolgend angegebene bevorzugte Bedeutung haben.

In Verbindungen der Formel (4) ist R bevorzugt H, entsprechend der Formel (4a), 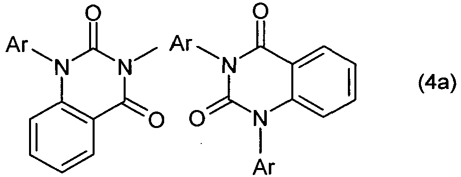 wobei Ar, R¹ und R² jeweils unabhängig voneinander eine zuvor genannte Bedeutung oder eine nachfolgend angegebene bevorzugte Bedeutung haben.

In Verbindungen der Formel (5) ist R bevorzugt H, entsprechend der Formel (5a), 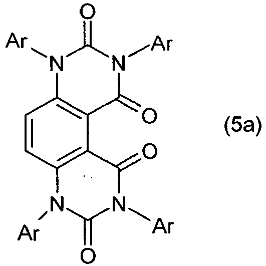 wobei Ar, R¹ und R² jeweils unabhängig voneinander eine zuvor genannte Bedeutung oder eine nachfolgend angegebene bevorzugte Bedeutung haben.

In den Verbindungen der Formeln (2), (2a), (3), (3a) bis (3j), (4), (4a), (5) und (5a) steht Ar jeweils unabhängig voneinander bevorzugt für ein aromatisches oder heteroaromatisches Ringsystem mit 5 bis 24 aromatischen Ringatomen, das mit einem oder mehreren Resten R¹ substituiert sein kann. Besonders bevorzugt ist Ar in dieser Ausführungsform der Erfindung durch einen bevorzugten Rest R¹ substituiert, wie zuvor beschrieben. Besonders bevorzugt ist in dieser Ausführungsform Ar unsubstituiert.
In den Verbindungen der Formeln (2), (2a), (3), (3a) bis (3j), (4), (4a), (5) und (5a) steht Ar jeweils unabhängig voneinander besonders bevorzugt für Phenyl, Naphthyl, Anthracenyl, Phenanthrenyl oder einen der Reste der Formeln (Ar-1) bis (Ar-145), 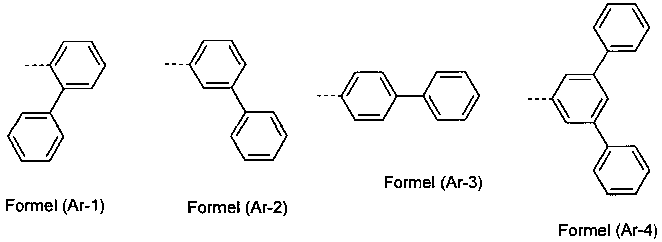 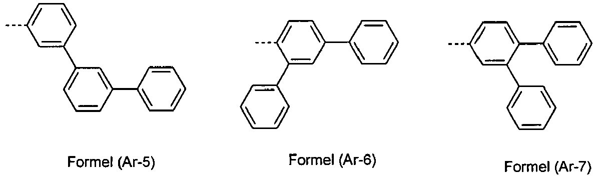 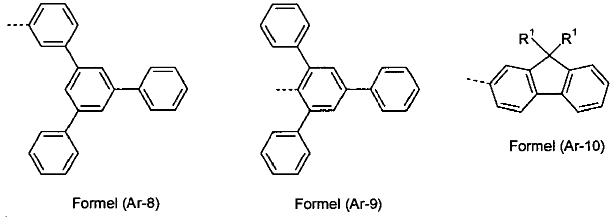 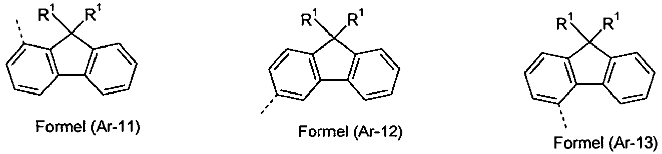 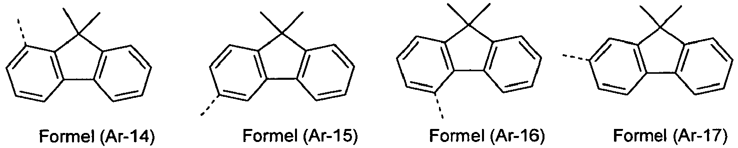 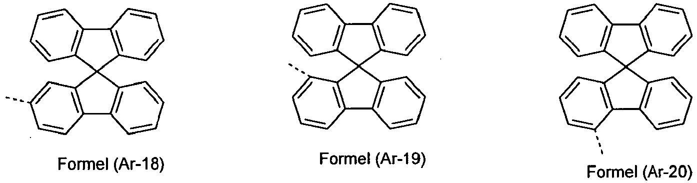 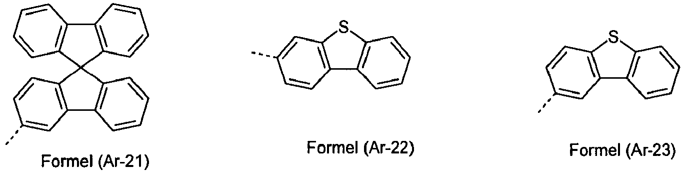 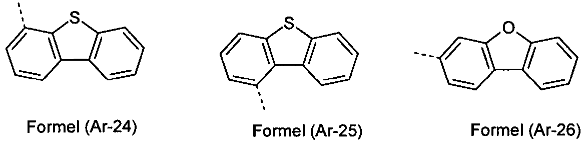 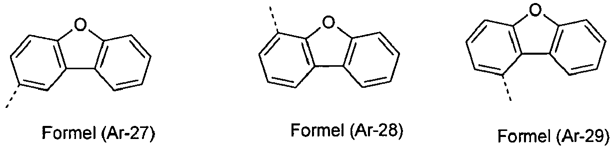 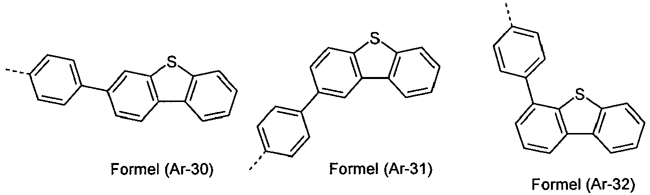 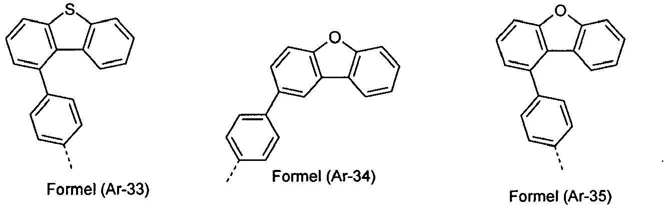 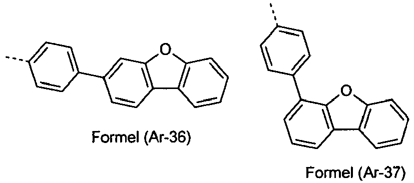 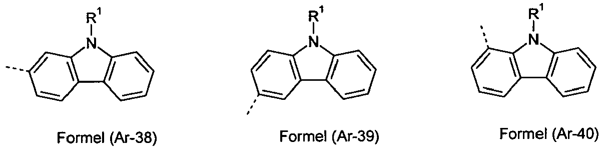 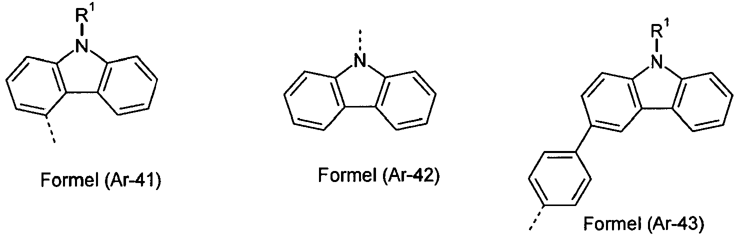 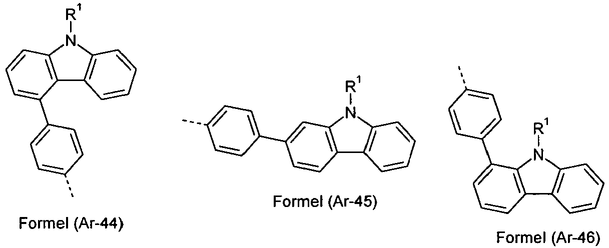 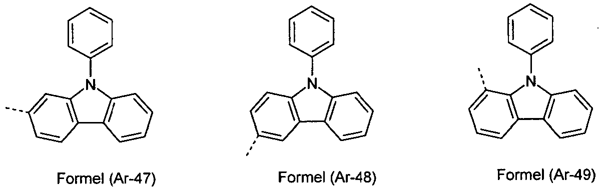 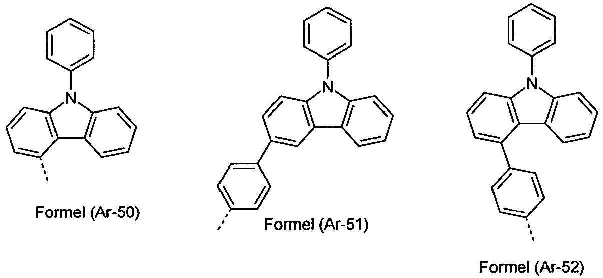 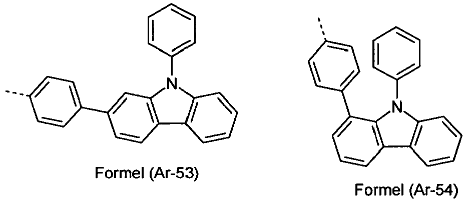 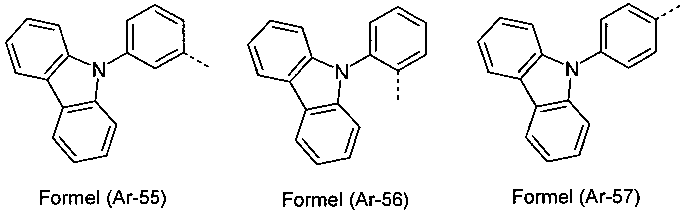 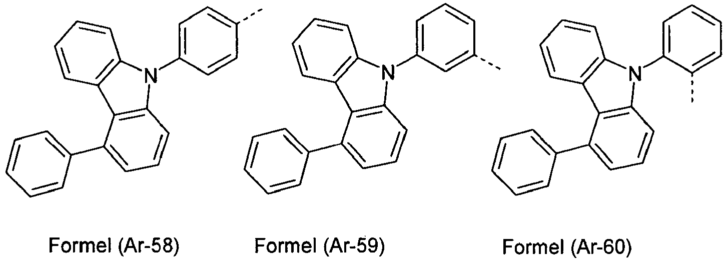 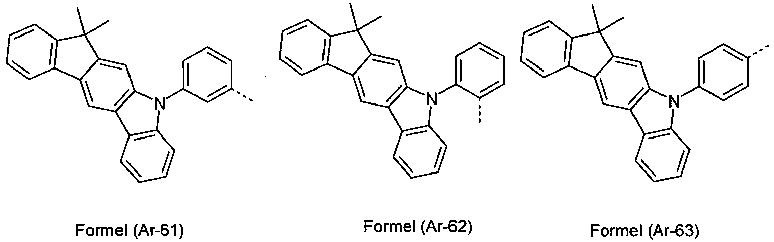 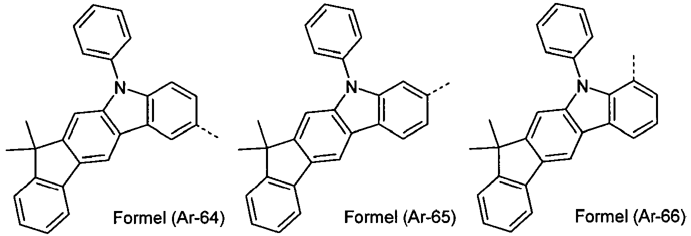 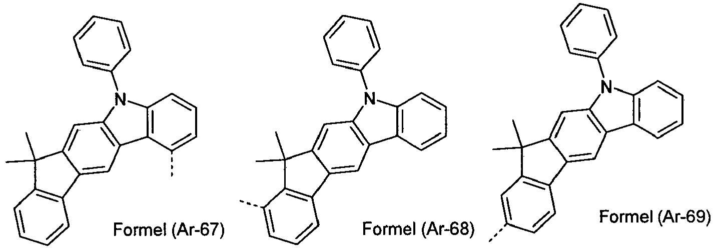 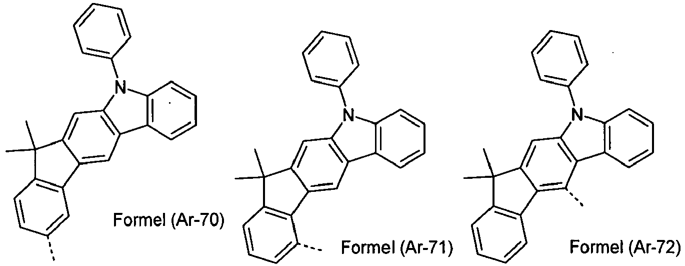 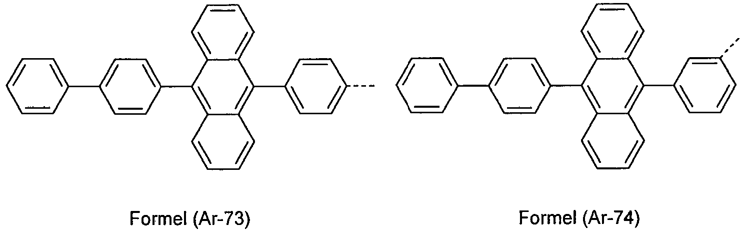 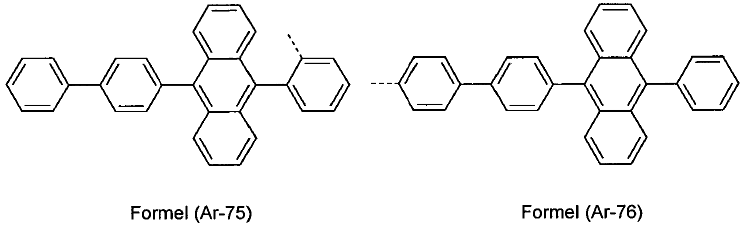 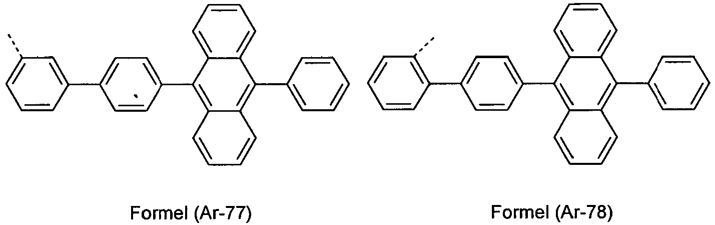 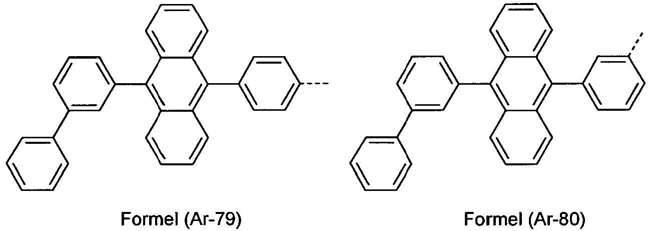 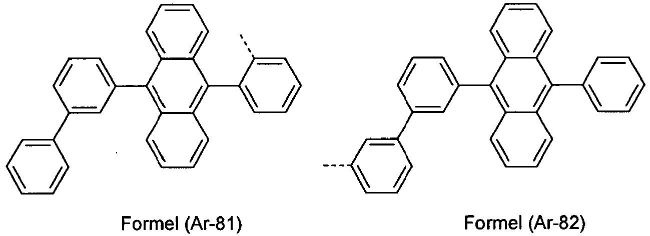 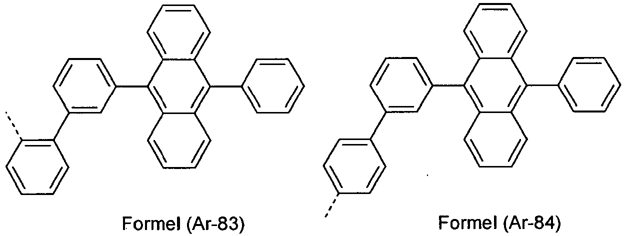 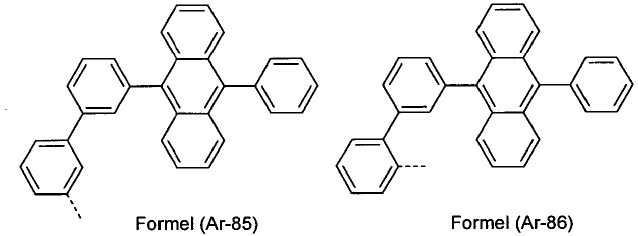 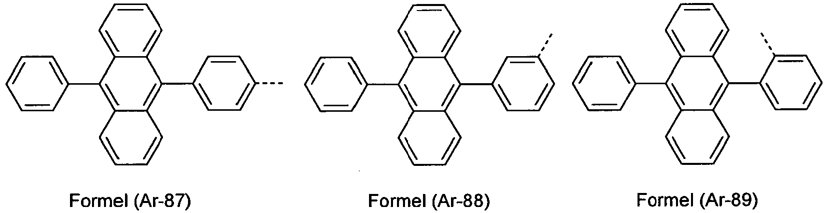 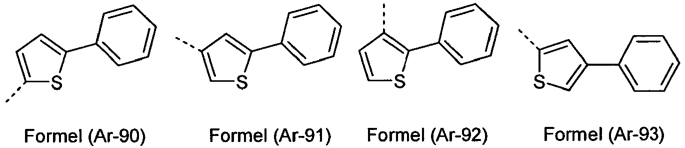 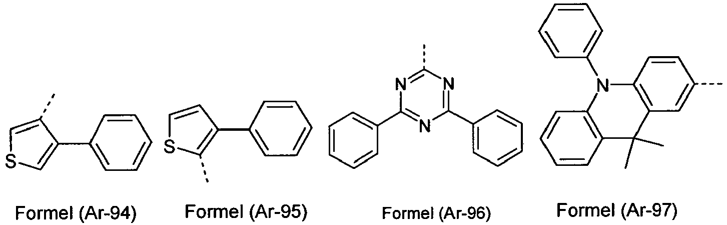 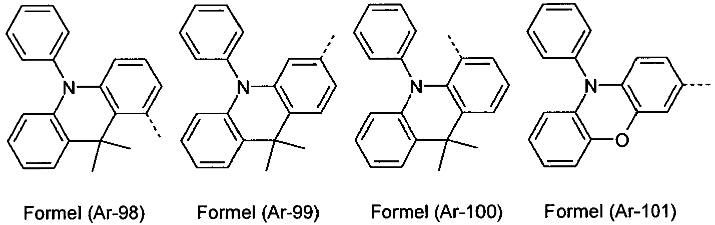 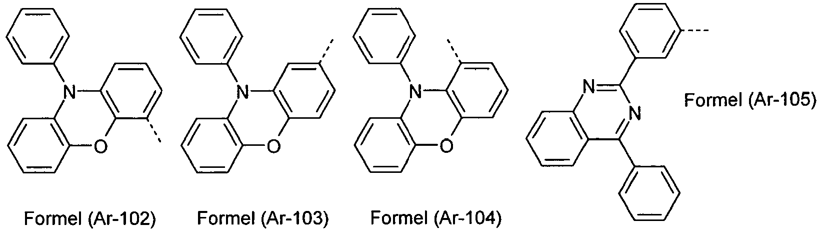 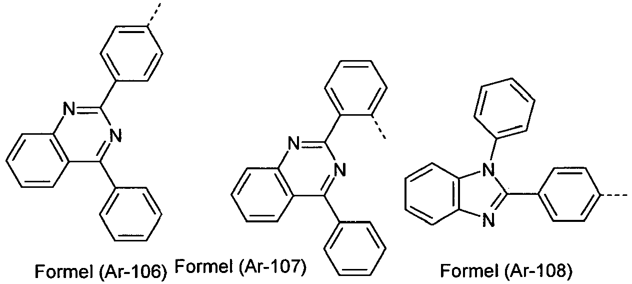 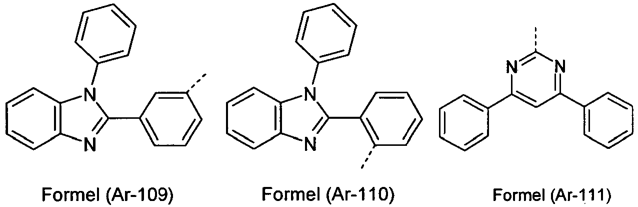 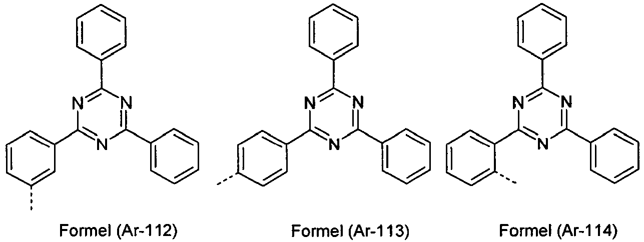 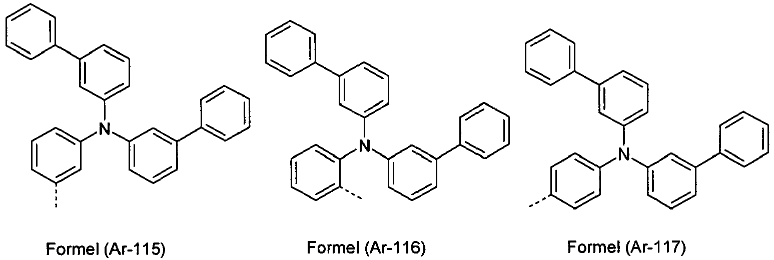 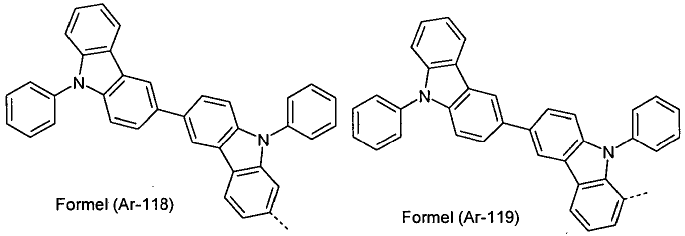 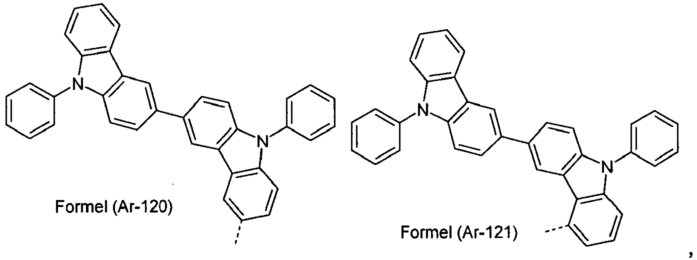 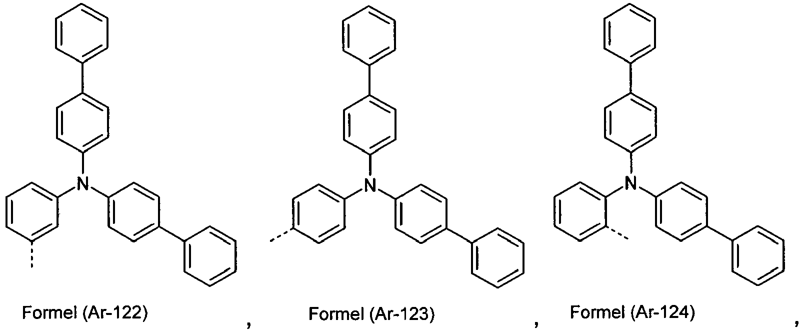 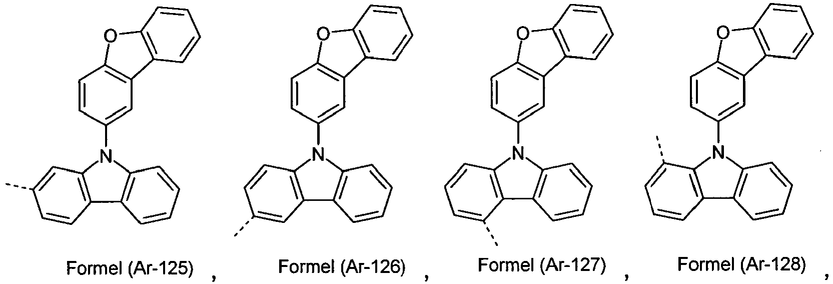 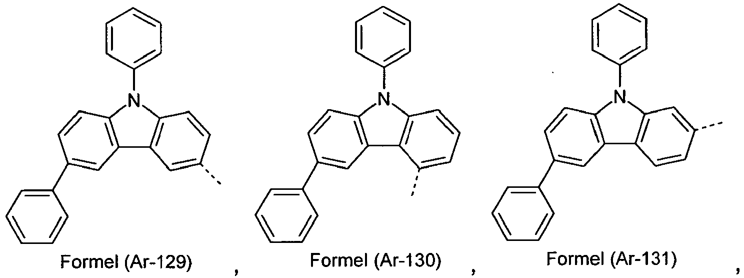 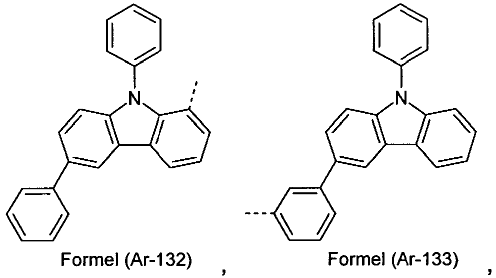 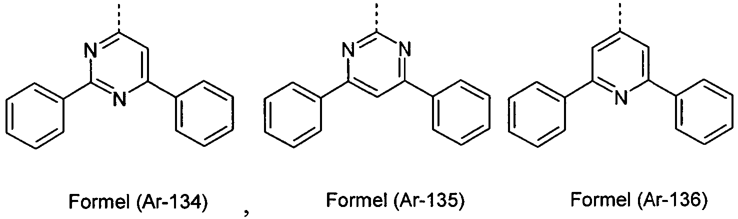 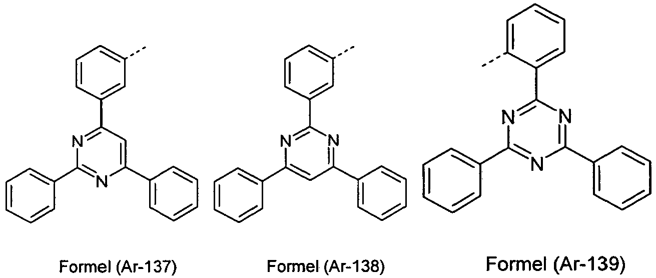 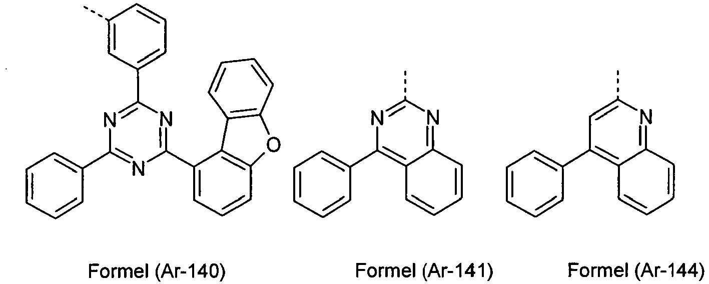 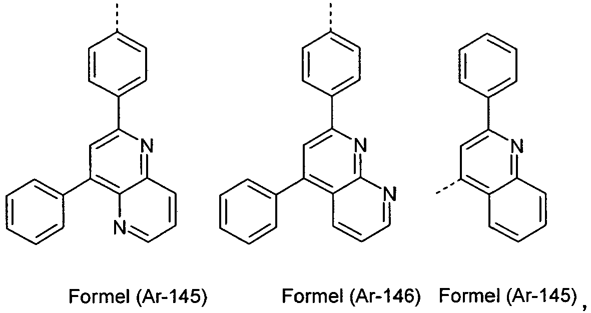 wobei die gestrichelte Bindung die Bindung an das Grundgerüst angibt und die angegebenen Gruppen für Ar jeweils durch einen oder mehrere Reste R¹ substituiert sein können, bevorzugt aber unsubstituiert sind.

In Verbindungen der Formel (4) oder (4a), wie zuvor beschrieben, steht das verbindende aromatische oder heteroaromatische Ringsystem Ar ganz besonders bevorzugt für einen Rest der Formel (Ar-10), (Ar-17), (Ar-22), (Ar-23), (Ar-29), (Ar-39) oder (Ar-43), wobei die gestrichelte Bindung eine Verknüpfung an das eine Grundgerüst angibt und die zweite Verknüpfungsstelle an das zweite Grundgerüst beliebig ist. In einer bevorzugten Ausführungsform der Verbindungen der Formel (4) oder (4a) sind die zwei Verknüpfungsstellen des verbindenden aromatischen oder heteroaromatischen Ringsystems Ar bevorzugt symmetrisch ausgewählt, beispielsweise dargestellt durch die Formeln (Ar-146) bis (Ar-152), 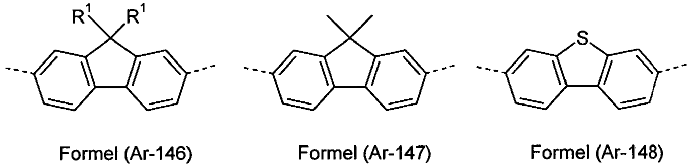 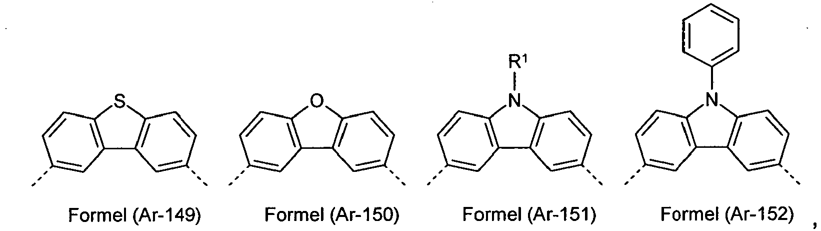 oder die zwei Verknüpfungsstellen des verbindenden aromatischen oder heteroaromatischen Ringsystems Ar werden bevorzugt aus den Teilformeln (Ar-153) oder (Ar-145) ausgewählt

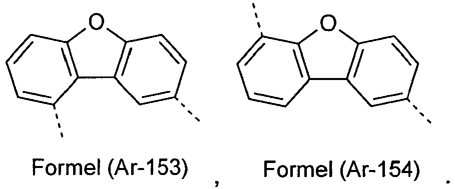

Bevorzugte Verbindungen der Formel (2a) sind Verbindungen der Formeln (6) bis (11) 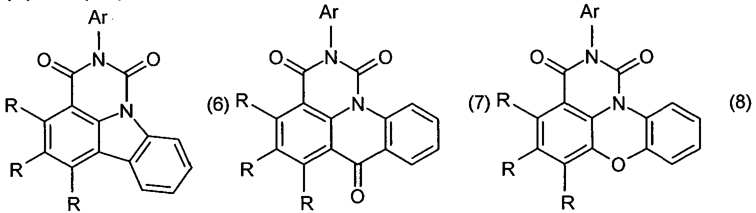 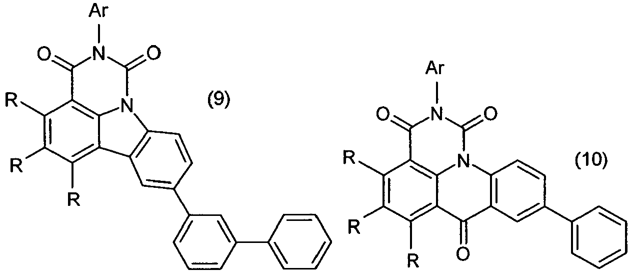 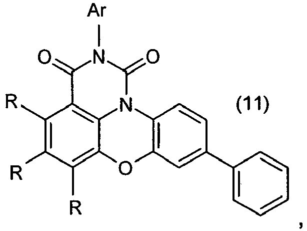 wobei Ar, R, R¹ und R² jeweils unabhängig voneinander eine zuvor genannte Bedeutung oder eine zuvor oder nachfolgend angegebene bevorzugte Bedeutung haben. In Verbindungen der Formeln (6) bis (11) sind 2 Substituenten R bevorzugt H und ein Substituent R entspricht einem Substituenten R³, wobei
R³ aus der Gruppe bestehend aus D, F, CN, einer geradkettigen oder verzweigten Alkylgruppe mit 1 bis 20 C-Atomen, einer geradkettigen oder verzweigten Alkenylgruppe mit 2 bis 20 C-Atomen, einem aromatischem oder heteroaromatischem Ringsystem mit 5 bis 30 aromatischen Ringatomen, in dem ein oder mehrere H-Atome durch D, F, Cl, Br, I, CN oder eine geradkettige oder verzweigte Alkylgruppe mit 1 bis 10 C-Atomen oder eine geradkettige oder verzweigte Alkenylgruppe mit 2 bis 10 C-Atomen ersetzt sein können, ausgewählt wird.

Bevorzugte Verbindungen werden durch die Formeln (6*) bis (11*) dargestellt, 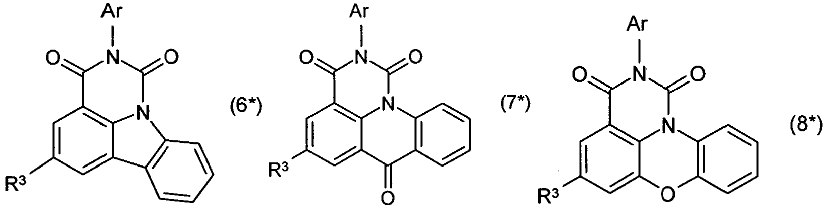 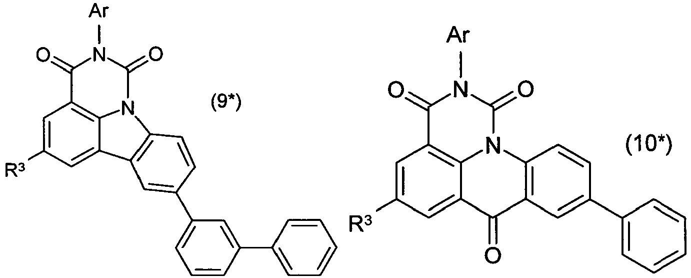 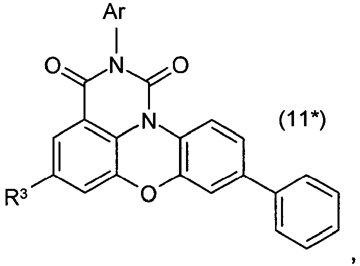 wobei Ar, R, R¹, R² und R³ jeweils unabhängig voneinander eine zuvor genannte Bedeutung oder eine zuvor oder nachfolgend angegebene bevorzugte Bedeutung haben.

Bevorzugte Verbindungen der Formeln (3g), (3h), (3i) und (3j) sind Verbindungen der Formeln (12) bis (33), die mit einem oder mehreren Resten R¹ substituiert sein können, 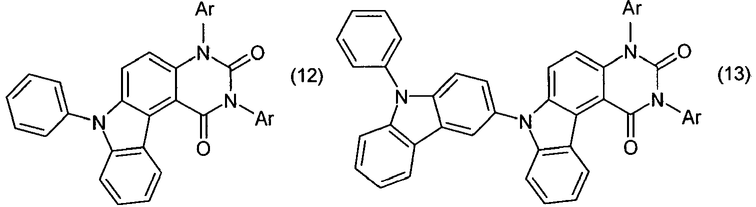 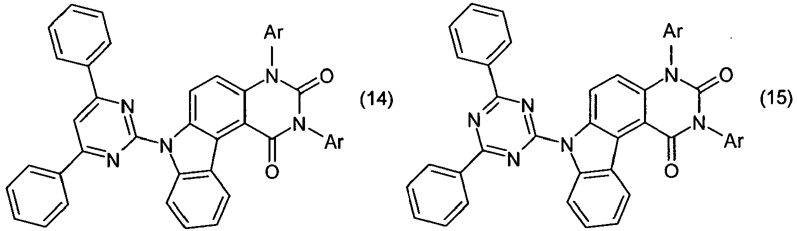 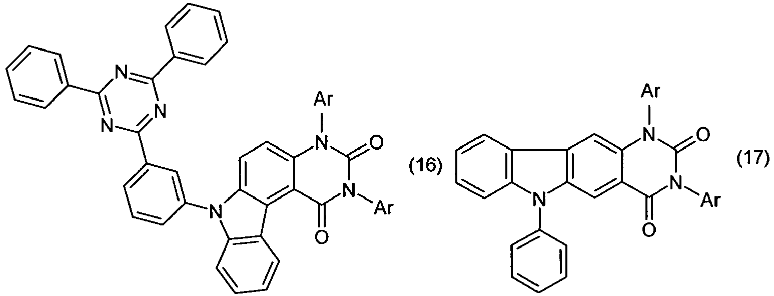 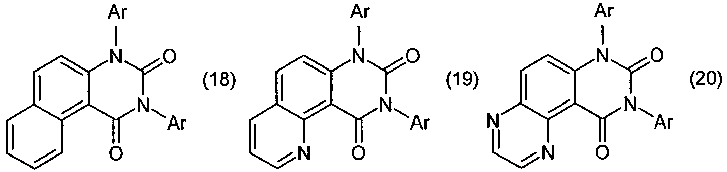 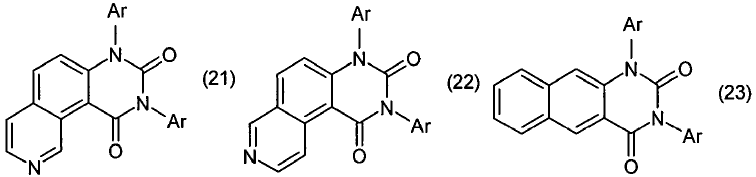 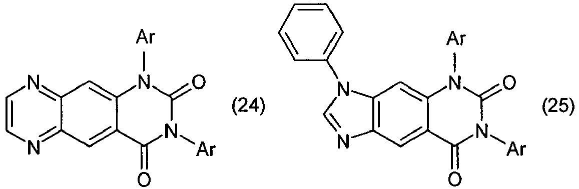 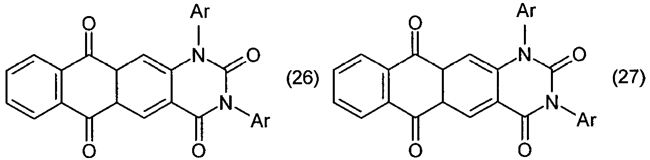 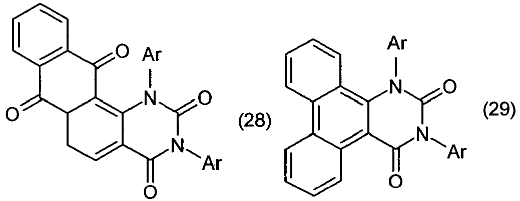 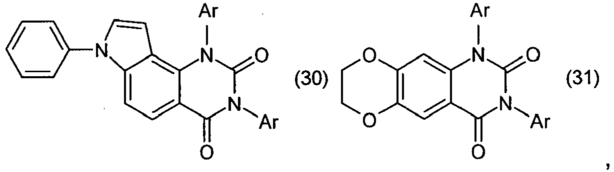 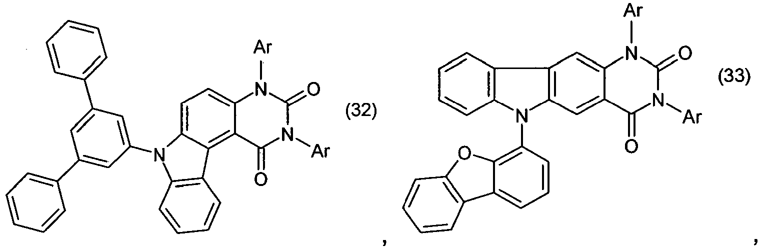 wobei Ar, R¹ und R² jeweils unabhängig voneinander eine zuvor genannte Bedeutung oder eine zuvor oder nachfolgend angegebene bevorzugte Bedeutung haben. In den Verbindungen der Formeln (12) bis (33) ist R¹ ganz besonders bevorzugt H.

In Verbindungen der Formel (2), wie zuvor beschrieben oder als bevorzugte Verbindungen der Formeln (2a), (3), (3a) bis (3j), (4), (4a), (5) und (5a), (6) bis (33) oder (6*) bis (11*) beschrieben, ist R oder R³ jeweils unabhängig voneinander bevorzugt H oder ein aromatisches oder heteroaromatisches Ringsystem mit 5 bis 24 Ringatomen, wie zuvor beschrieben. R ist jeweils unabhängig voneinander besonders bevorzugt
H,
Phenyl,

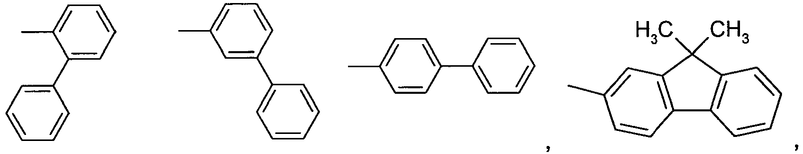

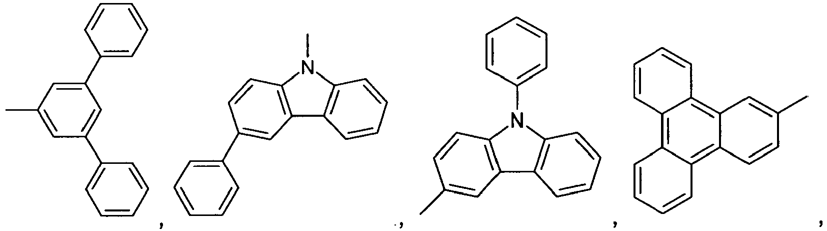

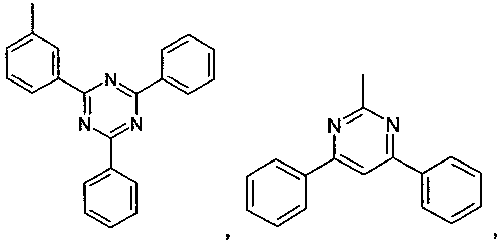

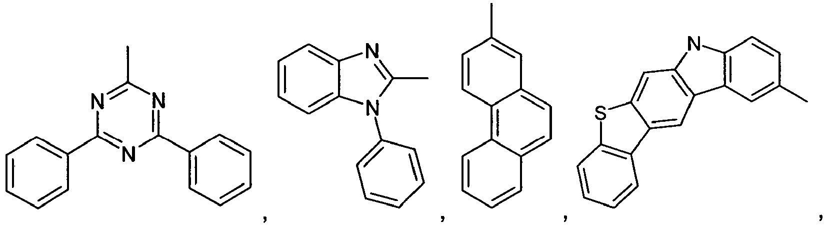

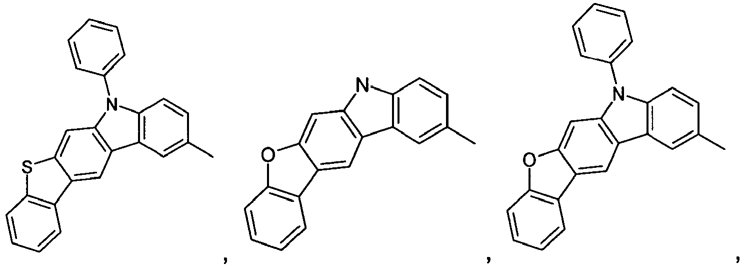

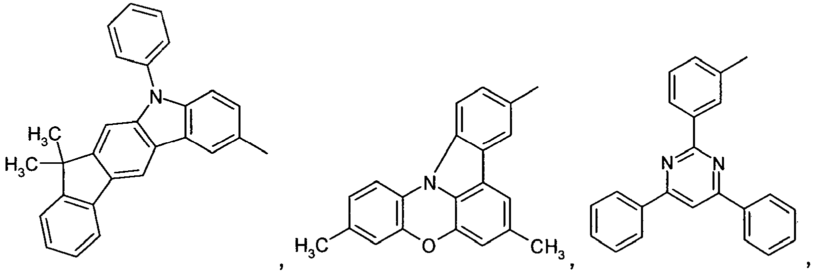

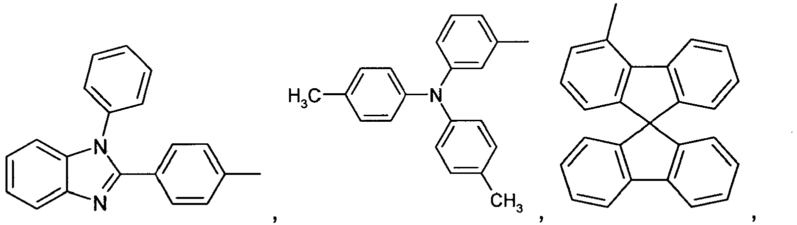

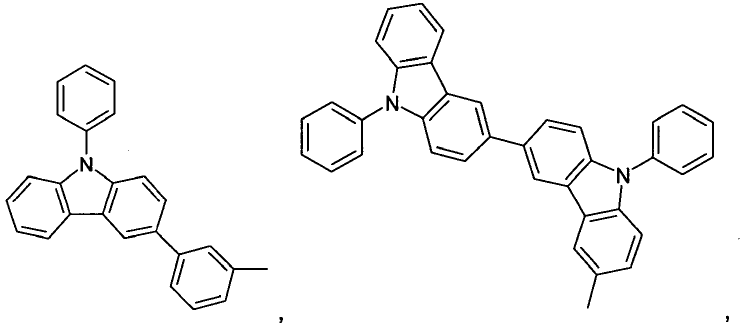

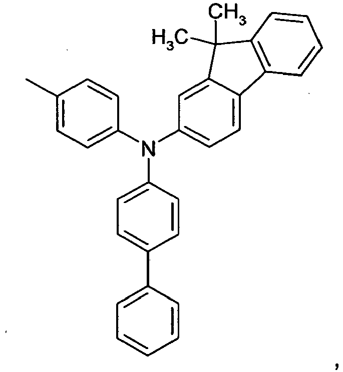

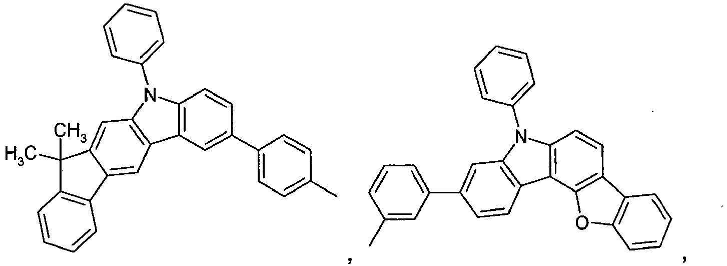

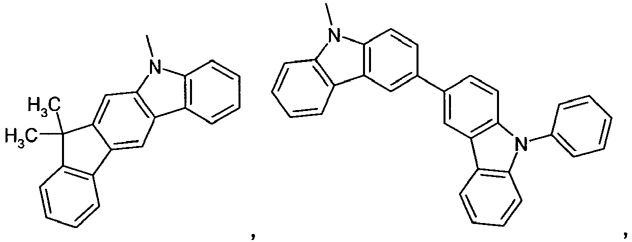

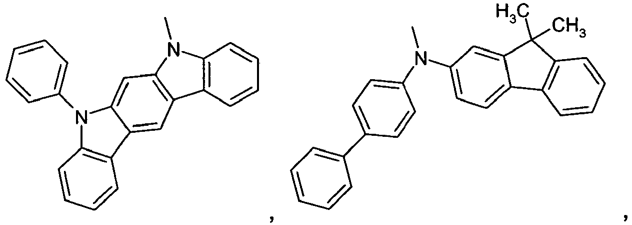

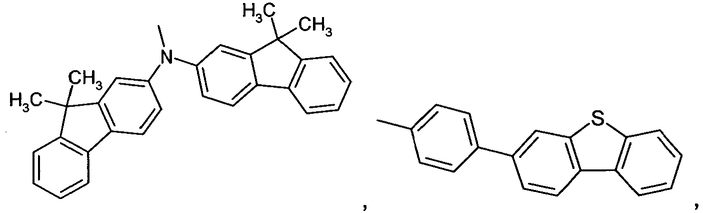

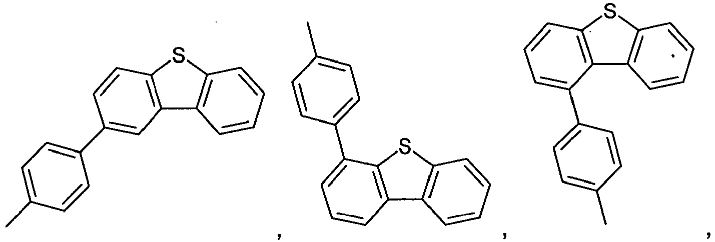

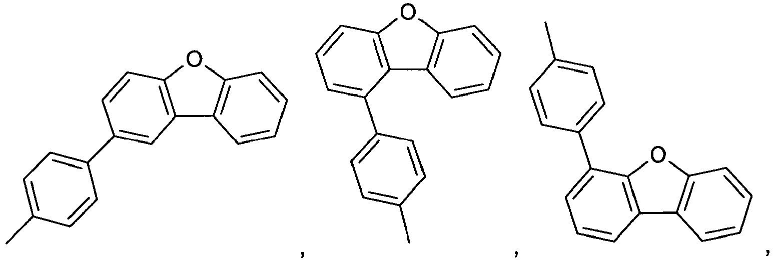

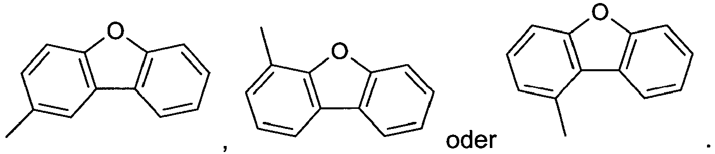

R ist jeweils unabhängig voneinander ganz besonders bevorzugt H oder Phenyl.

R³ ist jeweils unabhängig voneinander besonders bevorzugt Phenyl,

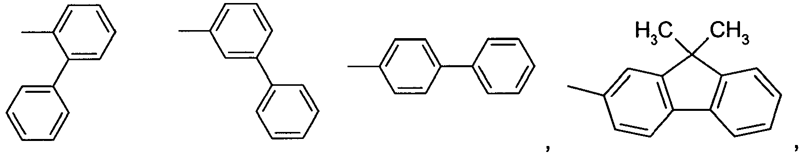

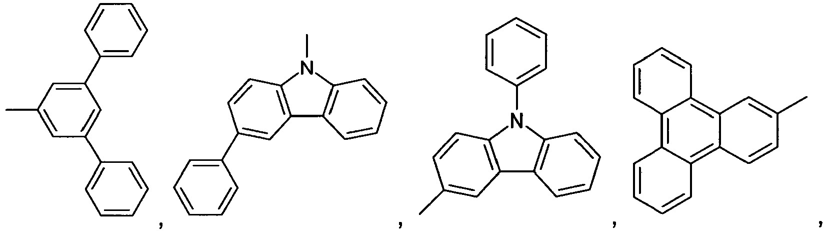

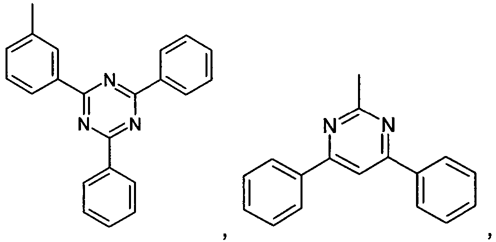

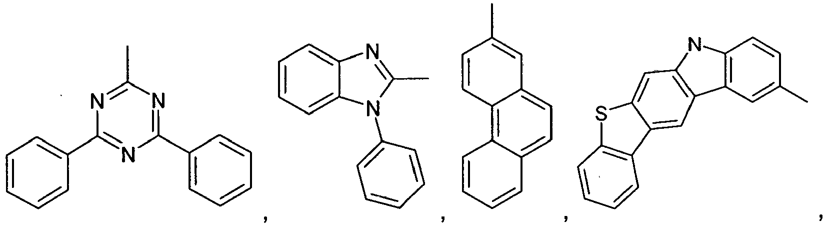

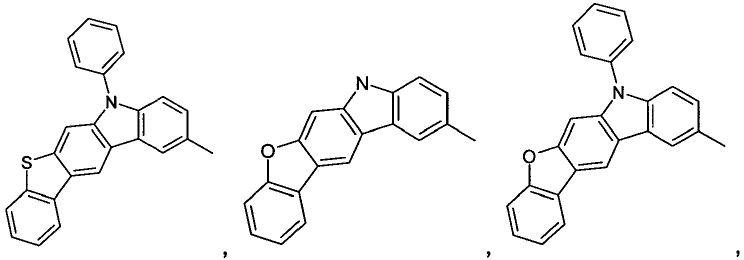

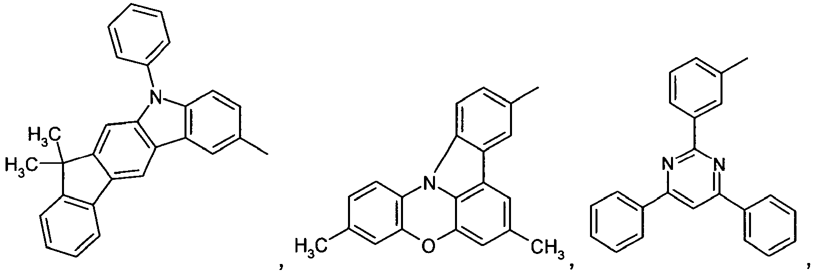

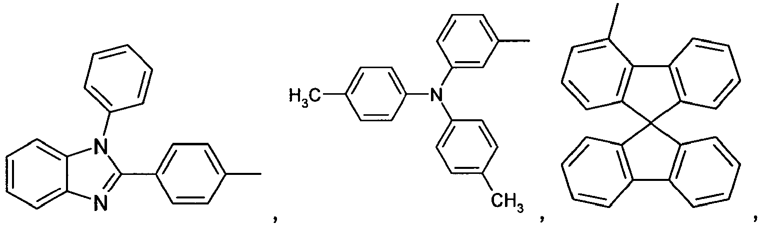

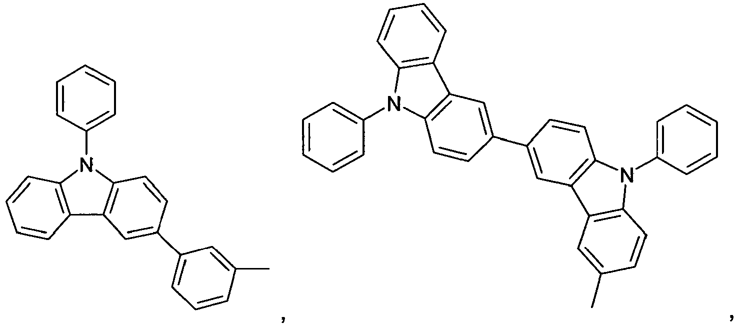

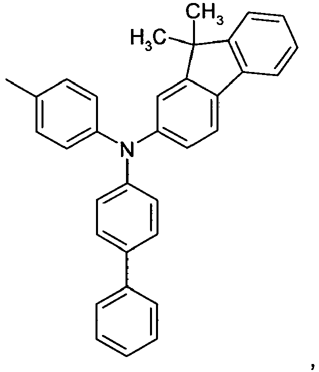

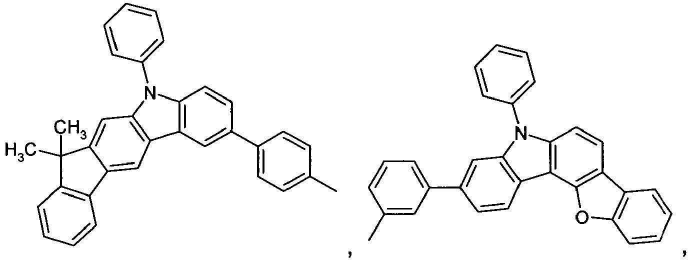

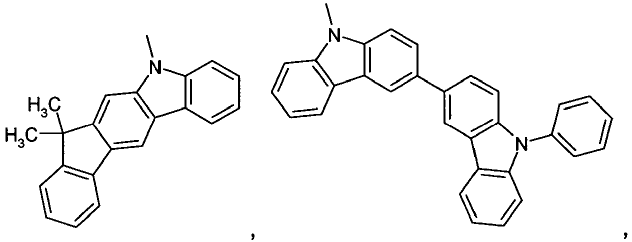

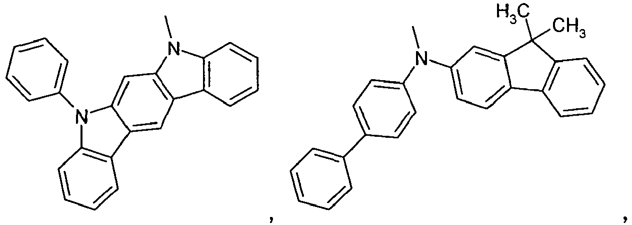

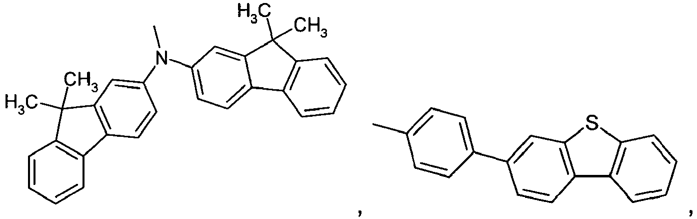

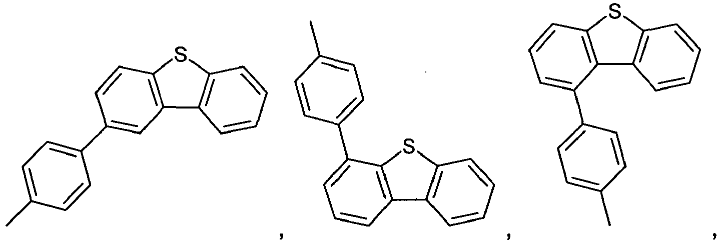

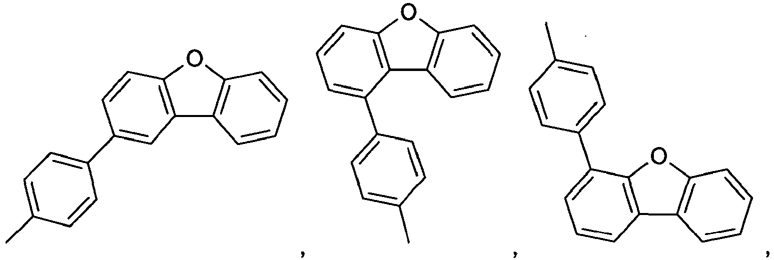

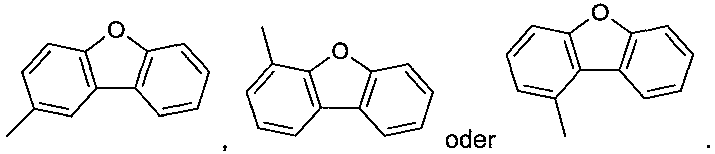

In Verbindungen der Formel (2), wie zuvor beschrieben oder als bevorzugte Verbindungen der Formeln (2a), (3), (3a) bis (3k), (4), (4a), (5) und (5a), (6) bis (33), (6*) bis (11*) beschrieben, bedeutet Ar jeweils unabhängig voneinander bevorzugt ein Ringsystem entsprechend der Formeln (Ar-1) bis (Ar-145).

Die erfindungsgemäß zu verwendenden Verbindungen der Formel (2), wie zuvor beschrieben oder als bevorzugt beschrieben, können nach dem Fachmann prinzipiell bekannten Syntheseschritten dargestellt werden, wie nachfolgend beschrieben.

Arylsubstituierte Verbindungen der Formel (2) wie zuvor beschrieben oder als bevorzugt beschrieben, können nach Schema 1 hergestellt werden: 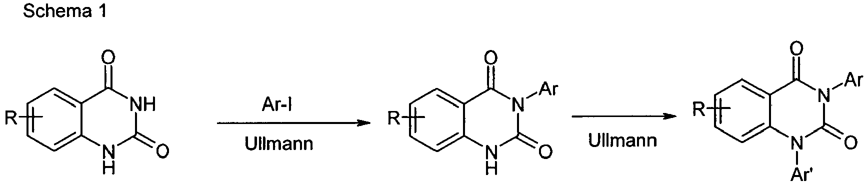 In Schema 1 wird das 2,4(1 H,3H)-Chinazolin-Grundgerüst in einer Ullmann-Reaktion zu einer symmetrischen oder unsymmetrischen Verbindung der Formel (2) umgesetzt, wie zuvor beschrieben. Die Reaktionsbedingungen sowie Startmaterialien der Ullmann-Reaktion sind dem Fachmann bekannt.

Schema 2 zeigt eine weitere Alternative zur Herstellung der arylsubstituierte Verbindungen der Formel (2), wie zuvor beschrieben, wobei gegebenenfalls durch R substituiertes Chinoxalin reduziert wird und das entstehende Amin entsprechend mit einer Buchwald-Reaktion aryliert wird und nachfolgend mit einem Oxidationsmittel, beispielsweise Kaliumpermanganat, oxidiert wird. 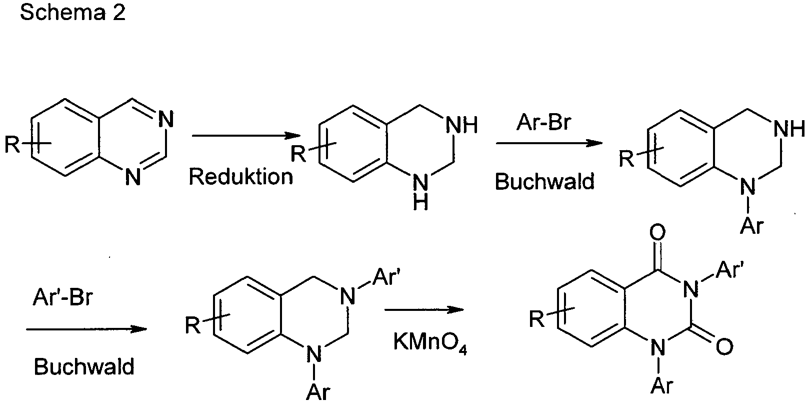 In Schema 2 haben R und Ar eine Bedeutung, wie zuvor angegeben. Die Kennzeichnung Ar' bedeutet lediglich, dass Ar jeweils unabhängig voneinander ist. Die Reaktionsbedingungen einer Reduktion sowie der Buchwald-Arylierung sind dem Fachmann bekannt. Die Reduktion kann beispielsweise durch Umsetzung mit Natriumtetrahydridoborat erfolgen.

Schema 3 zeigt eine alternative Herstellung der Verbindungen der Formel (2), wie zuvor beschrieben, wobei als Startmaterial eine gegebenenfalls durch R substituierte 2-Amino-5-bromo-benzoesäure eingesetzt wird: 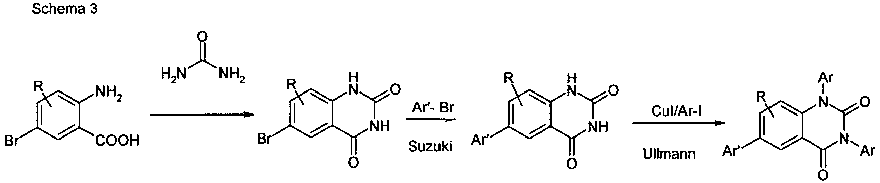 Schema 4 zeigt eine alternative Herstellung der Verbindungen der Formel (2), wie zuvor beschrieben, wobei als Startmaterial eine gegebenenfalls durch R substituierte 2-Amino-5-bromo-benzoesäure eingesetzt wird:

In Schema 3 und Schema 4 haben die Bezeichnungen R und Ar eine Bedeutung, wie zuvor angegeben. Die Kennzeichnung Ar' bedeutet lediglich, dass Ar jeweils unabhängig voneinander ist.

Die Reaktionsbedingungen einer Suzuki-Reaktion, einer Ullmann-Reaktion oder einer Buchwald-Reaktion sind dem Fachmann bekannt.

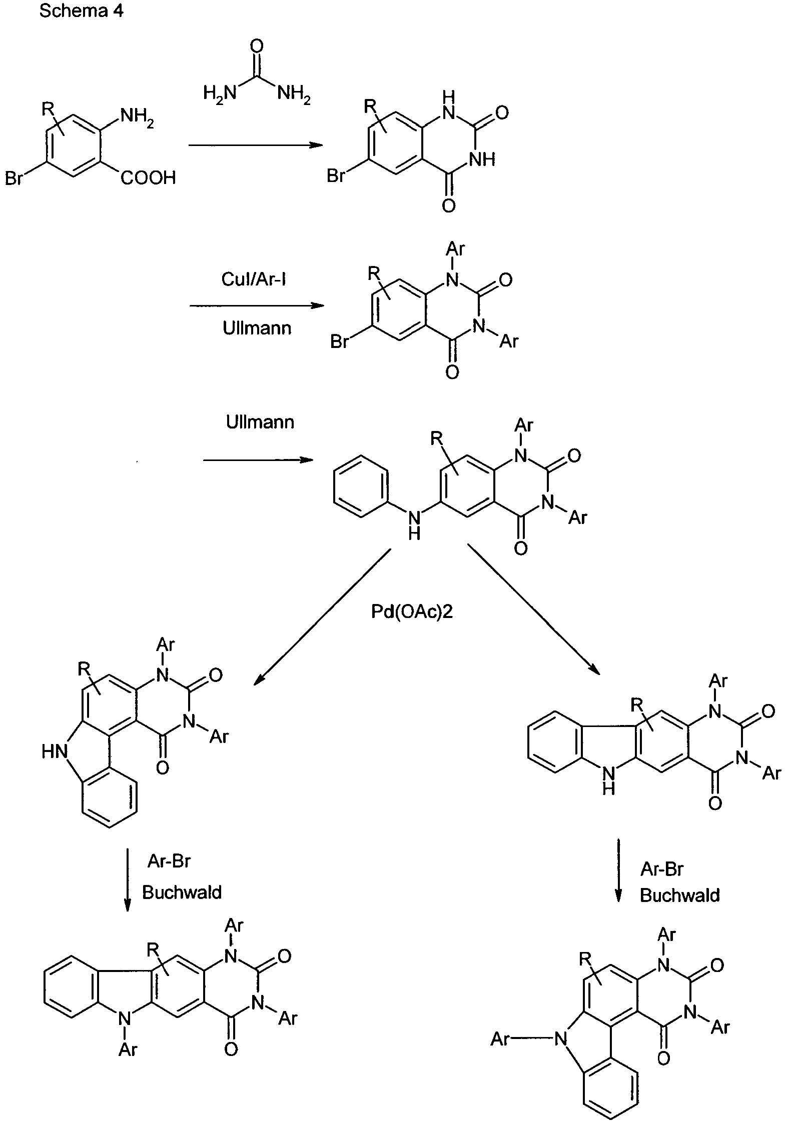

Geeignete Reaktionsbedingungen werden auch im Ausführungsteil beschrieben.

Verbindungen der Formel (1), in denen Y für 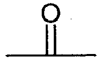 steht, können synonym durch die Formel (34) beschrieben werden, 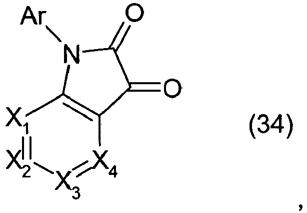 wobei X₁, X₂, X₃, X₄, Ar, R, R¹ und R² jeweils unabhängig voneinander eine bei Formel (1) genannte Bedeutung haben.

In einer bevorzugten Ausführungsform von Verbindungen der Formel (34), bedeuten X₁, X₂, X₃ und X₄ CR. Besonders bevorzugt stehen in dieser Ausführungsform drei Substituenten R für H und ein Substituent entspricht bevorzugt
Phenyl, 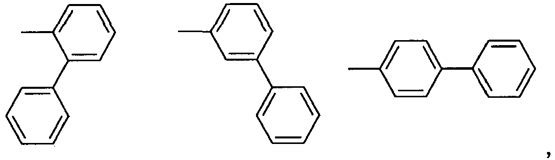 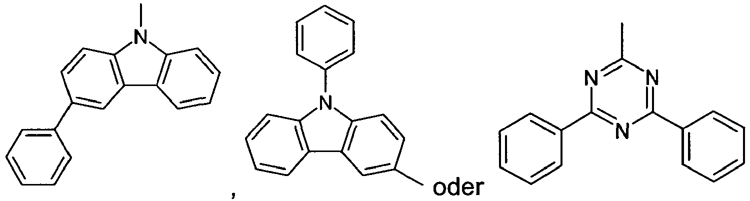 oder zwei benachbarte Substituenten R sind miteinander derart verbunden, dass ein monocyclisches oder polycyclisches, aliphatisches, aromatisches oder heteroaromatisches Ringsystem gebildet wird.
Ganz besonders bevorzugt stehen in dieser Ausführungsform drei Substituenten R für H und ein Substituent entspricht bevorzugt Phenyl,

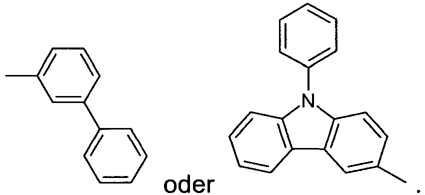

Ausführungsformen der Verbindung der Formel (34), in der zwei benachbarte Substituenten R miteinander verbunden sind, dass ein monocyclisches oder polycyclisches, aliphatisches, aromatisches oder heteroaromatisches Ringsystem gebildet wird, werden bevorzugt durch die Verbindungen der Formeln (35) bis (38) beschrieben, 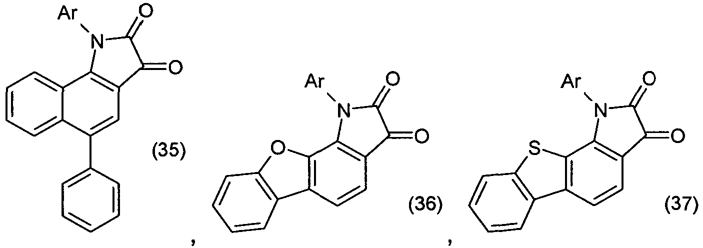 oder 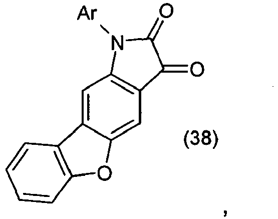 wobei Ar eine zuvor genannte Bedeutung oder eine nachfolgend angegebene bevorzugte Bedeutung hat.

In einer bevorzugten Ausführungsform der elektronischen Vorrichtung sind Verbindungen der Formel (39) enthalten, in denen zwei Verbindungen der Formel (1), in denen Y für 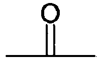 steht, über ein aromatisches oder heteroaromatisches Ringsystem miteinander verbunden sind, 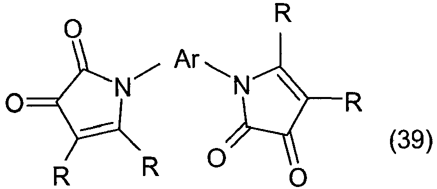 wobei Ar, R, R¹ und R² jeweils unabhängig voneinander eine zuvor genannte Bedeutung oder eine nachfolgend angegebene bevorzugte Bedeutung haben. Das verbindende aromatische oder heteroaromatische Ringsystem Ar ist bevorzugt Phenylen oder entspricht einer der Formeln (Ar-146) bis (Ar-152).

Bevorzugt sind in Verbindungen der Formel (39) die Substituenten R H oder Phenyl oder sie sind miteinander verbunden, so dass ein monocyclisches oder polycyclisches, aliphatisches, aromatisches oder heteroaromatisches Ringsystem gebildet wird.
Bevorzugte Verbindungen der Formel (39) entsprechen den Formeln (40) und (41), 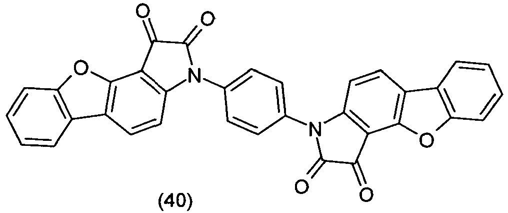 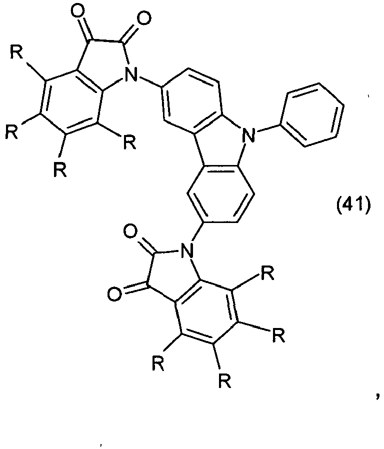 wobei R jeweils unabhängig voneinander eine zuvor genannte Bedeutung oder eine nachfolgend angegebene bevorzugte Bedeutung haben. In den Verbindungen der Formel (41) stehen ganz besonders bevorzugt 3 Substituenten R für H und 1 Substituent R ist Phenyl.

In einer Ausführungsform von Verbindungen der Formeln (34) bis (41), wie zuvor beschrieben oder als bevorzugt hinsichtlich der Substituenten R beschrieben, steht Ar bevorzugt für Phenyl, Naphthyl, Anthracenyl, Phenanthrenyl oder einen der Reste der Formeln (Ar-1) bis (Ar-145).

In einer Ausführungsform von Verbindungen der Formeln (34) bis (41), wie zuvor beschrieben oder als bevorzugt hinsichtlich der Substituenten R beschrieben, steht Ar besonders bevorzugt für einen der Reste der Formel (Ar-2), (Ar-4), (Ar-18), (Ar-27), (Ar-48), (Ar-113), (Ar-126) oder (Ar-129).

Verbindungen der Formel (34) können ausgehend von Diarylaminen durch Umsetzung mit beispielsweise Oxalylchlorid hergestellt werden. Schema 5 beschreibt derartige Umsetzungen, wobei Ar und R eine zuvor genannte Bedeutung oder bevorzugte Bedeutung haben: 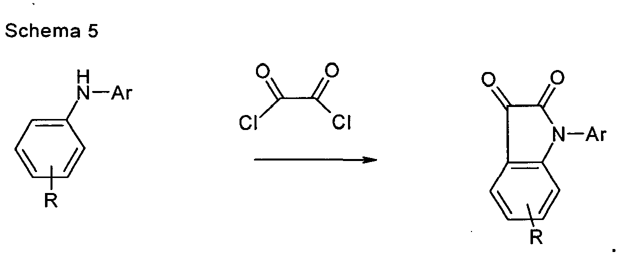 Alternativ können Verbindungen der Formel (34) durch Umsetzung eines kommerziell erhältlichen 1H-Indol-2,3-dions mit einem Arylhalogenid nach Bedingungen der Ullmann-Reaktion oder mit einer Arylboronsäure nach der Literatur Organic Letters, 2004, 6, 18, 3079-82 hergestellt werden. Gegebenenfalls kann 1H-Indol-2,3-dion auch aus einem Anilinderivat hergestellt werden. Schema 6 fasst diese alternative Synthese zusammen, wobei Ar und R eine zuvor genannte Bedeutung oder bevorzugte Bedeutung haben.

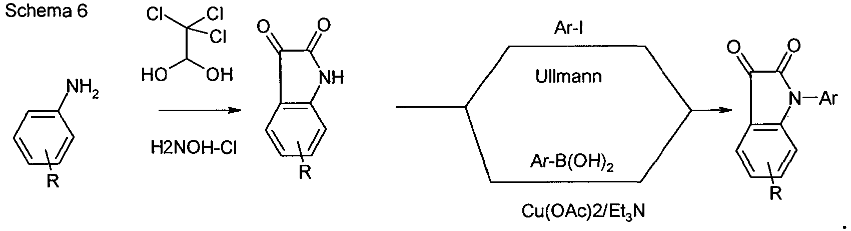

Verbindungen der Formel (1), in denen Y für 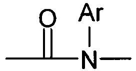 steht, können synonym durch die Formel (42) beschrieben werden, 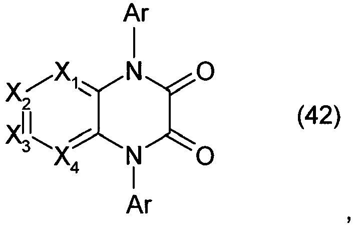 wobei X₁, X₂, X₃, X₄, Ar, R, R¹ und R² jeweils unabhängig voneinander eine bei Formel (1) genannte Bedeutung haben.

In einer bevorzugten Ausführungsform der erfindungsgemäßen elektronischen Vorrichtung sind Verbindungen der Formel (42) enthalten, in denen die Variablen X₁, X₂, X₃ und X₄ CR bedeuten und R und Ar jeweils unabhängig voneinander eine der in Formel (1) genannten Bedeutungen haben.

Ein weiterer Gegenstand der Erfindung ist demzufolge die erfindungsgemäße elektronische Vorrichtung, wie zuvor beschrieben oder als bevorzugt beschrieben, wobei in Formel (42) die Variablen X₁, X₂, X₃ und X₄ CR bedeuten und R und Ar jeweils unabhängig voneinander eine der in Formel (1) genannten Bedeutungen haben.

In einer bevorzugten Ausführungsform der erfindungsgemäßen elektronischen Vorrichtung sind Verbindungen der Formel (42) enthalten, wie zuvor beschrieben oder als bevorzugt beschrieben, wobei X₁, X₂, X₃ und X₄ jeweils unabhängig voneinander CR bedeuten und R jeweils unabhängig voneinander H oder ein aromatisches oder heteroaromatisches Ringsystem mit 5 bis 60 aromatischen Ringatomen ist, das jeweils mit einem oder mehreren Resten R¹ substituiert sein kann oder wobei zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann oder
wobei der Substituent R in X₁ und/oder der Substituent R in X₄ zusammen mit dem jeweils benachbarten N-Ar ein monocyclisches oder polycyclisches, aliphatisches, aromatisches oder heteroaromatisches Ringsystem bilden, das mit einem oder mehreren Resten R¹ substituiert sein kann.

R¹ bedeutet bevorzugt H, D, F, CN oder eine geradkettige oder verzweigte Alkylgruppe mit 1 bis 12 C-Atomen.

R¹ bedeutet besonders bevorzugt H, F, CN, eine geradkettige oder verzweigte Alkylgruppe mit 1 bis 4 C-Atomen.

Die Formel (43) beschreibt demzufolge Verbindungen der Formel (42), in denen die Substituenten X₁ bis X₄ für CR stehen, 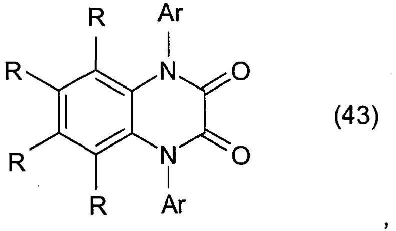 wobei Ar, R¹ und R² jeweils unabhängig voneinander eine zuvor genannte Bedeutung oder eine nachfolgend angegebene bevorzugte Bedeutung haben und R H oder ein aromatisches oder heteroaromatisches Ringsystem mit 5 bis 60 aromatischen Ringatomen ist, das jeweils mit einem oder mehreren Resten R¹ substituiert sein kann bedeuten.

In dieser Ausführungsform der elektronischen Vorrichtung enthaltend mindestens eine Verbindung der Formel (43) ist R besonders bevorzugt jeweils unabhängig voneinander H, Phenyl, 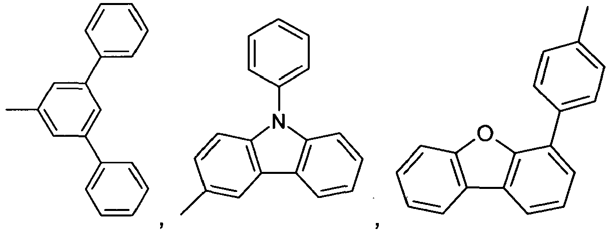 oder

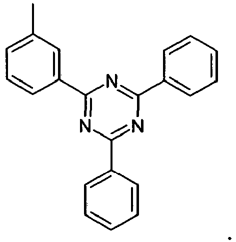

Die Formeln (44), (45) und (46) beschreiben demzufolge Verbindungen der Formel (42), wobei X₁, X₂, X₃ und X₄ jeweils unabhängig voneinander CR bedeuten und zwei oder mehr benachbarte Substituenten R ein monocyclisches oder polycyclisches, aliphatisches, aromatisches oder heteroaromatisches Ringsystem bilden, welches durch das Symbol "(A" gekennzeichnet ist und die restlichen Substituenten R H bedeuten, 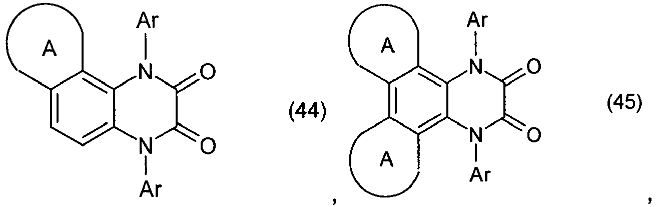 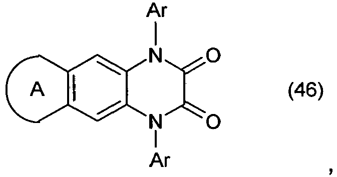 wobei Ar, R¹ und R² jeweils unabhängig voneinander eine zuvor genannte Bedeutung oder eine nachfolgend angegebene bevorzugte Bedeutung haben.

Bevorzugte Verbindungen der Formeln (44) bis (46) entsprechen den Verbindungen der Formeln (47) bis (51) 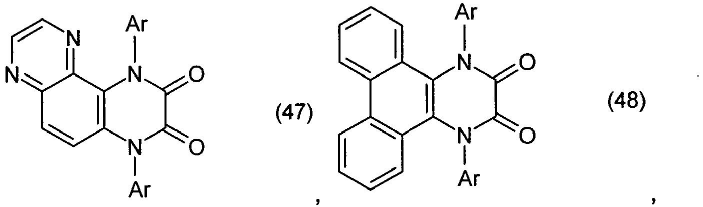 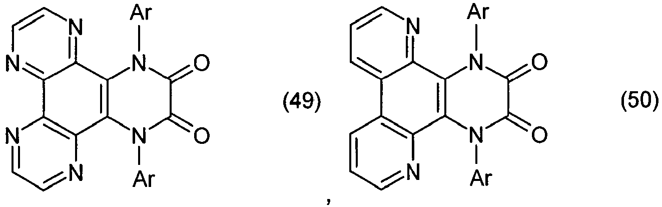 oder 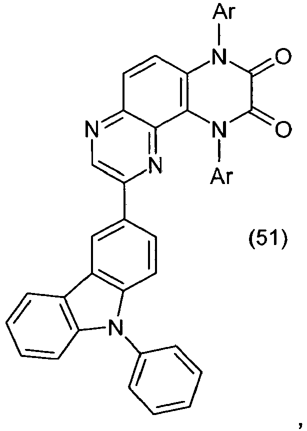 wobei Ar, R¹ und R² jeweils unabhängig voneinander eine zuvor genannte Bedeutung oder eine nachfolgend angegebene bevorzugte Bedeutung haben.

In einer bevorzugten Ausführungsform der erfindungsgemäßen elektronischen Vorrichtung sind Verbindungen der Formel (42) enthalten, wobei X₁, X₂, X₃ und X₄ jeweils unabhängig voneinander CR bedeuten, wobei der Substituent R in X₁ und/oder der Substituent R in X₄ zusammen mit dem jeweils benachbarten N-Ar ein monocyclisches oder polycyclisches, aliphatisches, aromatisches oder heteroaromatisches Ringsystem bilden, das mit einem oder mehreren Resten R¹ substituiert sein kann,
und Ar, R, R¹ und R² jeweils unabhängig voneinander eine zuvor genannte Bedeutung oder eine nachfolgend angegebene bevorzugte Bedeutung haben.

Gegenstand der Erfindung sind ebenfalls die Verbindungen der Formel (42), 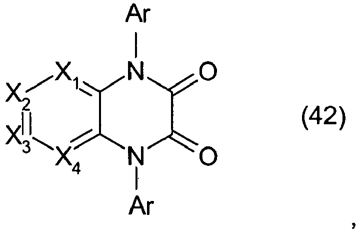 wobei X₁, X₂, X₃ und X₄ jeweils unabhängig voneinander CR bedeuten, wobei der Substituent R in X₁ und/oder der Substituent R in X₄ zusammen mit dem jeweils benachbarten N-Ar ein monocyclisches oder polycyclisches, aliphatisches, aromatisches oder heteroaromatisches Ringsystem bilden, das mit einem oder mehreren Resten R¹ substituiert sein kann,
und Ar, R, R¹ und R² jeweils unabhängig voneinander eine zuvor genannte Bedeutung oder eine nachfolgend angegebene bevorzugte Bedeutung haben.

Die Formel (52) beschreibt demzufolge Verbindungen der Formel (42), wobei X₁, X₂, X₃ und X₄ jeweils unabhängig voneinander CR bedeuten und der Substituent R von X₁ und/oder der Substituent R von X₄ zusammen mit dem jeweils benachbarten N-Ar ein monocyclisches oder polycyclisches, aliphatisches, aromatisches oder heteroaromatisches Ringsystem bilden, das mit einem oder mehreren Resten R¹ substituiert sein kann, 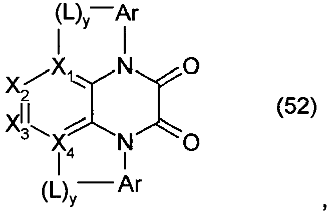 wobei
y jeweils unabhängig voneinander 0 oder 1 bedeutet,
L jeweils unabhängig voneinander -C(R¹)₂- bedeutet und Ar, R, R¹ und R² jeweils unabhängig voneinander eine zuvor genannte Bedeutung oder eine nachfolgend angegebene bevorzugte Bedeutung haben.

Bevorzugte Verbindungen der Formel (52) werden durch die Formeln (53) bis (55) beschrieben, 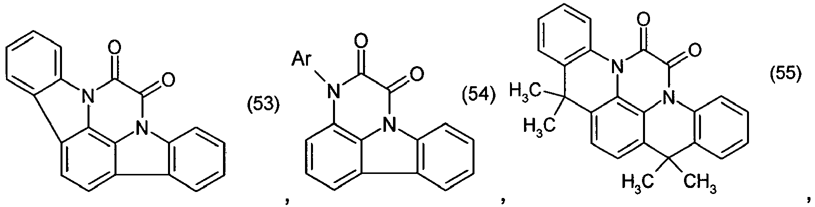 die mit einem oder mehreren Resten R¹ substituiert sein können und Ar, R¹ und R² jeweils unabhängig voneinander eine zuvor genannte Bedeutung haben.

In den Verbindungen der Formeln (42) bis (55) steht Ar jeweils unabhängig voneinander bevorzugt für ein aromatisches oder heteroaromatisches Ringsystem mit 5 bis 24 aromatischen Ringatomen, das mit einem oder mehreren Resten R¹ substituiert sein kann. Besonders bevorzugt ist Ar in dieser Ausführungsform der Erfindung durch einen bevorzugten Rest R¹ substituiert, wie zuvor beschrieben. Besonders bevorzugt ist in dieser Ausführungsform Ar unsubstituiert.
In den Verbindungen der Formeln (42) bis (55) steht Ar jeweils unabhängig voneinander besonders bevorzugt für
Phenyl, Naphthyl, Anthracenyl, Phenanthrenyl oder einen der Reste der Formeln (Ar-1) bis (Ar-145).

Ein weiterer Gegenstand der Erfindung sind die neuen Verbindungen der Formel (56) 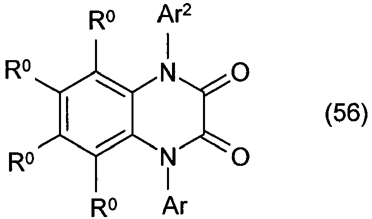 wobei
Ar ein aromatisches oder heteroaromatisches Ringsystem mit 5-60 aromatischen Ringatomen bedeutet, das mit einem oder mehreren nicht-aromatischen Resten R¹ substituiert sein kann,
Ar² ein aromatisches Ringsystem mit 13 bis 40 C-Atomen oder ein heteroaromatisches Ringsystem mit 4 bis 40 C-Atomen bedeutet, das mit einem oder mehreren nicht-aromatischen Resten R¹ substituiert sein kann,
R⁰ jeweils unabhängig voneinander aus der Gruppe bestehend aus H, D, F, CN, einer geradkettigen oder verzweigten Alkylgruppe mit 1 bis 20 C-Atomen, einer geradkettigen oder verzweigten Alkenylgruppe mit 2 bis 20 C-Atomen, einem aromatischem Ringsystem mit 5 bis 30 aromatischen Ringatomen, in dem ein oder mehrere H-Atome durch D, F, Cl, Br, I, CN oder eine geradkettige oder verzweigte Alkylgruppe mit 1 bis 10 C-Atomen oder eine geradkettige oder verzweigte Alkenylgruppe mit 2 bis 10 C-Atomen ersetzt sein können, ausgewählt wird und
wobei R¹ und R jeweils unabhängig voneinander eine wie bei Formel (1) angegebene Bedeutung haben.

Die Verbindungen der Formel (56) sind spezielle Verbindungen der Formel (42), wie zuvor beschrieben.
In bevorzugten Verbindungen der Formel (56) ist R⁰ H oder Phenyl.
In besonders bevorzugten Verbindungen der Formel (56) ist R⁰ H oder Phenyl und Ar entspricht jeweils unabhängig voneinander Phenyl, Naphthyl, Anthracenyl, Phenanthrenyl oder einem der Reste der Formeln (Ar-1) bis (Ar-133).
In besonders bevorzugten Verbindungen der Formel (56) ist R⁰ H oder Phenyl und Ar² entspricht jeweils unabhängig voneinander einem der Reste der Formeln (Ar-1) bis (Ar-133).
In ganz besonders bevorzugten Verbindungen der Formel (56) ist R⁰ H oder Phenyl, Ar² entspricht jeweils unabhängig voneinander einem der Reste der Formeln (Ar-1) bis (Ar-145) und Ar entspricht jeweils unabhängig voneinander Phenyl, Naphthyl, Anthracenyl, Phenanthrenyl oder einem der Reste der Formeln (Ar-1) bis (Ar-145).

In einer bevorzugten Ausführungsform der erfindungsgemäßen elektronischen Vorrichtung sind Verbindungen der Formel (42) enthalten, in denen mindestens eine Variable der Gruppe X₁, X₂, X₃ oder X₄ N bedeutet und die restlichen Variablen CR oder N bedeuten und R und Ar jeweils unabhängig voneinander eine der in Formel (1) genannten Bedeutungen haben.

Ein weiterer Gegenstand der Erfindung ist demzufolge die erfindungsgemäße elektronische Vorrichtung, wie zuvor beschrieben oder als bevorzugt beschrieben, wobei in Formel (42) mindestens eine Variable der Gruppe X₁, X₂, X₃ oder X₄ N bedeutet und die restlichen Variablen CR bedeuten und R und Ar jeweils unabhängig voneinander eine der in Formel (1) genannten Bedeutungen haben.

Bevorzugte Verbindungen der Formel (42), in denen mindestens eine Variable der Gruppe X₁, X₂, X₃ oder X₄ N bedeutet und die restlichen Variablen CR oder N bedeuten und R und Ar jeweils unabhängig voneinander eine der in Formel (1) genannten Bedeutungen haben, entsprechen der Formel (57), 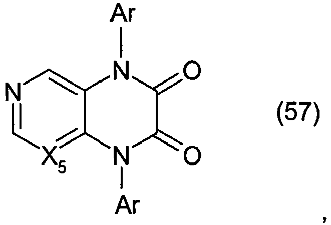 wobei
X₅ CR oder N entspricht und Ar eine bei Formel (1) angegebene Bedeutung hat.

Ein weiterer Gegenstand der Erfindung sind ebenfalls die Verbindungen der Formel (57), 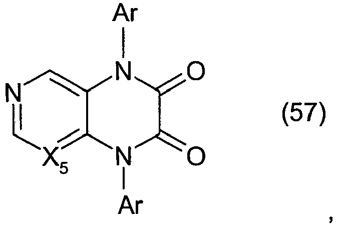 wobei
X₅ CR oder N entspricht und Ar und R eine bei Formel (1) angegebene Bedeutung haben.

Die Verbindungen der Formel (57) sind spezielle Verbindungen der Formel (42), wie zuvor beschrieben.
In bevorzugten Verbindungen der Formel (57) entspricht Ar jeweils unabhängig voneinander Phenyl, Naphthyl, Anthracenyl, Phenanthrenyl oder einem der Reste der Formeln (Ar-1) bis (Ar-133), die gegebenenfalls einfach oder mehrfach mit R¹ substituiert sein können, und R und R¹ haben eine bei Formel (1) angegebene Bedeutung oder eine bevorzugt angegebene Bedeutung.
In besonders bevorzugten Verbindungen der Formel (57) entspricht Ar jeweils unabhängig voneinander Phenyl, Naphthyl, Anthracenyl, Phenanthrenyl oder einem der Reste der Formeln (Ar-1) bis (Ar-133), die gegebenenfalls einfach oder mehrfach mit R¹ substituiert sein können, und R entspricht H.

Die erfindungsgemäß zu verwendenden Verbindungen der Formel (42) bzw. die erfindungsgemäßen Verbindungen der Formel (42), wie zuvor beschrieben oder als bevorzugt beschrieben, können nach dem Fachmann prinzipiell bekannten Syntheseschritten dargestellt werden, wie nachfolgend beschrieben. Geeignete Startmaterialien, wie in den folgenden Schemata angegeben, sind zum Teil kommerziell erhältlich oder können nach bekannten Syntheseverfahren hergestellt werden.

1,4-Dihydro-1,4-diaryl-2,3-chinoxalindion-Derivate der Formel (42) werden beispielsweise synthetisiert, indem ein ortho-Dibrom-substituierter Aromat mit einem primären Amin oder ein ortho-Diamino-substituierter Aromat mit einem Arylbromid in einer Hartwig-Buchwald-Kupplung umgesetzt wird und anschließend mit Ethyloxalat eine Umsetzung durchgeführt wird, wie in Schema 7 beschrieben, wobei R und Ar eine Bedeutung hat, wie zuvor beschrieben oder als bevorzugt beschrieben:

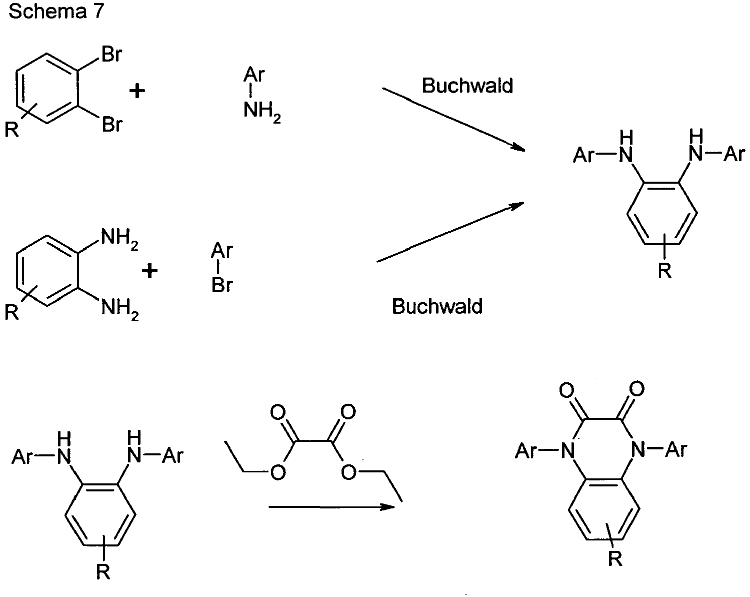

Alternativ kann die Synthese nach Schema 8 stattfinden, wobei R und Ar eine zuvor genannte Bedeutung oder bevorzugte Bedeutung haben. Die Kennzeichnung Ar' bedeutet, dass Ar jeweils unabhängig voneinander eine Bedeutung haben, wie zuvor angegeben. 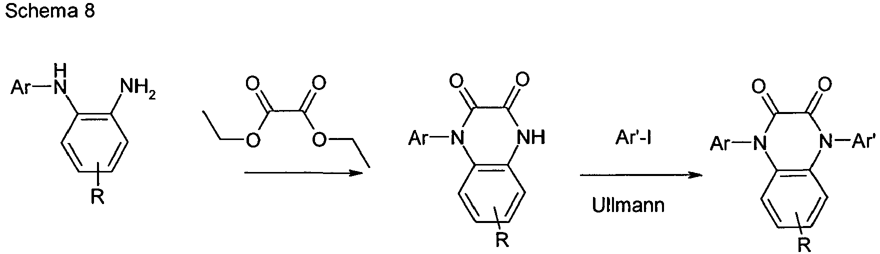 Die Reaktionsbedingungen für eine Ullmann-Reaktion sind dem Fachmann bekannt und sind ebenfalls im Ausführungsteil beschrieben.

Alternativ kann die Synthese nach Schema 9 stattfinden, wobei R und Ar eine zuvor genannte Bedeutung oder bevorzugte Bedeutung haben. Die Kennzeichnung Ar' bedeutet, dass Ar jeweils unabhängig voneinander eine Bedeutung haben, wie zuvor angegeben.

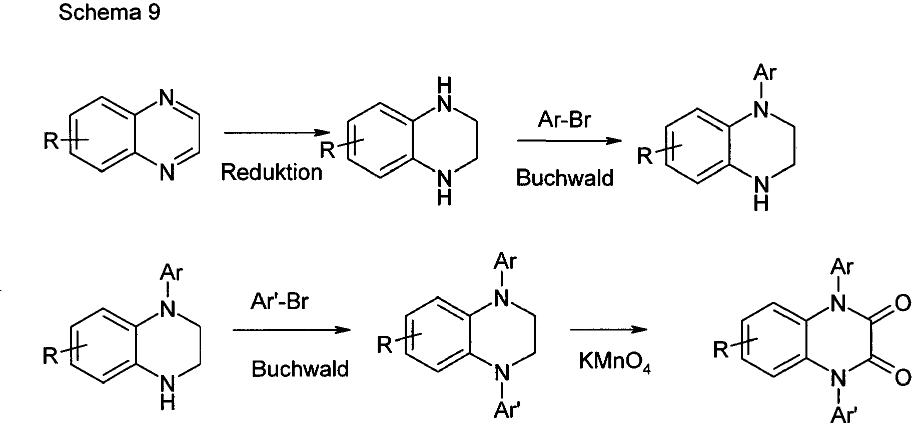

In Schema 9 wird beschrieben, dass gegebenenfalls durch R substituiertes Chinoxalin reduziert wird und das entstehende Amin entsprechend mit einer Buchwald-Reaktion aryliert wird und nachfolgend mit einem Oxidationsmittel, beispielsweise Kaliumpermanganat, oxidiert wird. Die Reaktionsbedingungen für eine Buchwald-Reaktion sind dem Fachmann bekannt. Als Reduktionsmittel kann Natriumtetrahydridoborat verwendet werden.

Im Folgenden werden bevorzugte Verbindungen der Formel (1) aufgelistet, die in der erfindungsgemäßen elektronischen Vorrichtung enthalten sind. Die Einzelverbindungen können ebenfalls einer bevorzugt angegebenen Verbindung der Formeln (2), (2a), (3), (3a) bis (3j), (4), (4a), (5) und (5a), (6) bis (11), (6*) bis (11*), (12) bis (33), (34) bis (41) und (42 bis (57) zugeordnet werden. Die Einzelverbindungen sind ganz besonders bevorzugt in der elektronischen Vorrichtung zu verwenden.

Bevorzugte Verbindungen der Formel (1), wie zuvor beschrieben, oder als bevorzugt beschrieben sind:

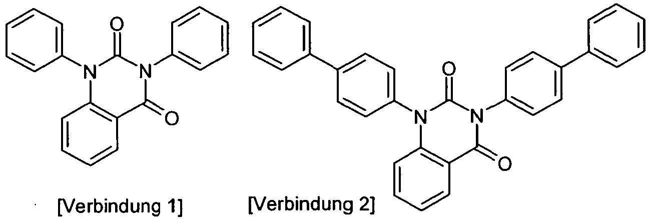

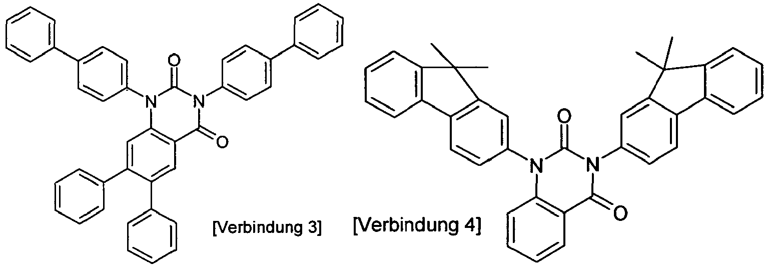

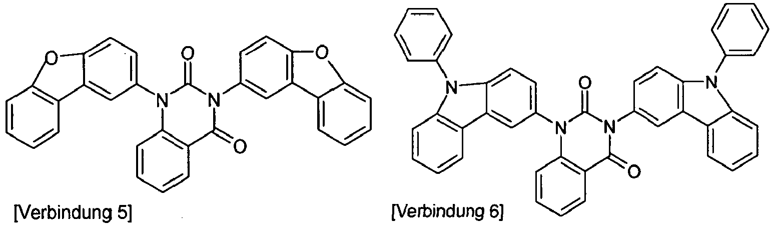

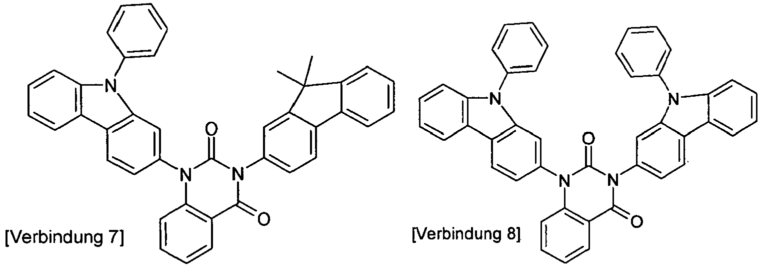

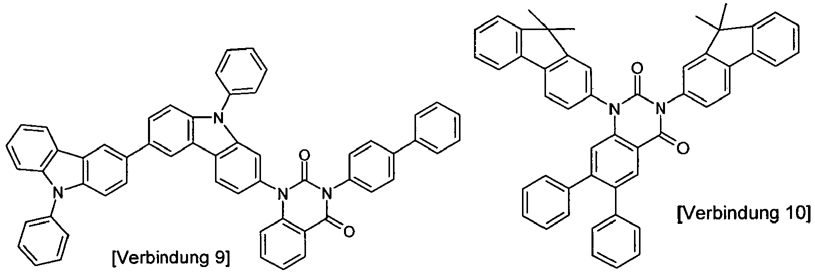

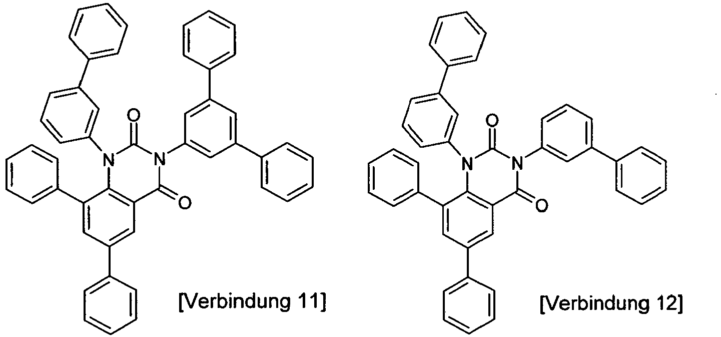

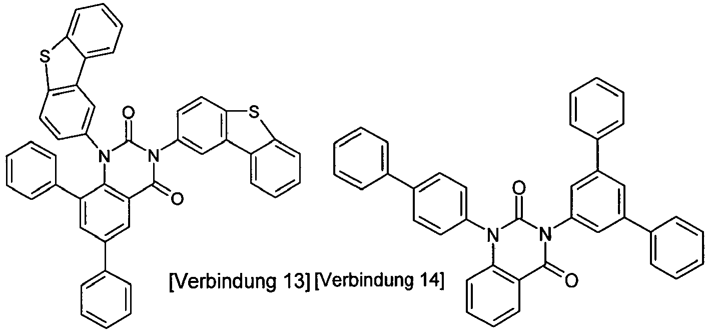

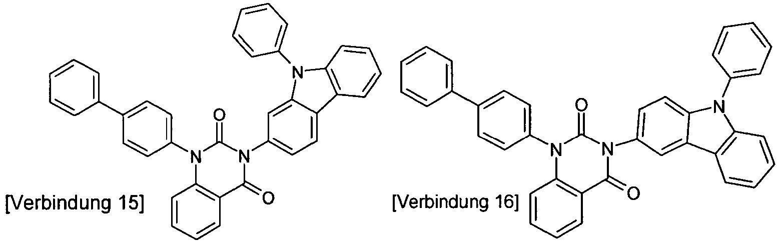

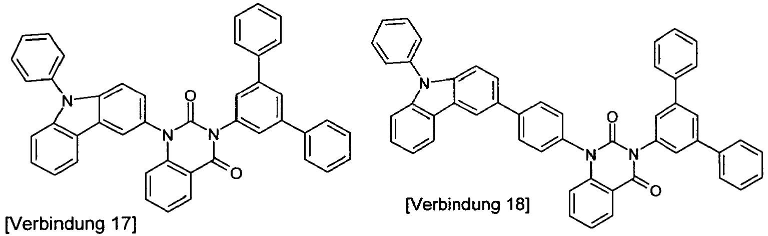

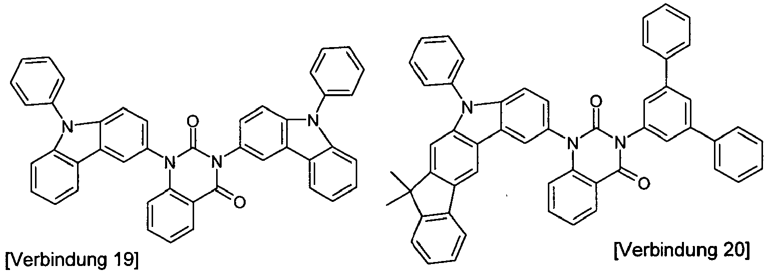

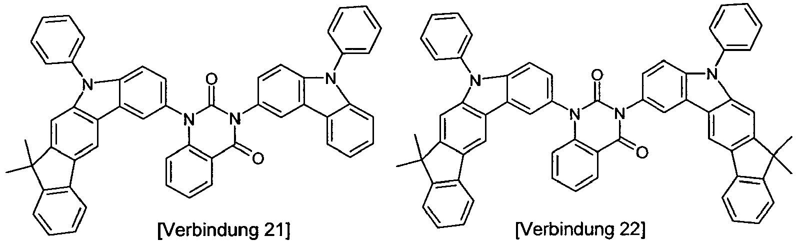

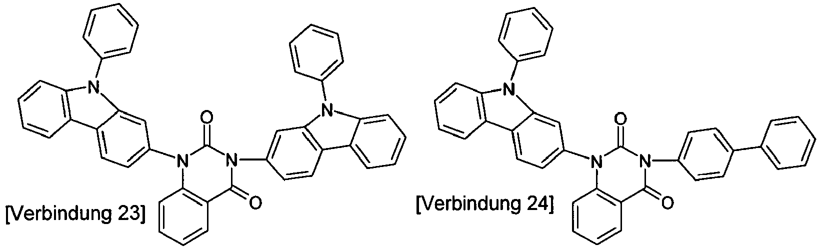

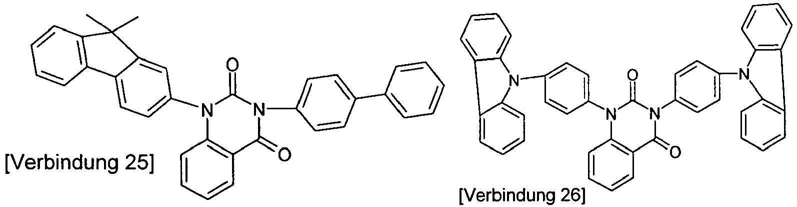

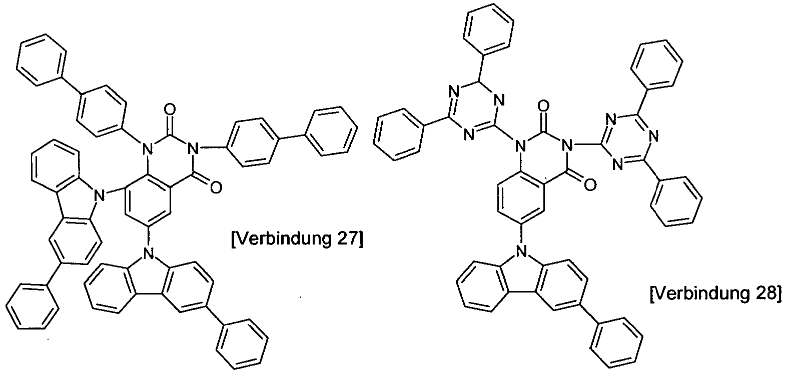

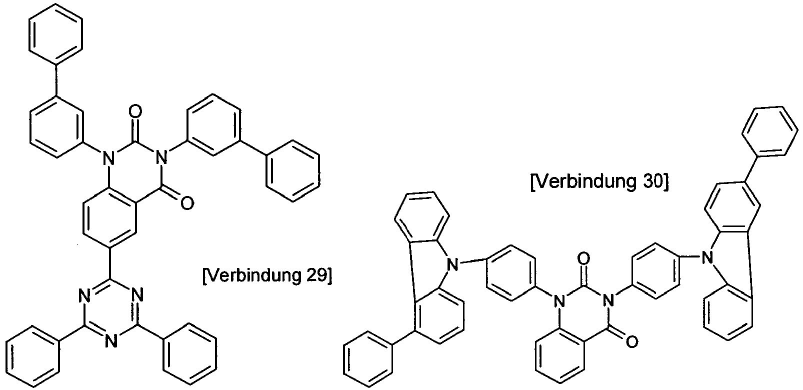

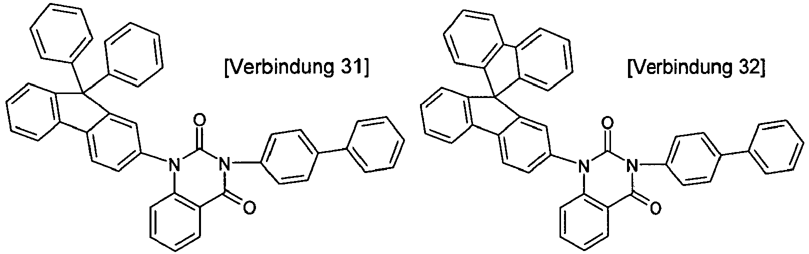

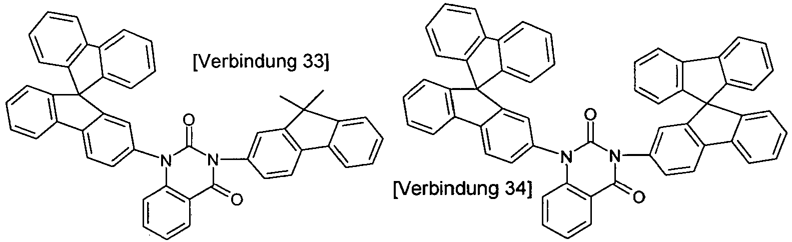

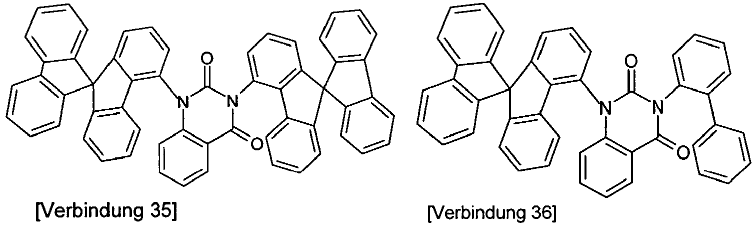

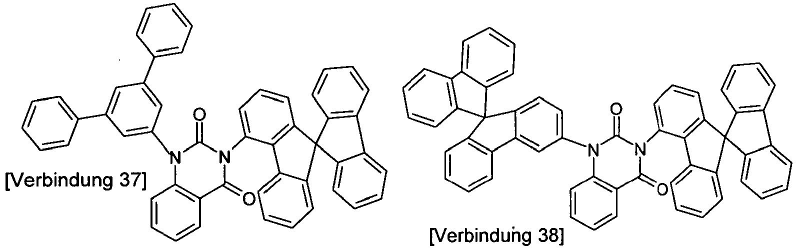

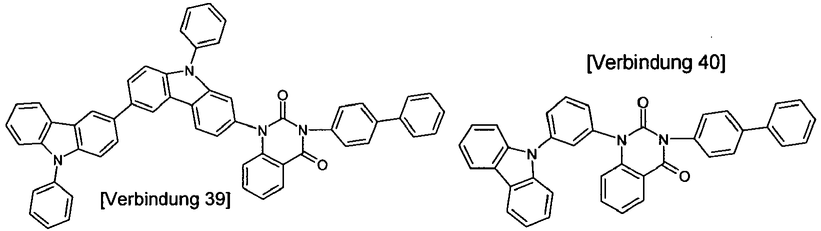

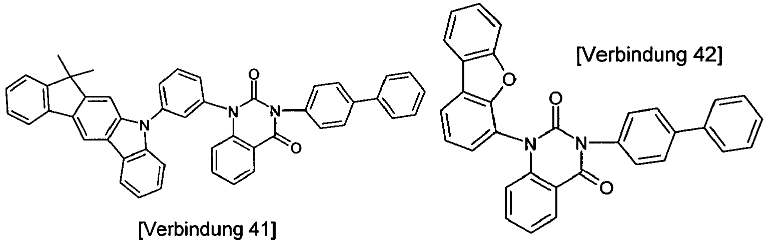

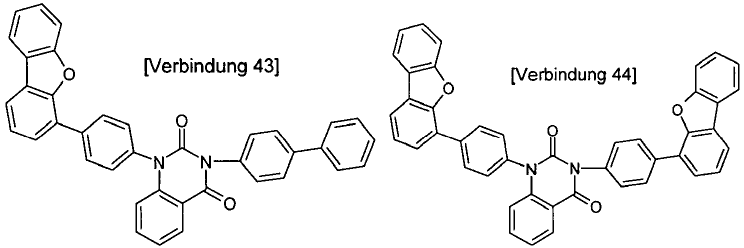

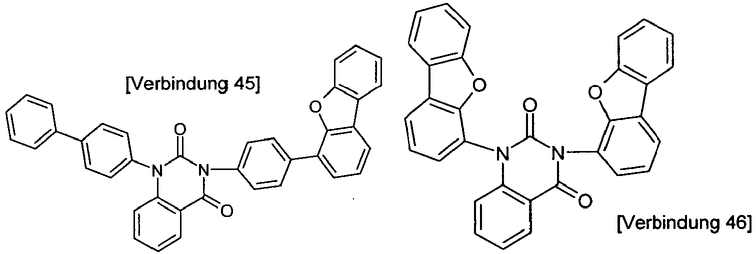

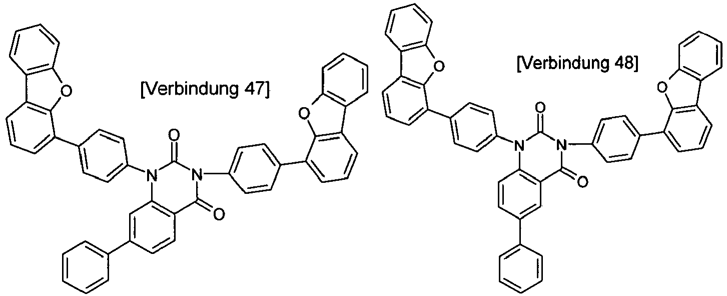

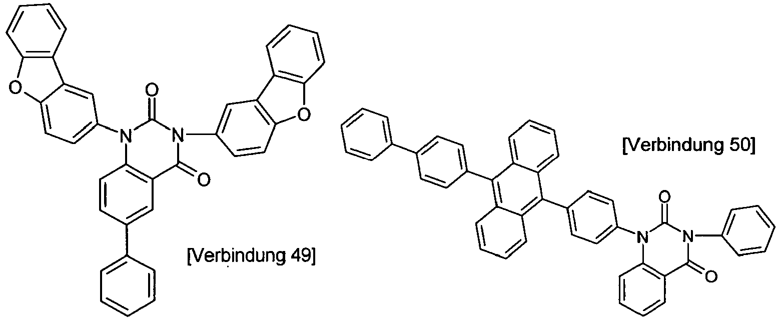

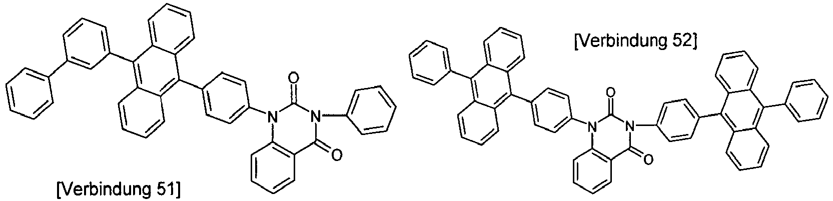

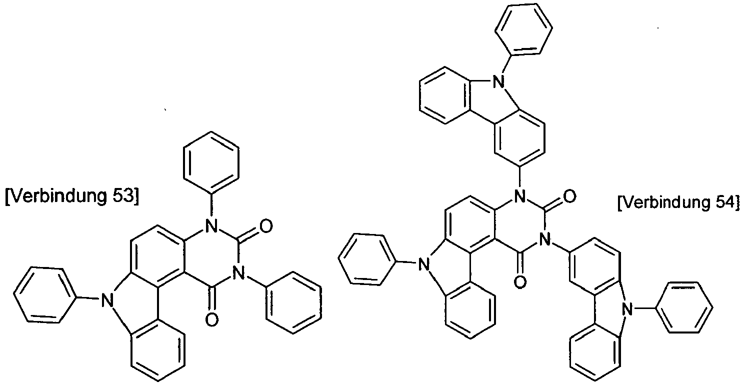

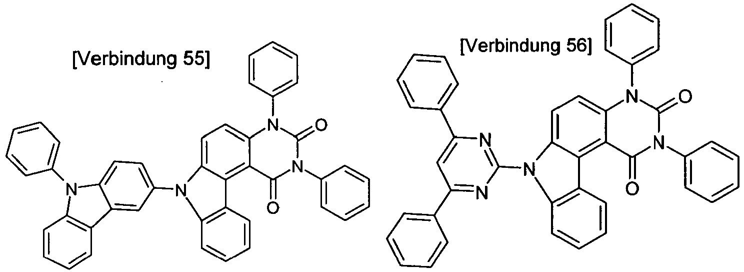

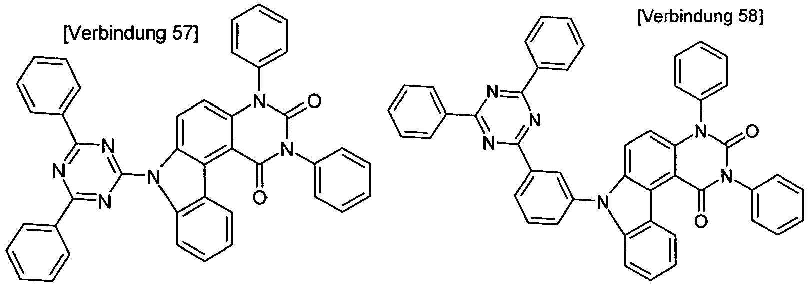

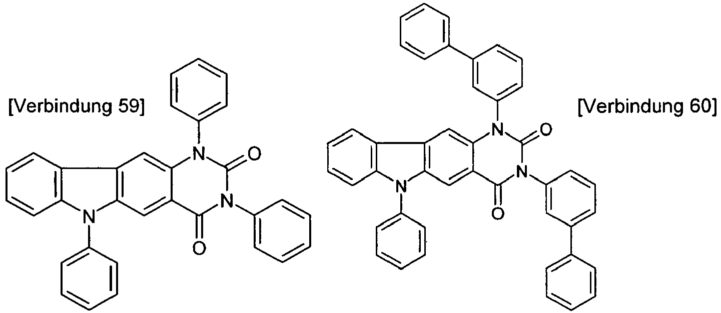

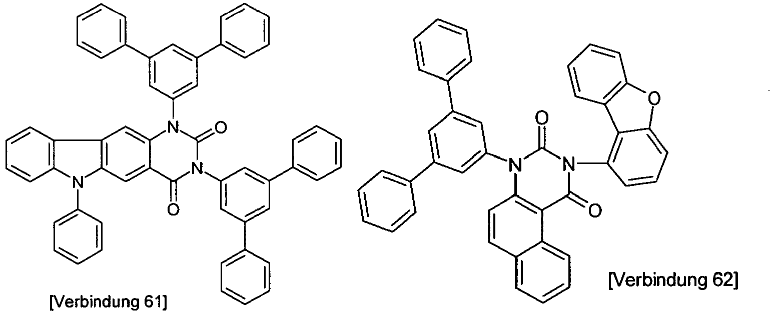

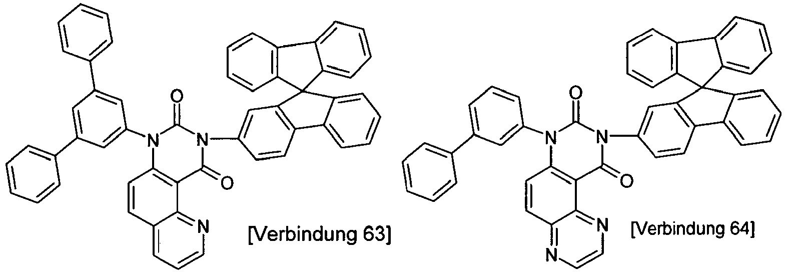

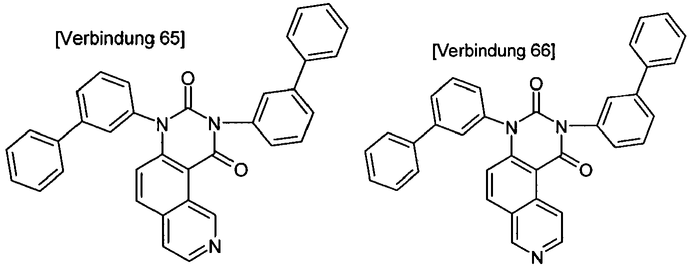

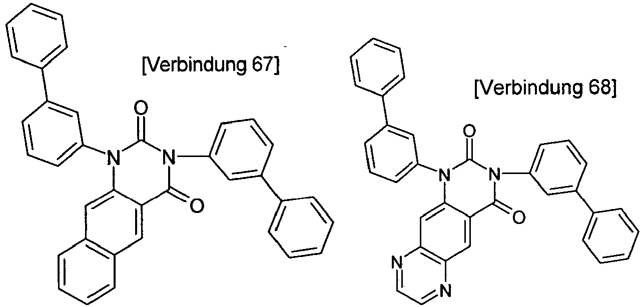

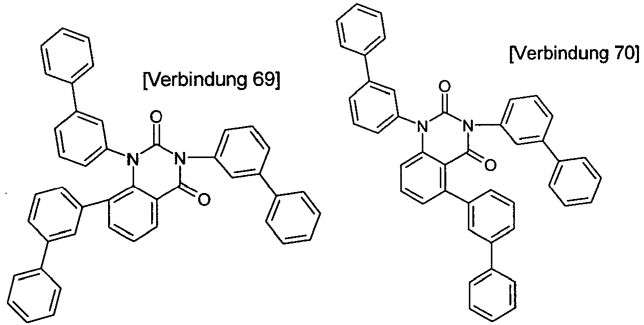

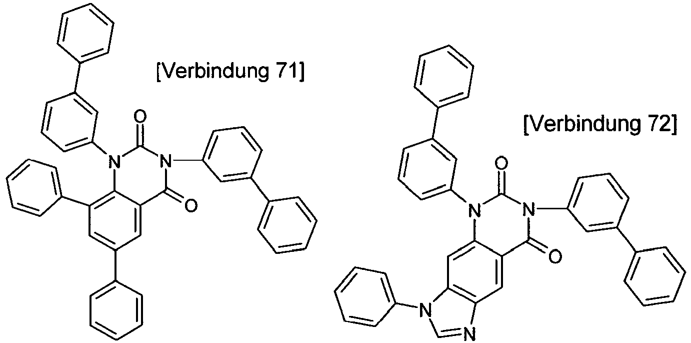

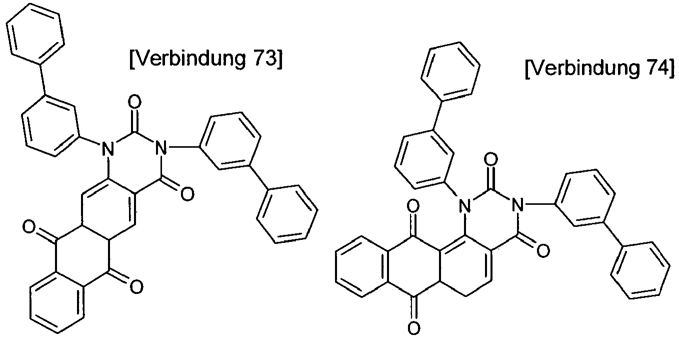

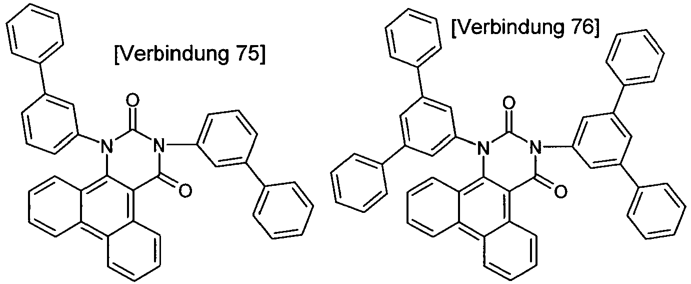

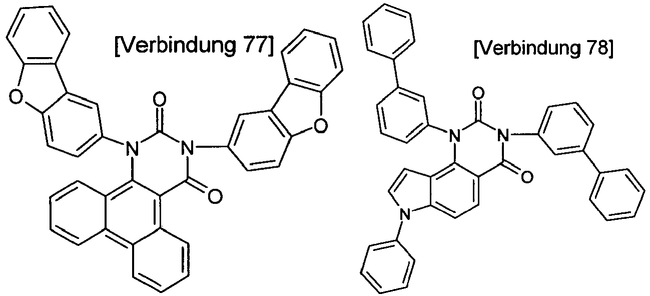

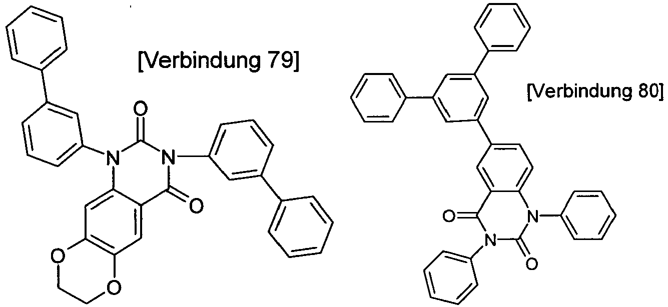

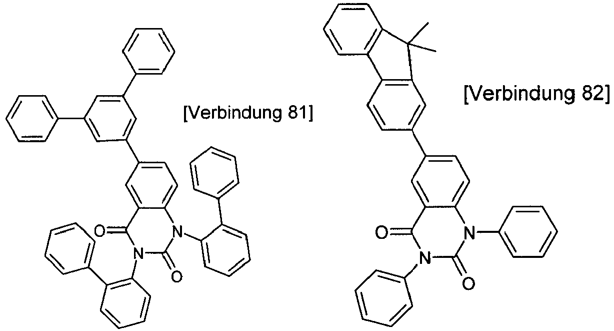

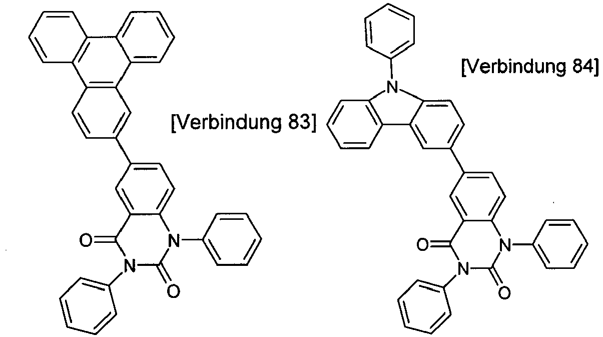

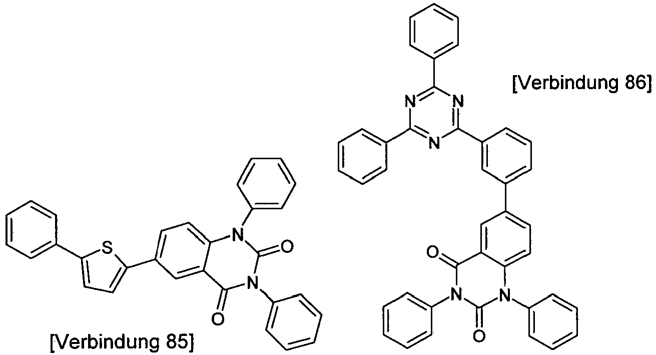

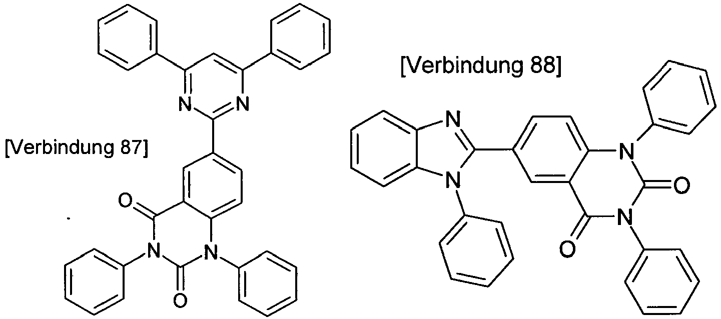

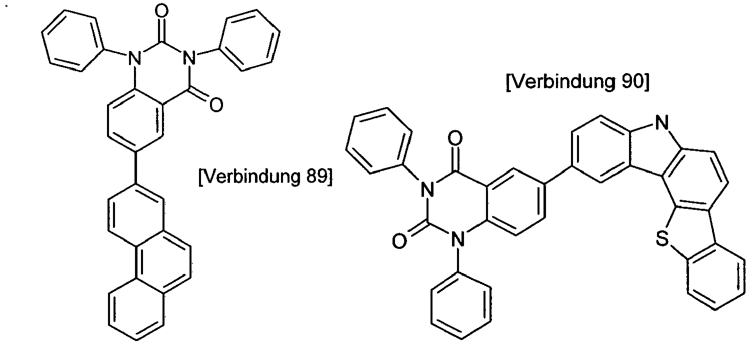

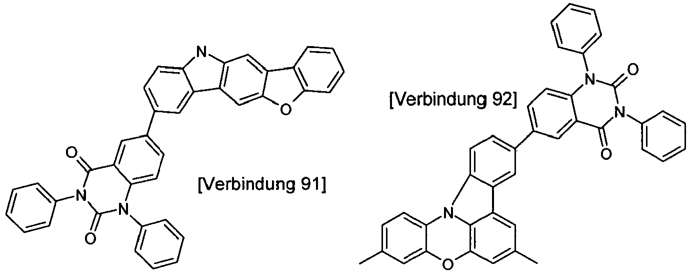

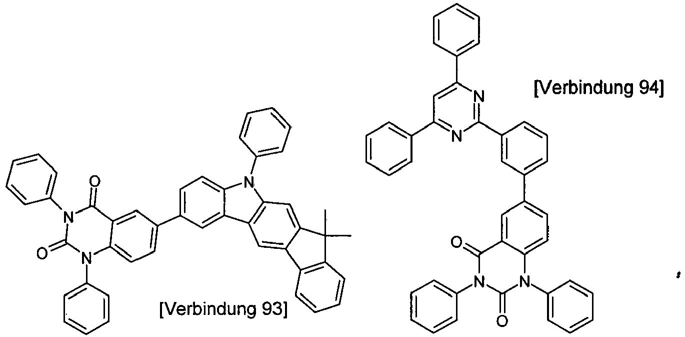

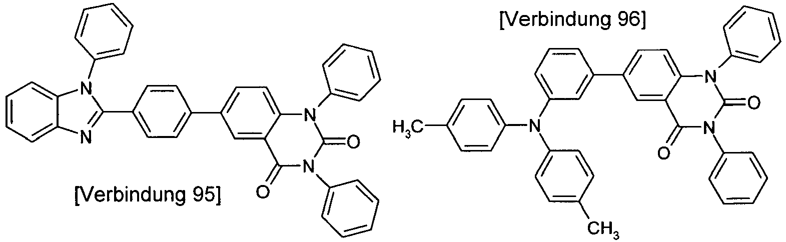

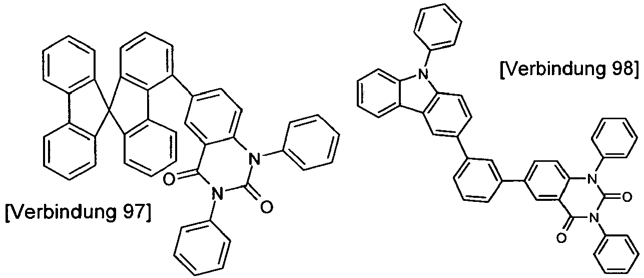

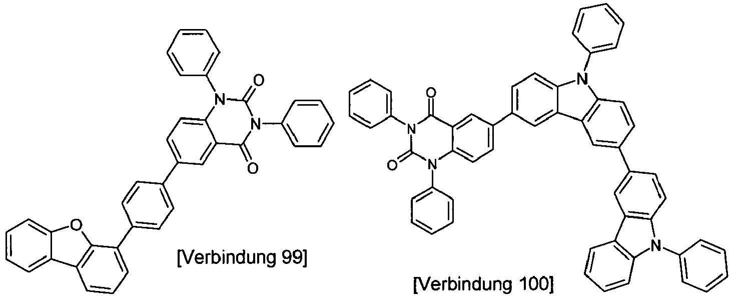

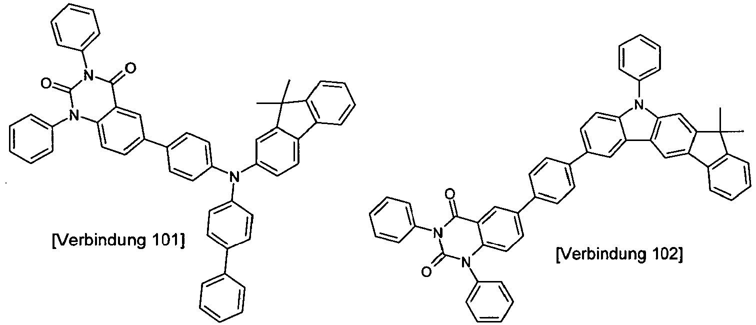

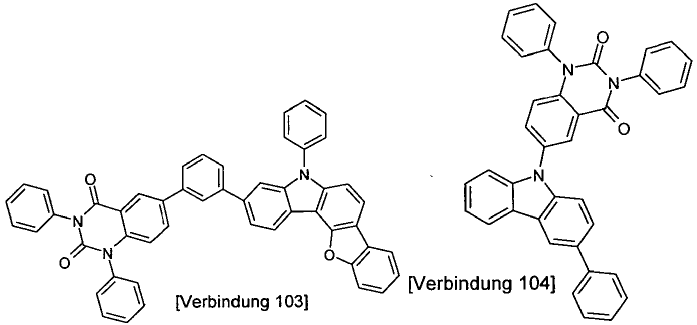

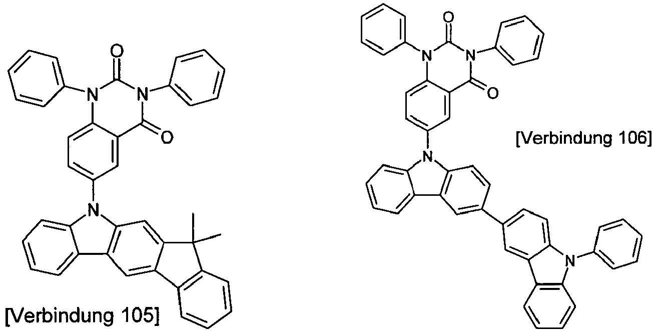

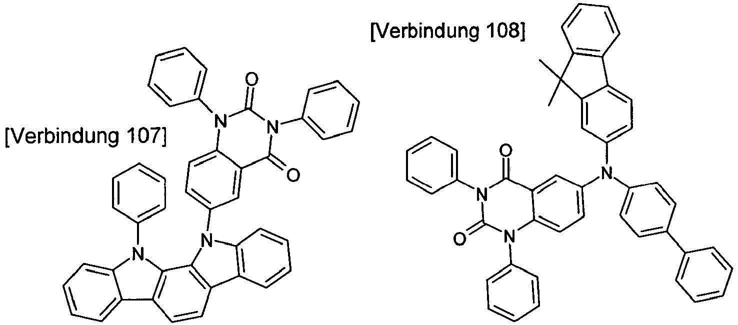

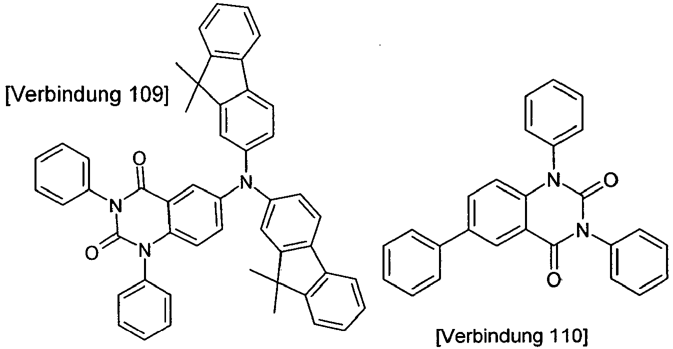

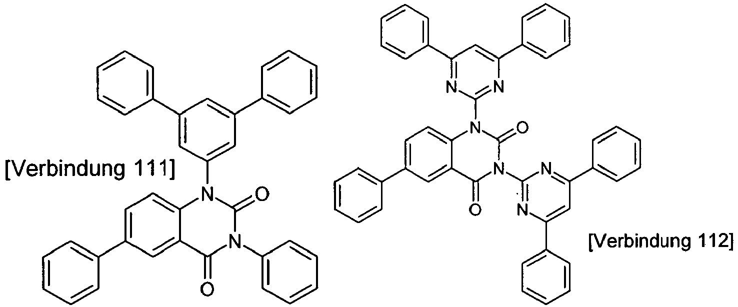

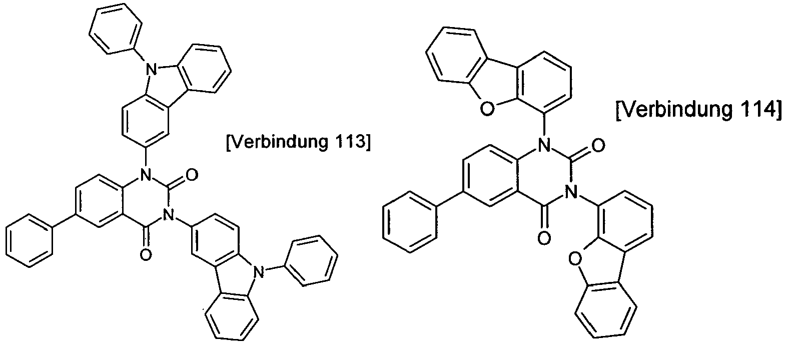

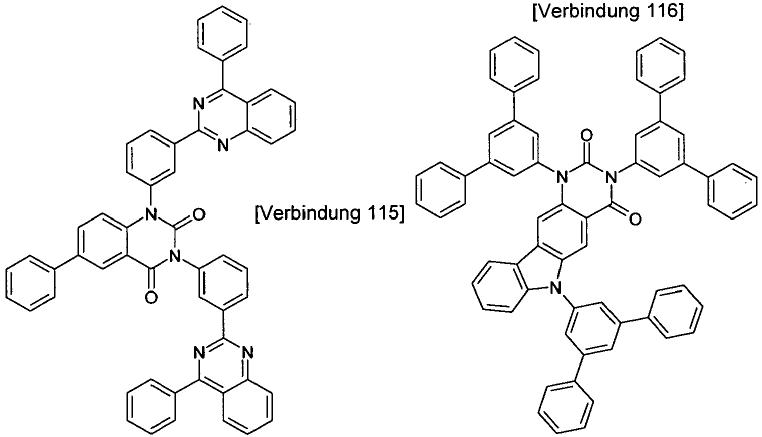

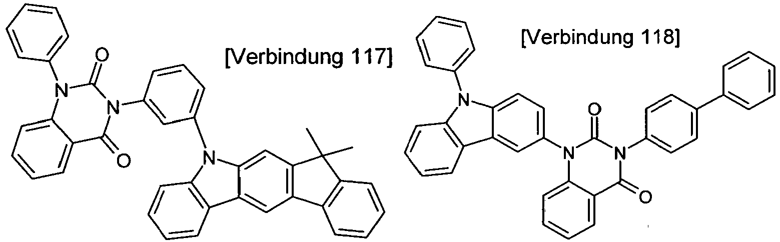

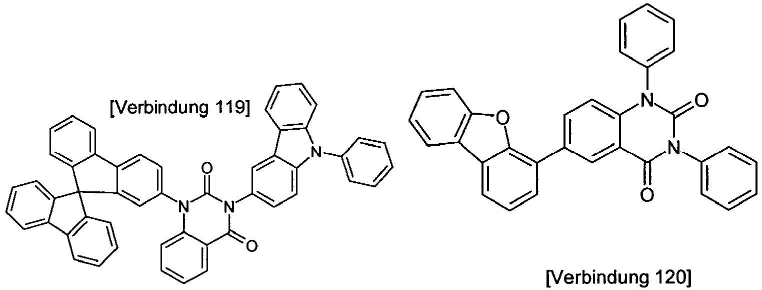

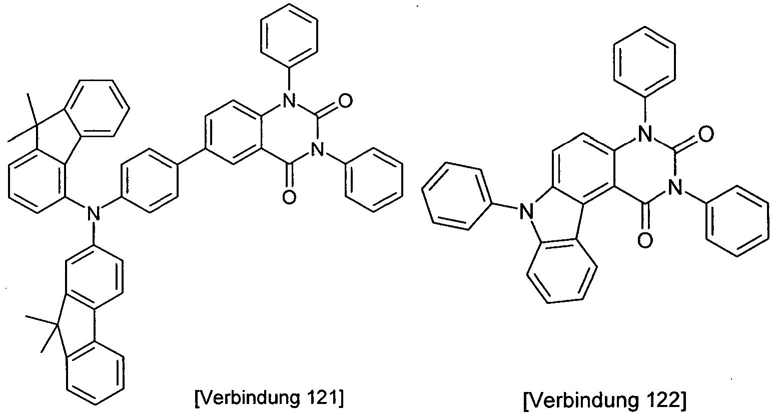

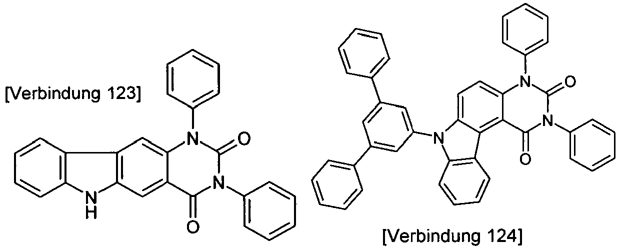

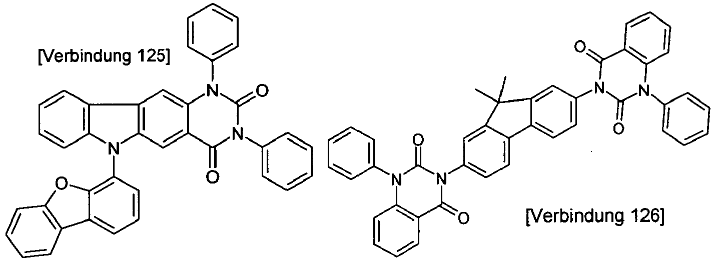

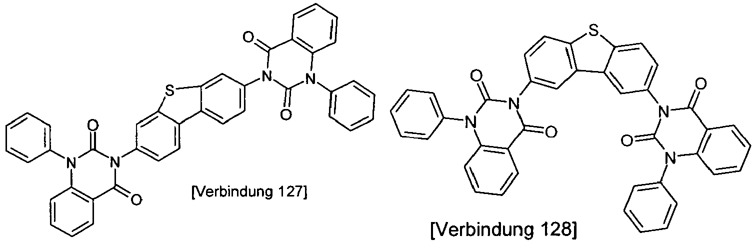

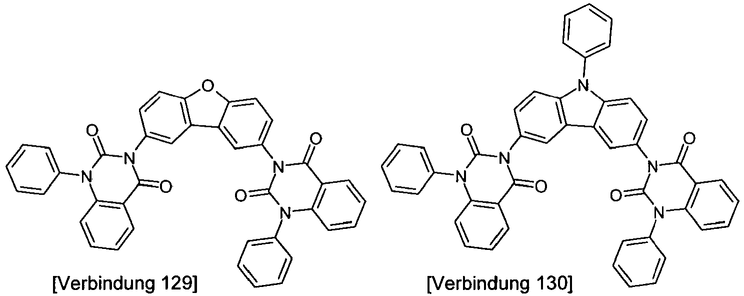

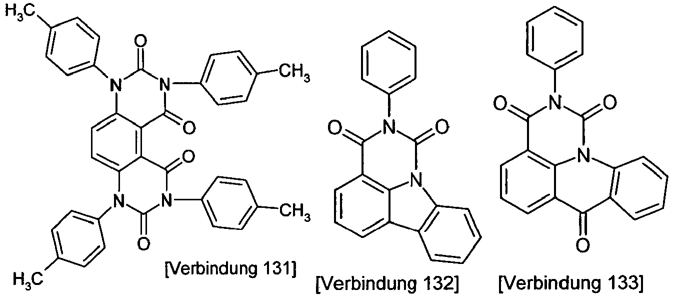

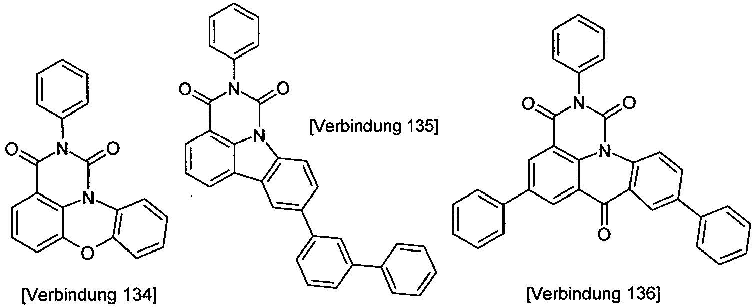

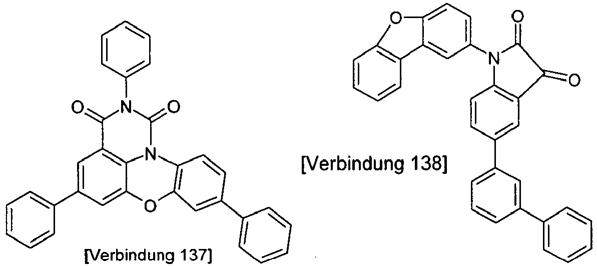

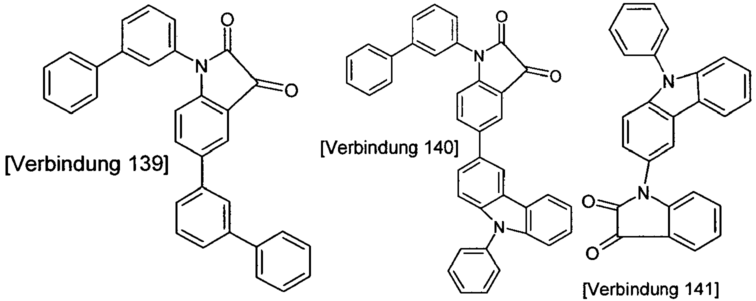

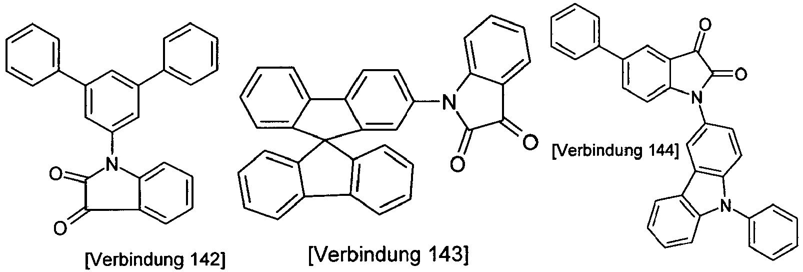

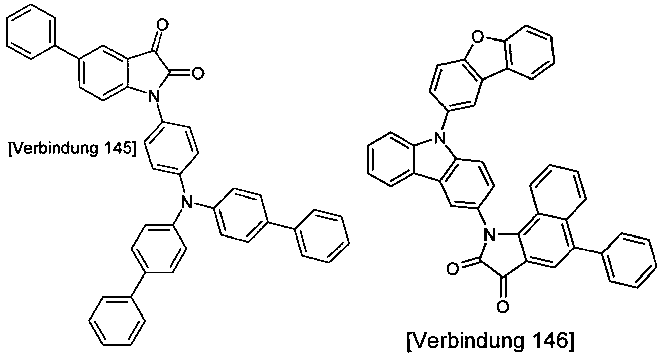

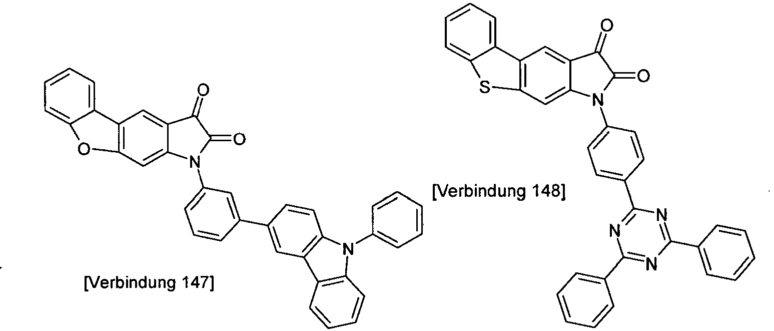

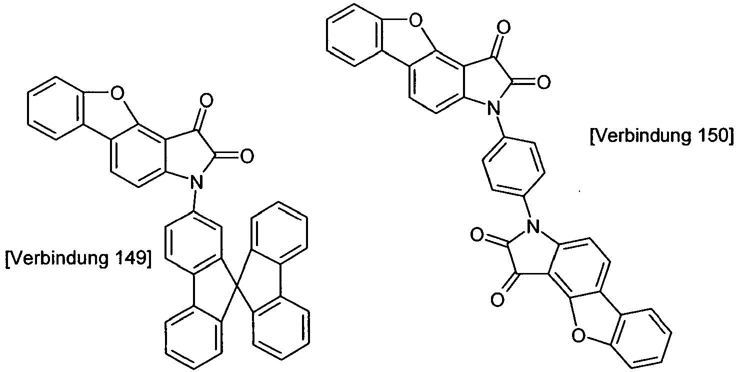

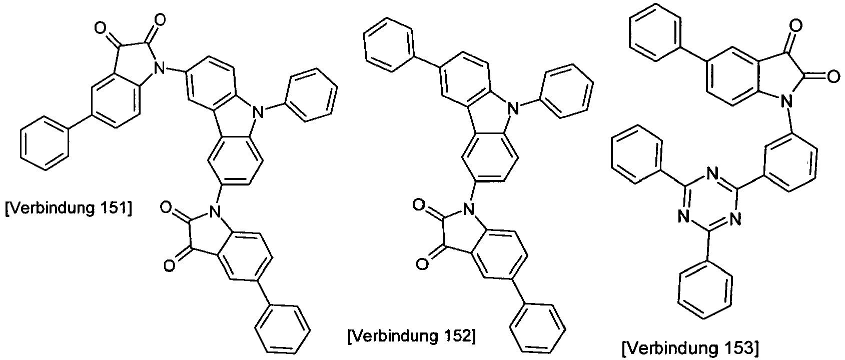

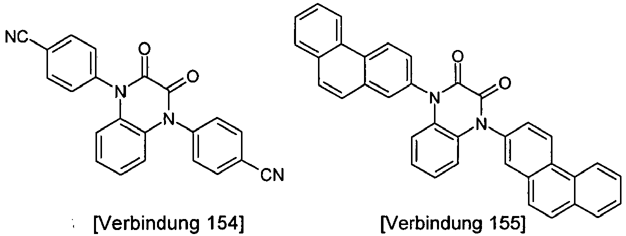

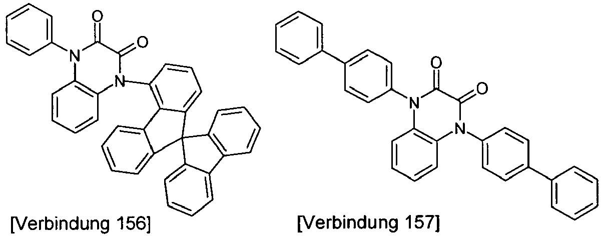

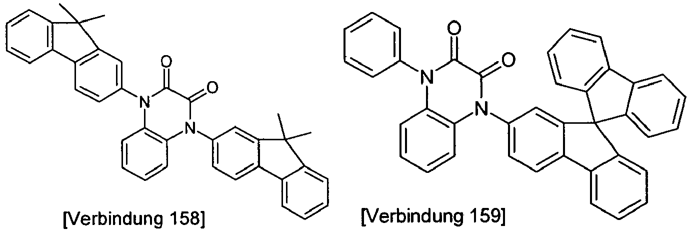

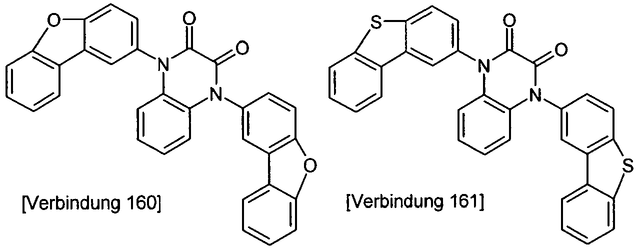

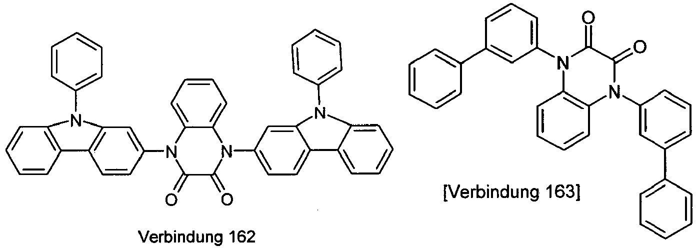

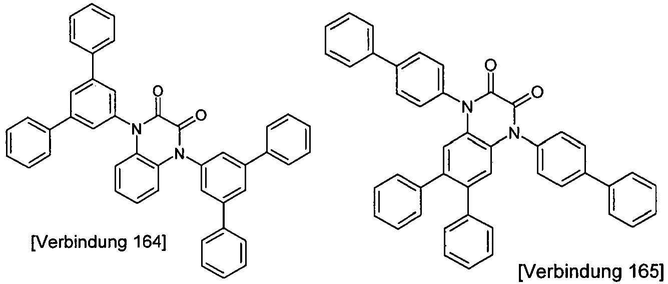

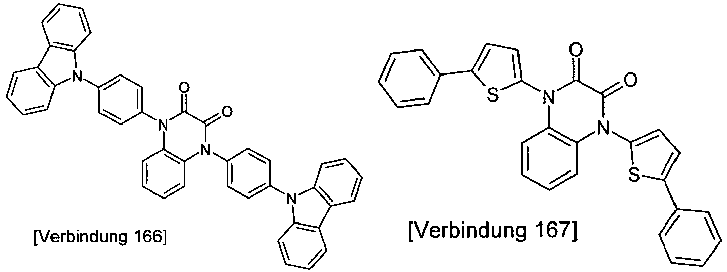

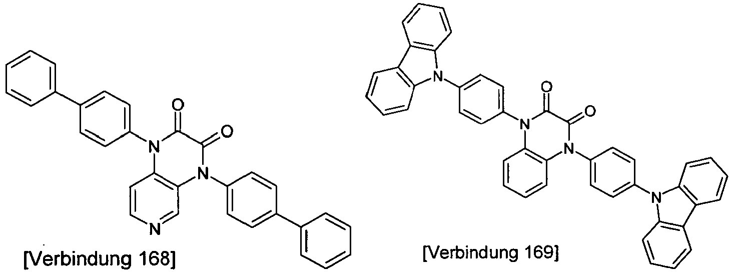

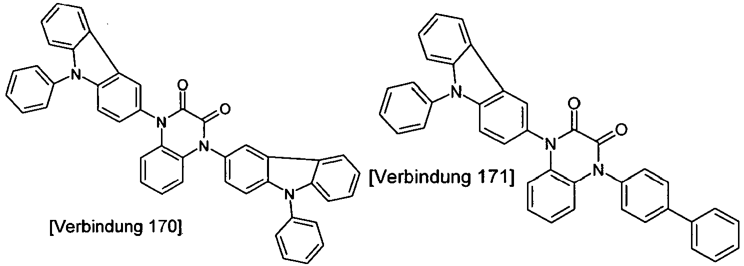

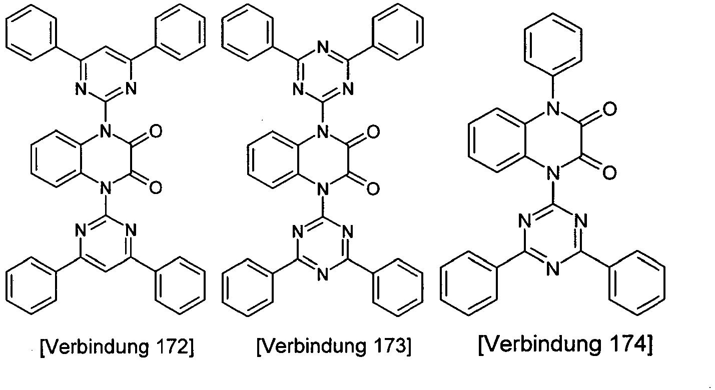

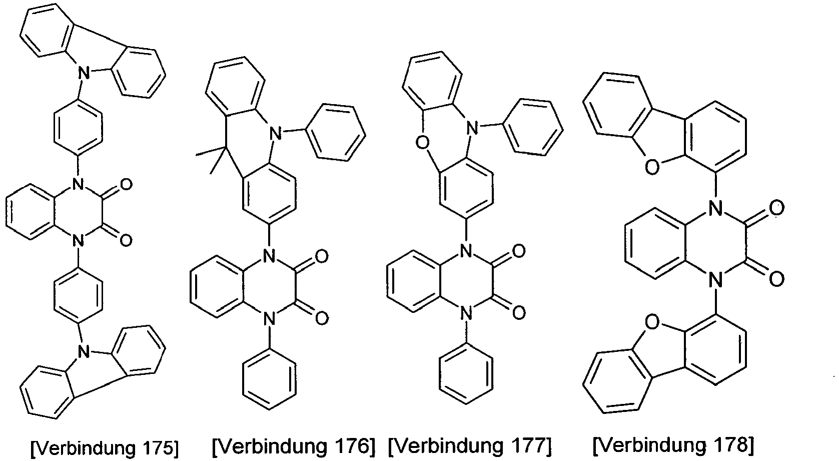

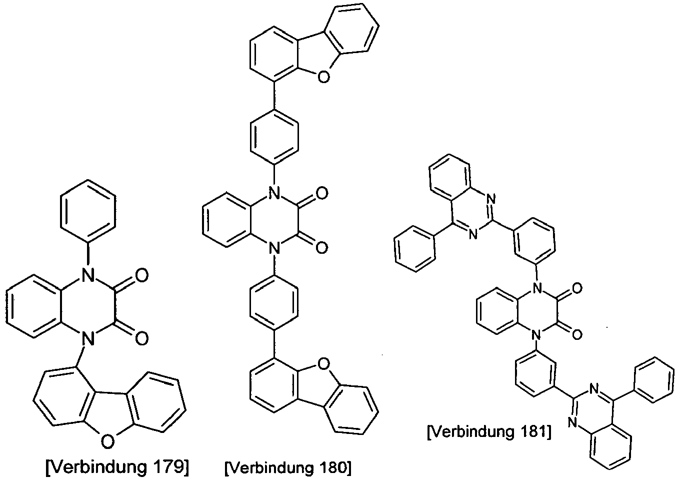

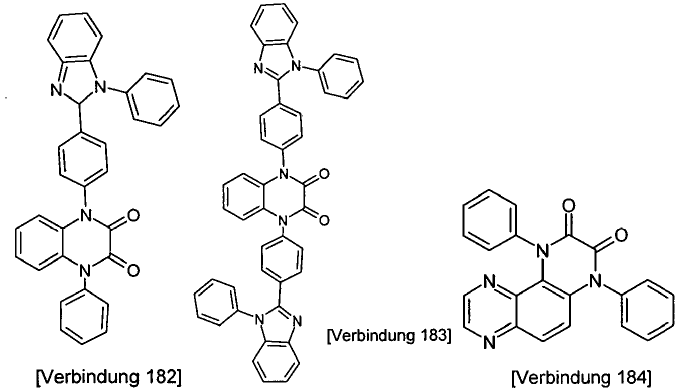

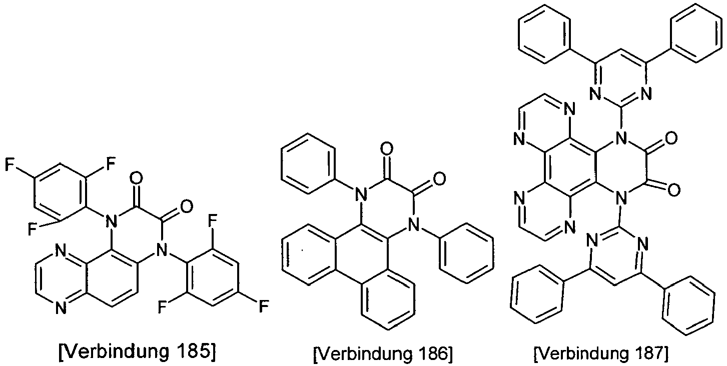

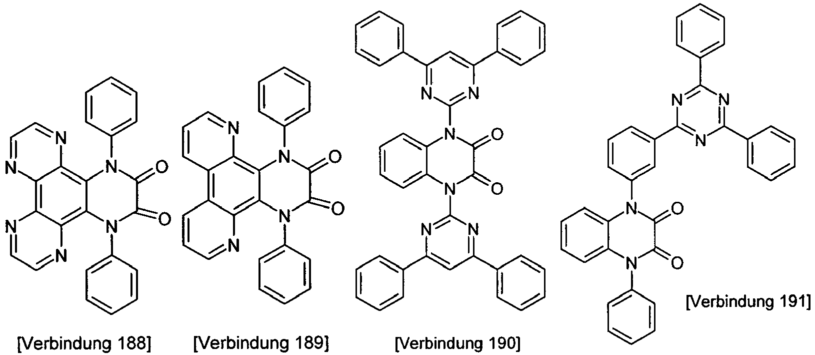

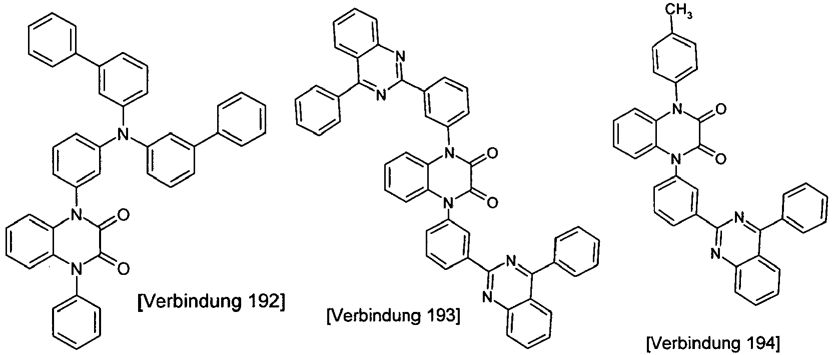

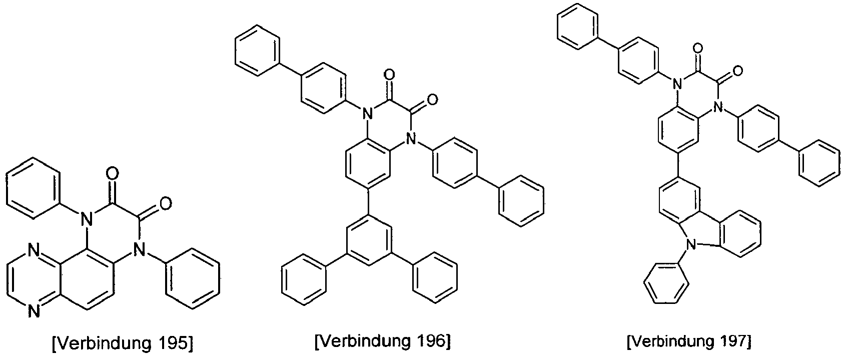

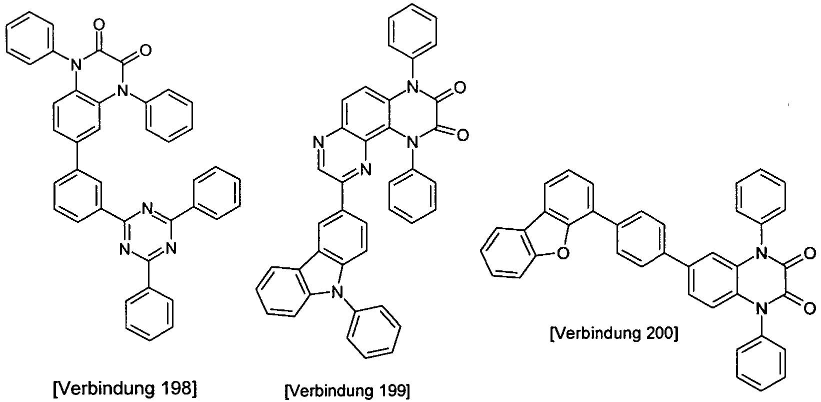

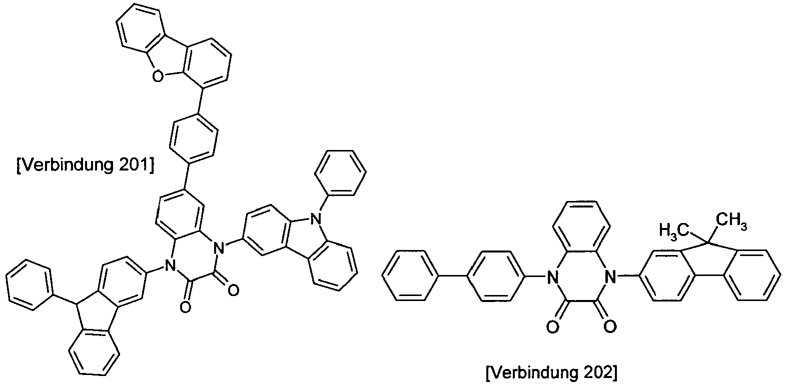

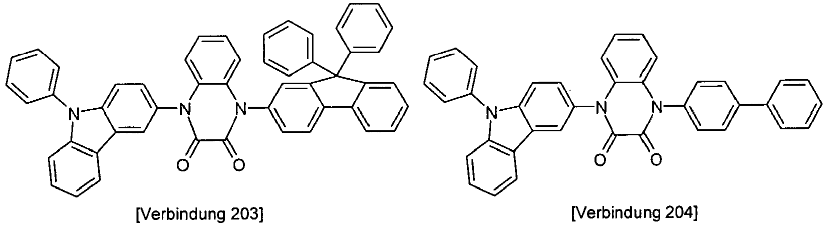

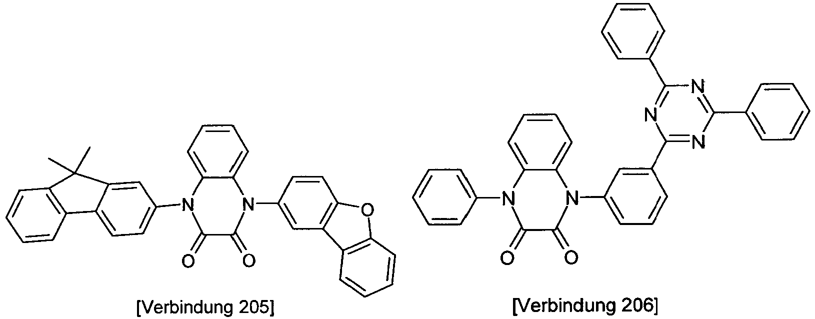

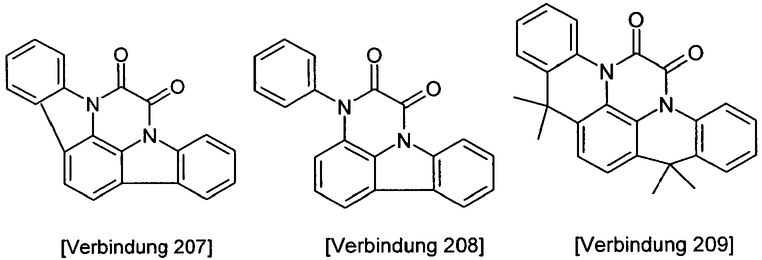

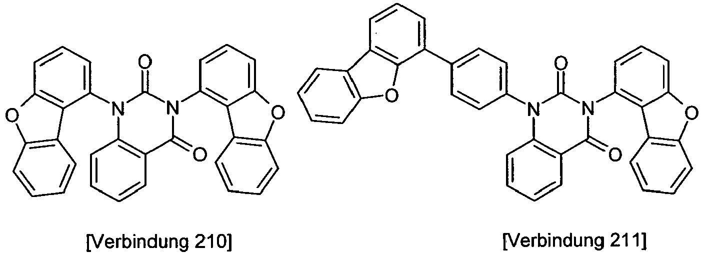

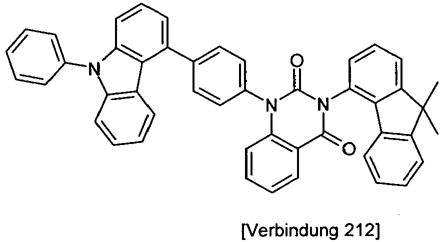

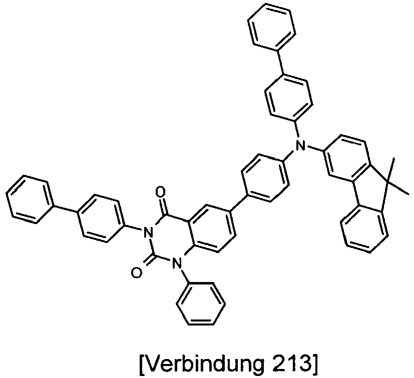

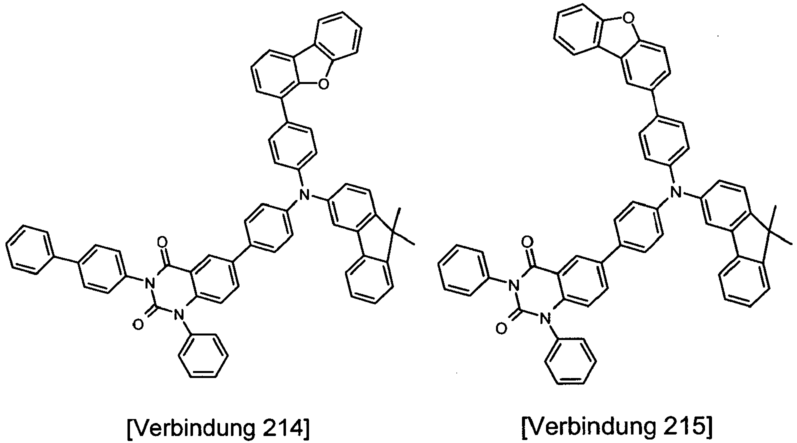

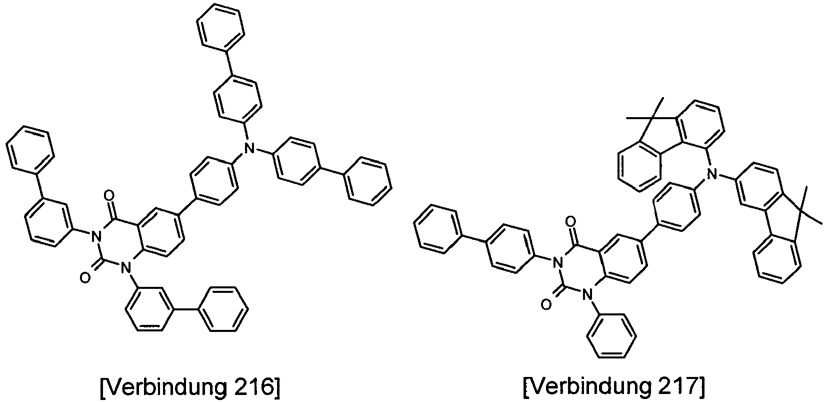

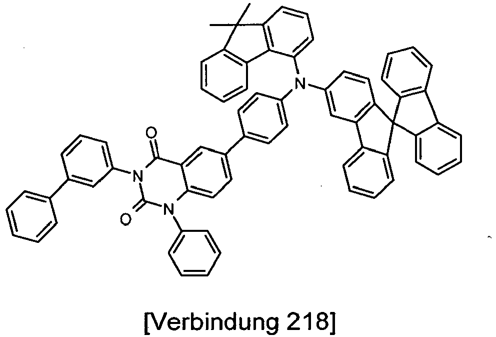

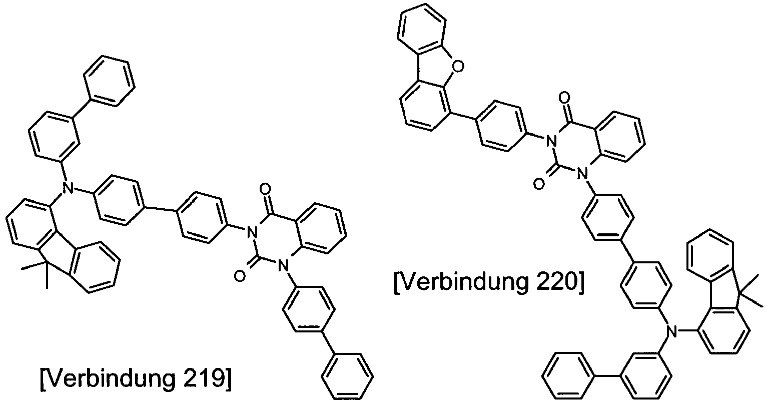

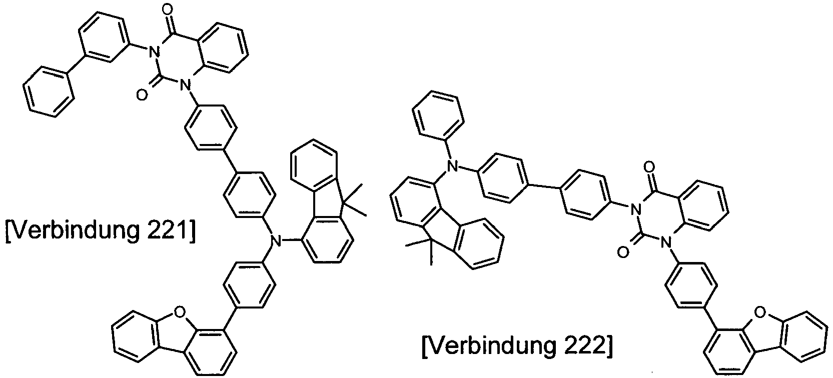

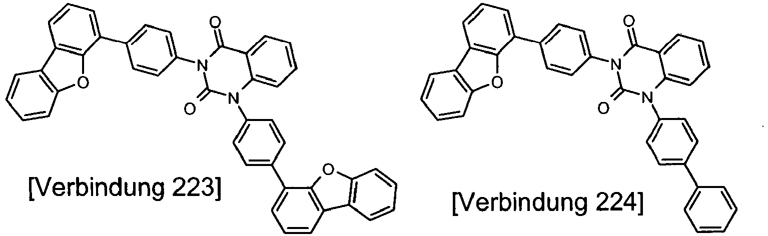

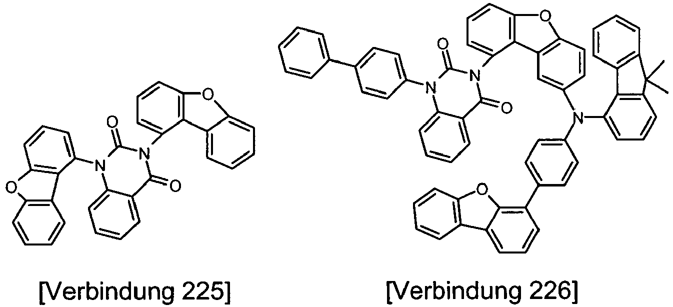

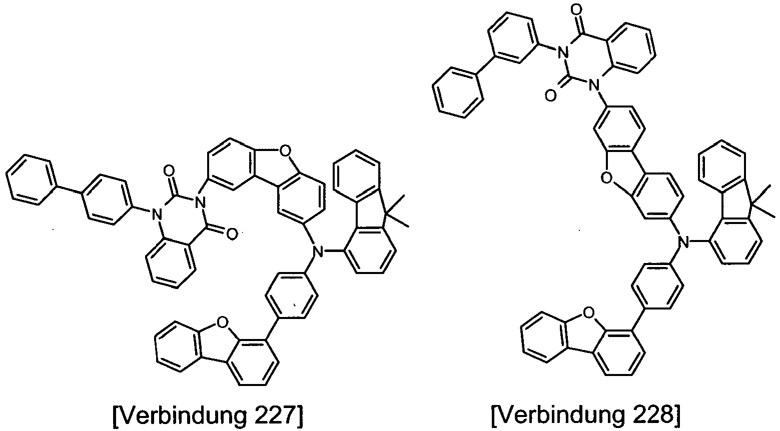

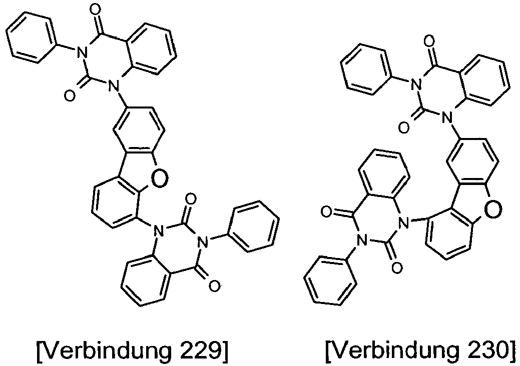

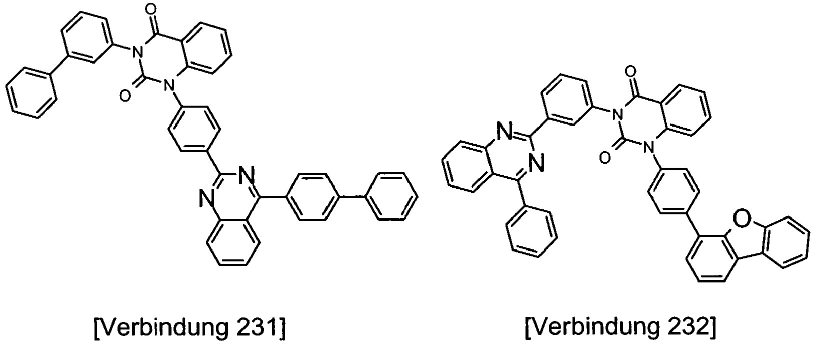

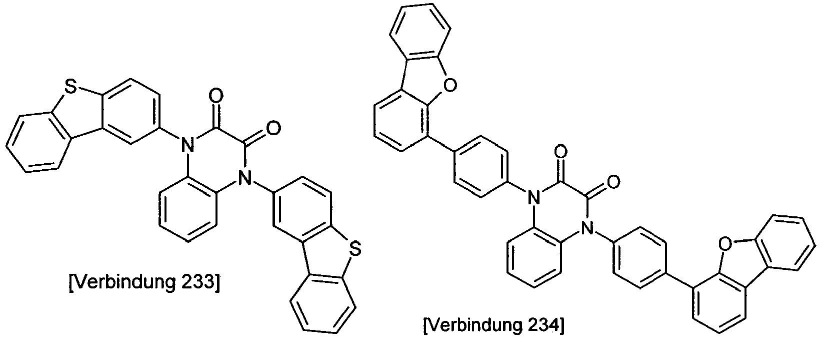

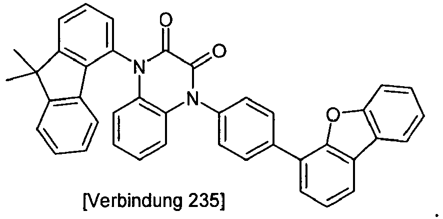

Besonders bevorzugte Einzelverbindungen für die erfindungsgemäße Verwendung in der elektronischen Vorrichtung sind die Verbindungen 1, 2, 6, 34, 55, 56, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 123, 124, 125, 141, 142, 143, 144, 145, 146, 147, 148, 149, 150, 151, 152, 153, 157, 162, 164, 170, 172, 178, 181, 184, 186, 187, 188, 199, 200, 201, 202, 203, 204, 205 und 206.

Für die Verarbeitung der erfindungsgemäßen bzw. der erfindungsgemäß zu verwendenden Verbindungen aus flüssiger Phase, beispielsweise durch Spin-Coating oder durch Druckverfahren, sind Formulierungen enthaltend die zuvor beschriebenen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethyleneglycoldimethylether, 2-Isopropylnaphthalin, Pentylbenzol, Hexylbenzol, Heptylbenzol, Octylbenzol, 1,1-Bis(3,4-dimethylphenyl)ethan oder Mischungen dieser Lösemittel.

Ein weiterer Gegenstand der vorliegenden Erfindung ist daher eine Formulierung, enthaltend mindestens eine erfindungsgemäße Verbindung der Formel (42), (47), (48), (49), (50), (51), (56) oder (57) und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch eine weitere organische oder anorganische Verbindung sein, die ebenfalls in der elektronischen Vorrichtung eingesetzt wird, beispielsweise eine emittierende Verbindung, insbesondere eine phosphoreszierende Verbindung.

Ein weiterer Gegenstand der vorliegenden Erfindung ist die Verwendung der erfindungsgemäßen Verbindungen der Formel (42), (47), (48), (49), (50), (51), (56) oder (57) in einer elektronischen Vorrichtung, insbesondere in einer organischen Elektrolumineszenzvorrichtung, wie zuvor beschrieben.

Die Ausführungen bezüglich der erfindungsgemäßen elektronischen Vorrichtung enthaltend mindestens eine Verbindung der Formel (1), gelten entsprechend auch für die speziellen Verbindungen der Formel (1), beschrieben als Verbindungen der Formel (42), (47), (48), (49), (50), (51), (56) oder (57).

In einer weiteren Ausführungsform der Erfindung enthält die organische Elektrolumineszenzvorrichtung die Verbindung gemäß Formel (1) bzw. die oben ausgeführten bevorzugten Ausführungsformen in einer optischen Auskopplungsschicht. Unter einer optischen Auskopplungsschicht wird dabei eine Schicht verstanden, die nicht zwischen der Anode und der Kathode liegt, sondern die außerhalb der eigentlichen Vorrichtung auf eine Elektrode aufgebracht wird, beispielsweise zwischen einer Elektrode und einem Substrat, um die optische Auskopplung zu verbessern.

In einer bevorzugten Ausführungsform der Erfindung wird die erfindungsgemäße Verbindung als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine Verbindung gemäß Formel (1) oder eine als bevorzugt angegebene Verbindung als Matrixmaterial enthält.

Wenn die Verbindung gemäß Formel (1) bzw. die oben ausgeführten bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand verstanden. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der Verbindung gemäß Formel (1) bzw. der oben ausgeführten bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. der oben ausgeführten bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial. Je nach Wahl des Matrixmaterials kann auch eine geringere Emitterkonzentration bevorzugt sein, wie z. B. in der nicht offen gelegten Anmeldung EP 11002816.4 beschrieben.

Eine weitere bevorzugte Ausführungsform der vorliegenden Erfindung ist der Einsatz der Verbindung gemäß Formel (1) bzw. der oben ausgeführten bevorzugten Ausführungsformen als Matrixmaterial für einen phosphoreszierenden Emitter in Kombination mit einem weiteren Matrixmaterial. Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 005/111172, Azaborole oder Boronester, z. B. gemäß WO 2006/117052, Triazinderivate, z. B. gemäß WO 2010/015306, WO 2007/063754 oder WO 2008/056746, Zinckomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951. Ebenso kann ein weiterer phosphoreszierender Emitter, welcher kürzerwellig als der eigentliche Emitter emittiert, als Co-Host in der Mischung vorhanden sein.

Als phosphoreszierende Verbindung (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In einer weiteren bevorzugten Ausführungsform der Erfindung wird die Verbindung gemäß Formel (1) bzw. die oben ausgeführten bevorzugten Ausführungsformen als Elektronentransportmaterial in einer Elektronentransport- oder Elektroneninjektionsschicht eingesetzt. Dabei kann die emittierende Schicht fluoreszierend oder phosphoreszierend sein. Wenn die Verbindung als Elektronentransportmaterial eingesetzt wird, kann es bevorzugt sein, wenn sie dotiert ist, beispielsweise mit Alkalimetallkomplexen, wie z. B. LiQ (Lithiumhydroxychinolinat).

In nochmals einer weiteren bevorzugten Ausführungsform der Erfindung wird die Verbindung gemäß Formel (1) bzw. die oben ausgeführten bevorzugten Ausführungsformen in einer Lochblockierschicht eingesetzt. Unter einer Lochblockierschicht wird eine Schicht verstanden, die auf Kathodenseite direkt an eine emittierende Schicht angrenzt.

Es ist weiterhin möglich, die Verbindung gemäß Formel (1) bzw. die oben ausgeführten bevorzugten Ausführungsformen sowohl in einer Lochblockierschicht bzw. Elektronentransportschicht als auch als Matrix in einer emittierenden Schicht zu verwenden.

In nochmals einer weiteren Ausführungsform der Erfindung wird die Verbindung gemäß Formel (1) bzw. die oben ausgeführten bevorzugten Ausführungsformen in einer Lochtransportschicht bzw. in einer Elektronenblockierschicht bzw. Exzitonenblockierschicht eingesetzt.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Diese Verfahren eignen sich insbesondere auch für Oligomere, Dendrimere und Polymere.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für fluoreszierende oder phosphoreszierende Emitter, führen zu sehr hohen Effizienzen sowie zu langen Lebensdauern. Dies gilt insbesondere, wenn die Verbindungen als Matrixmaterial für einen rot oder grün phosphoreszierenden Emitter eingesetzt werden.
2. Die erfindungsgemäßen Verbindungen weisen eine hohe thermische Stabilität auf.
3. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatzspannungen.
4. Auch bei Verwendung als Elektronentransportmaterial führen die erfindungsgemäßen Verbindungen zu sehr guten Eigenschaften in Bezug auf die Effizienz, die Lebensdauer und die Betriebsspannung von organischen Elektrolumineszenzvorrichtungen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Tri-o-tolylphosphin, Anorganika, Lösemittel) bezogen werden. Die Angaben bei den literaturbekannten Edukten sind die CAS-Nummern.

Als Ausgangsverbindung können z. B, N,N'-Diphenyl-1,2-benzoldiamin (Organic Letters 2007, 9(7), 1339-1342) oder N-Phenyl-o-phenylendiamin (Indian Journal of Pharmaceutical Sciences 2003, 65(2), 135-138) dienen.

### Beispiel a : 6-(4-Bromo-phenyl)-1H-quinazolin-2,4-dion

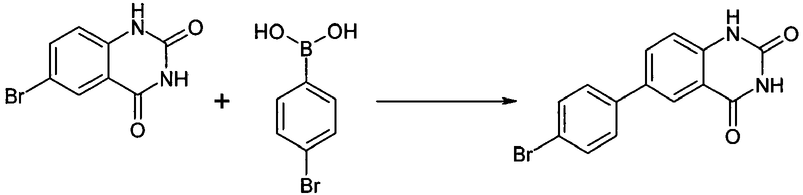

15,47 g (75 mmol) 4-Brom-benzolboronsäure, 18 g (75 mmol) 6-Brom-1H-quinazolin-2,4-dion (75 mmol) und 110 mL einer 2M NaHCO₃ enthaltenden wässrigen Lösung (163 mmol) werden in 500 mL Dimethoxyethan suspendiert. Zu dieser Suspension werden 3,0 g (3,45 mmol) Tetrakis-(triphenyl)phosphin-palladium(0) gegeben, und die Reaktionsmischung wird 22 h unter Rückfluss erhitzt. Nach Erkalten wird die organische Phase abgetrennt, über Kieselgel filtriert, viermal mit 400 mL Wasser gewaschen und anschließend zur Trockene eingeengt. Anschließend wird in Toluol umkristalisiert. Die Ausbeute beträgt 16,5 g (52 mmol), entsprechend 70 % der Theorie.

Analog dazu werden folgende Verbindungen hergestellt:

| | Edukt 1 | Edukt 2 | Produkt | Ausbeute [%] |
|---|---|---|---|---|
| a1 | 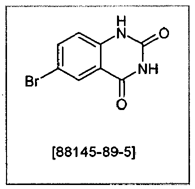 | 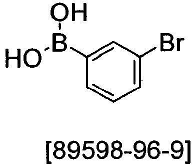 | 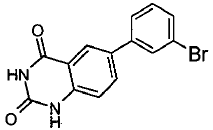 | 58% |
| a2 | 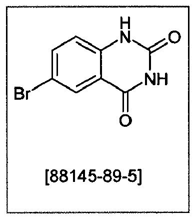 | 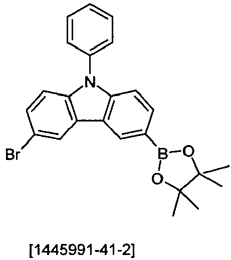 | 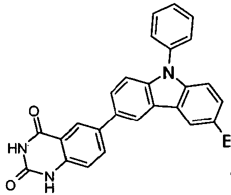 | 49% |

### Beispiel b: 6-Phenyl-1H-quinazolin-2,4-dion

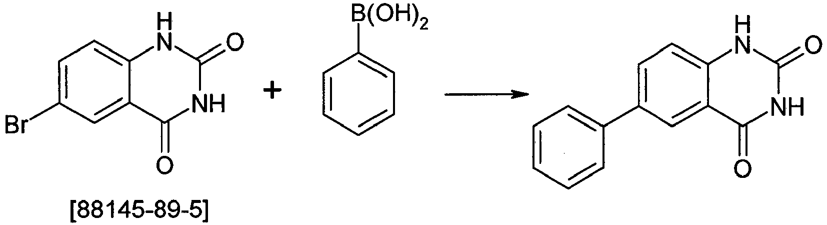

16,3 g (67,7mmol) 6-Brom-1H-quinazoline-2,4-dion, 7,3 g (80 mmol) Phenylboronsäure und 136 g (980 mmol) Trikaliumphosphat werden in 1000 mL THF, 300 mL Wasser suspendiert. Zu dieser Suspension werden 178mg (0,67 mmol) Triphenylphosphin und dann 152 mg (0.67 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol/Heptan umkristallisiert. Die Ausbeute beträgt 13,4 g (56 mmol), entsprechend 85 % der Theorie.

Analog können folgende Verbindungen erhalten werden:

| **Beispiel** | **Edukt 1** | **Edukt 2** | **Produkt** | **Ausbeute [%]** |
|---|---|---|---|---|
| **b1** | 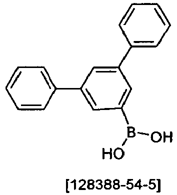 | 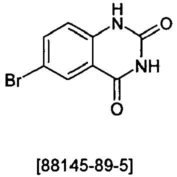 | 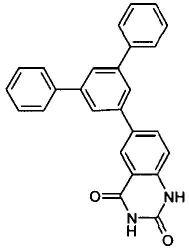 | 74 |
| **b2** | 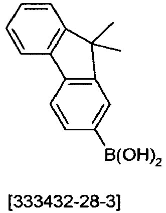 | 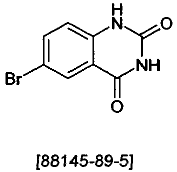 | 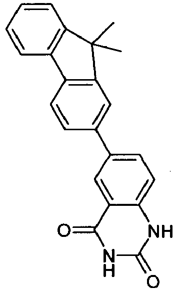 | 70 |
| **b3** | 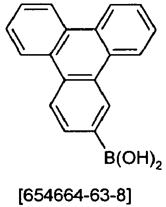 | 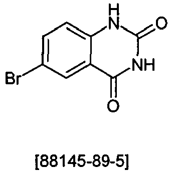 | 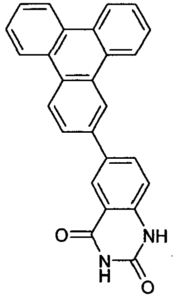 | 68 |
| **b4** | 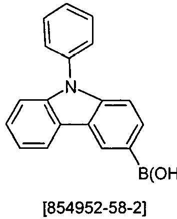 | 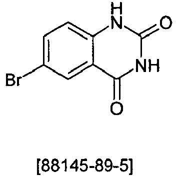 | 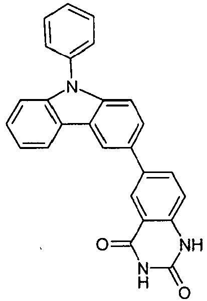 | 71 |
| **b5** | 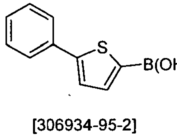 | 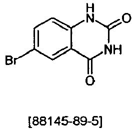 | 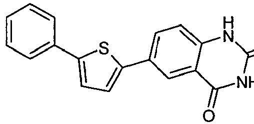 | 64 |
| **b6** | 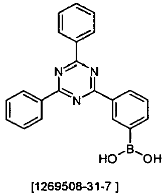 | 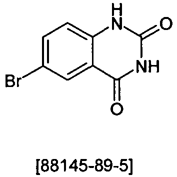 | 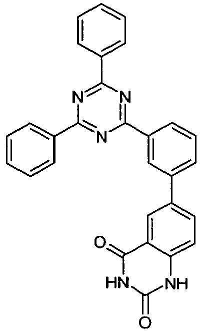 | 69 |
| **b7** | 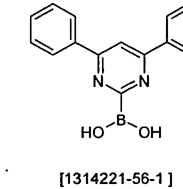 | 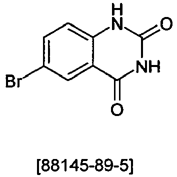 | 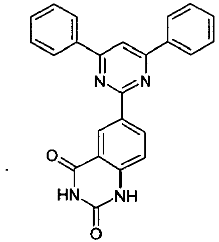 | 72 |
| **b8** | 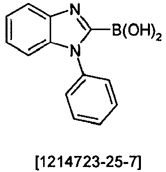 | 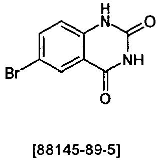 | 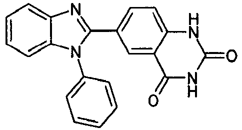 | 64 |
| **b9** | 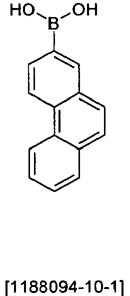 | 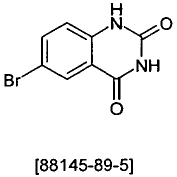 | 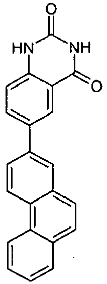 | 63 |
| **b10** | 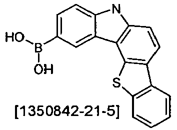 | 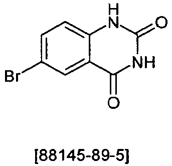 | 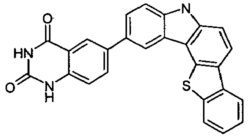 | 71 |
| **b11** | 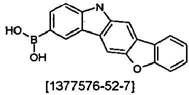 | 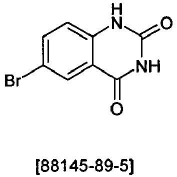 | 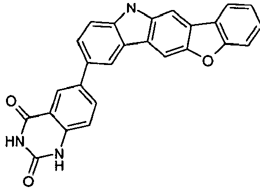 | 69 |
| **b12** | 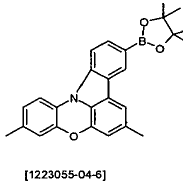 | 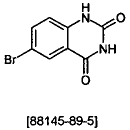 | 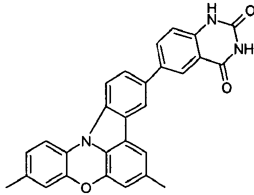 | 58 |
| **b13** | 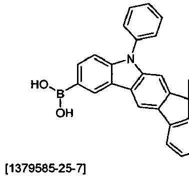 | 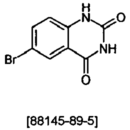 | 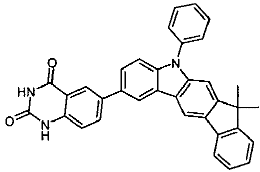 | 72 |
| **b14** | 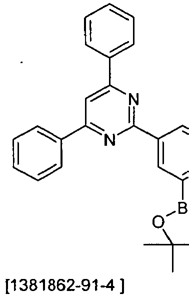 | 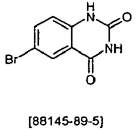 | 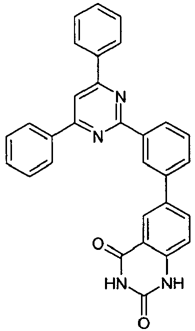 | 69 |
| **b15** | 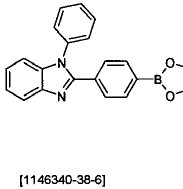 | 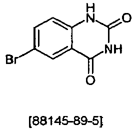 | 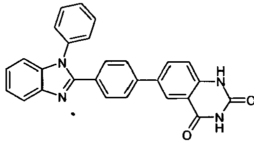 | 65 |
| **b16** | 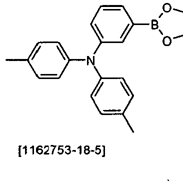 | 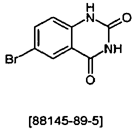 | 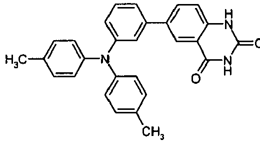 | 67 |
| **b17** | 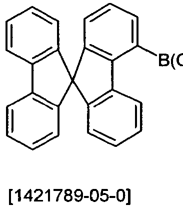 | 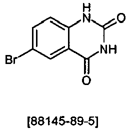 | 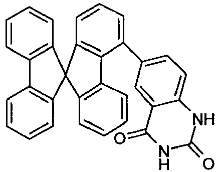 | 58 |
| **b18** | 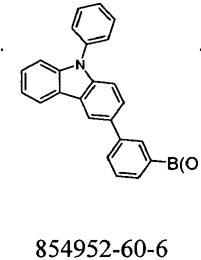 | 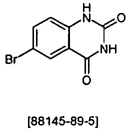 | 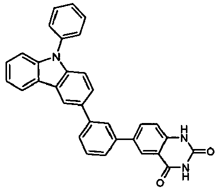 | 70 |
| **b19** | 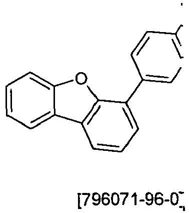 | 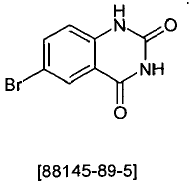 | 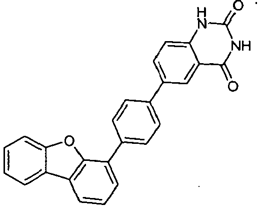 | 72 |
| **b20** | 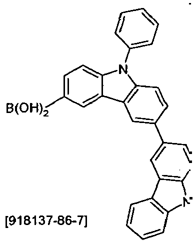 | 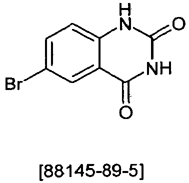 | 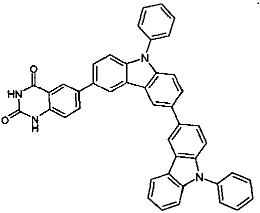 | 67 |
| **b21** | 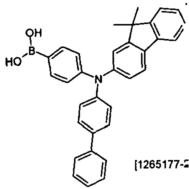 | 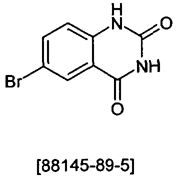 | 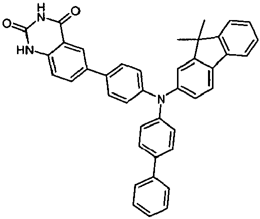 | 68 |
| **b22** | 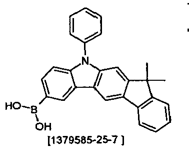 | 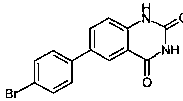 | 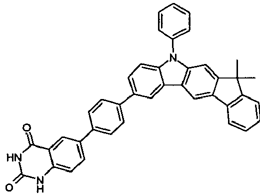 | 64 |
| **b23** | 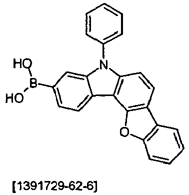 | 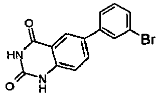 | 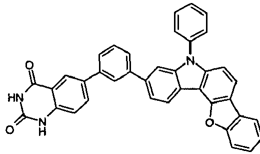 | 70 |
| **b24** | 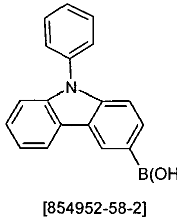 | 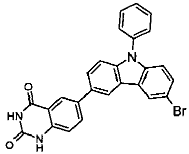 | 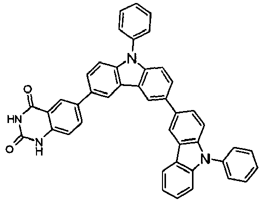 | 67 |
| **b25** | 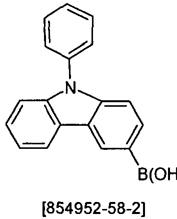 | 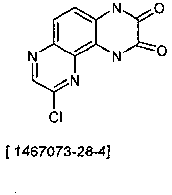 | 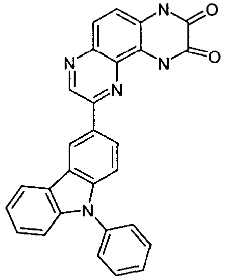 | 59 |
| **b26** | 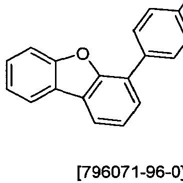 | 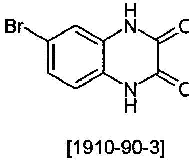 | 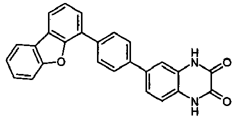 | 63 |

### Beispiel c: 6-(3-Phenyl-carbazol-9-yl)-1 H-quinazolin-2,4-dione

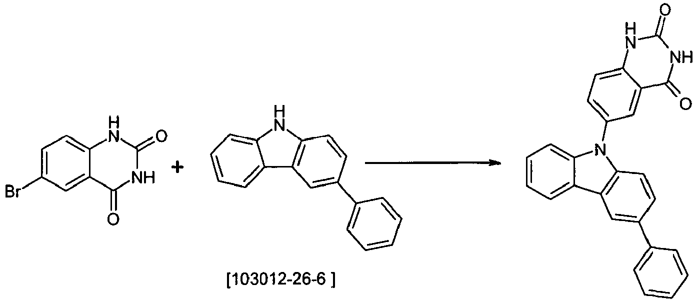

27,7 g (114 mmol) 3-Phenyl-9H-carbazol, 27,4 g (114 mmol) 6-Brom-1H-quinazoline-2,4-dion und 30,5 g NaOtBu werden in 1,5L p-Xylol, suspendiert. Zu dieser Suspension werden 0,5 g (2,11 mmol) Pd(OAc)2 und 1,6 ml eine 1M Tri.tert-butylphosohin Lösung gegeben. Die Reaktionsmischung wird 16 h unter Rückfluss erhitzt. Nach Erkalten wird die organische Phase abgetrennt dreimal mit 200 mL Wasser gewaschen und anschließend zur Trockene eingeengt. Der Rückstand wird mit Toluol heiß extrahiert und aus Toluol umkristallisiert. Die Ausbeute beträgt 39 g (97 mmol), entsprechend 87 % der Theorie.

Analog können folgende Verbindungen erhalten werden:

| **Beispiel** | **Edukt 1** | **Edukt 2** | **Produkt** | **Ausbeute [%]** |
|---|---|---|---|---|
| **c1** | 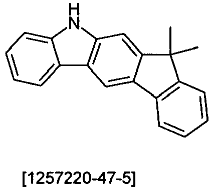 | 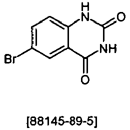 | 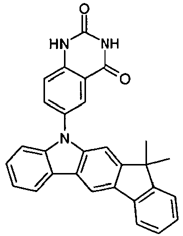 | **73** |
| **c2** | 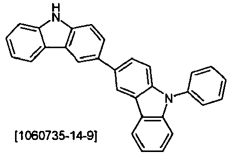 | 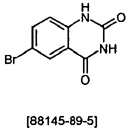 | 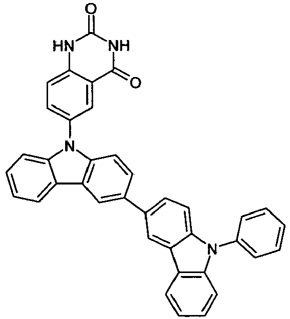 | **70** |
| **c3** | 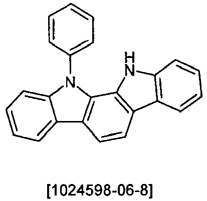 | 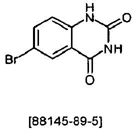 | 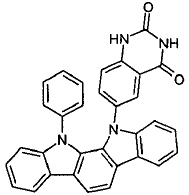 | **69** |
| **c4** | 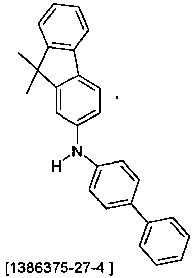 | 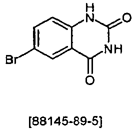 | 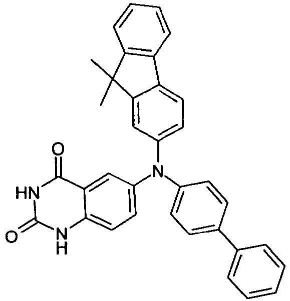 | **73** |
| **c5** | 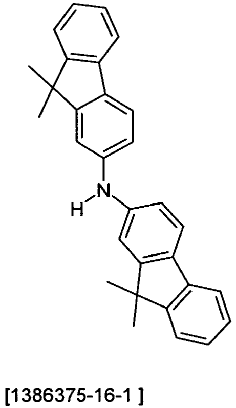 | 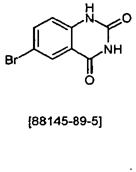 | 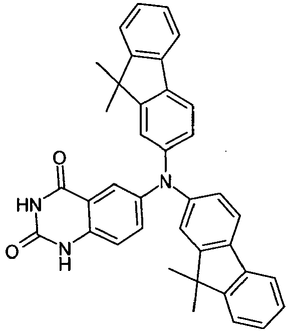 | **71** |

### Beispiel d: 1,3,6-Triphenyl-1H-quinazolin-2,4-dione

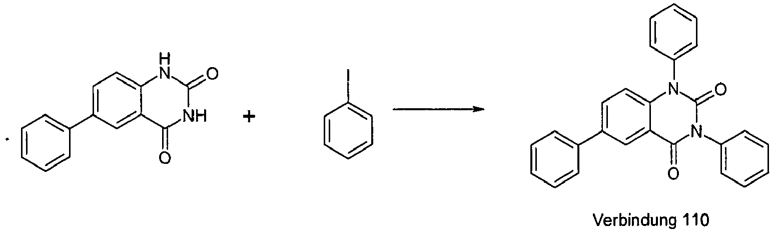

23 g (40mmol) 6-Phenyl-1H-quinazoline-2,4-dion und 61,2 g (85 mmol) 4-lodbenzol und 44,7 g (320 mmol) Kaliumcarbonat, 3 g (16 mmol) Kupfer(I])-iodid und 3,6 g (16 mmol) 1,3-Di-(pyridin-2-yl)-propan-1,3-dion werden im 100 ml DMF bei 150 °C für 30 h gerührt. Die Lösung wird mit Wasser verdünnt und mit Essigester zweimal extrahiert, die vereinigten organischen Phasen über Na₂SO₄ getrocknet und einrotiert. Der Rückstand wird chromatographisch (EtOAc/Hexan: 2/3) gereinigt. Der Rückstand wird aus Toluol umkristallisiert und abschließend im Hochvakuum (p = 5 x 10⁻⁵ mbar) sublimiert. Die Reinheit beträgt 99.9%. Die Ausbeute beträgt 24g (62 mmol) 65 % der Theorie.

Analog können folgende Verbindungen erhalten werden:

| **Bsp.** | **Edukt 1** | **Edukt 2** | **Produkt** | **Ausbeute [%]** |
|---|---|---|---|---|
| d1 | 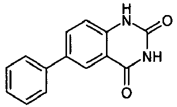 | 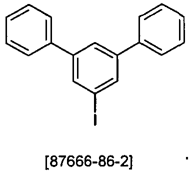 | 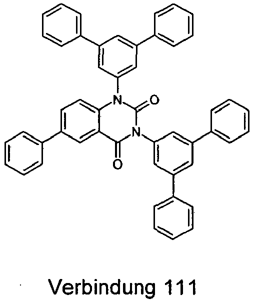 | 57 |
| d2 | 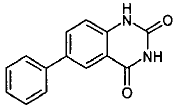 | 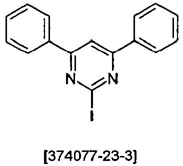 | 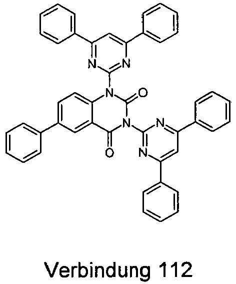 | 53 |
| d3 | 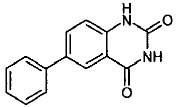 | 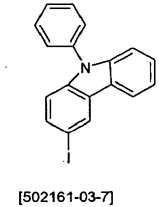 | 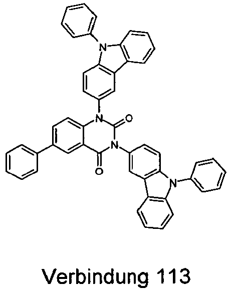 | 64 |
| d4 | 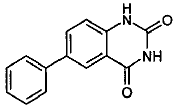 | 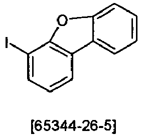 | 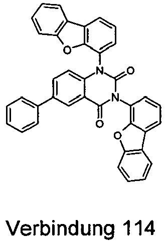 | **56** |
| d5 | 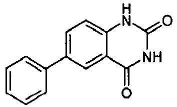 | 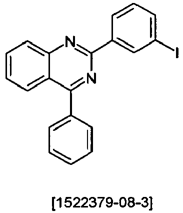 | 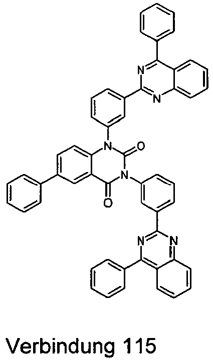 | **67** |
| d6 | 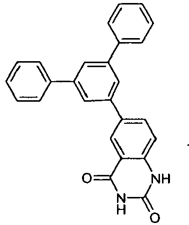 | 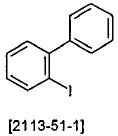 | 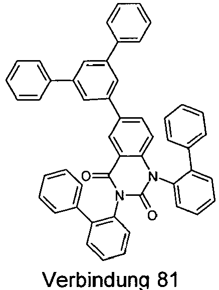 | **55** |
| d7 | 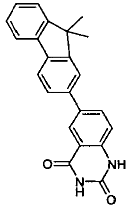 | 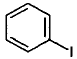 | 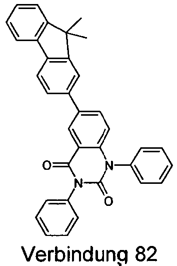 | **67** |
| d8 | 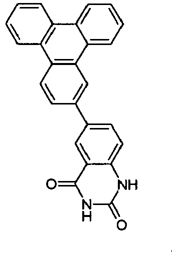 | 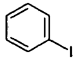 | 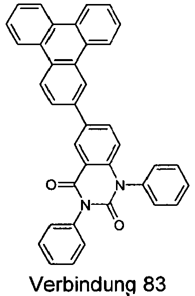 | **68** |
| d9 | 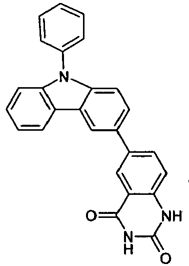 | 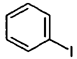 | 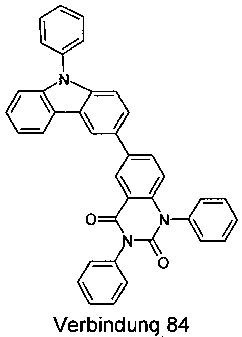 | **71** |
| d10 | 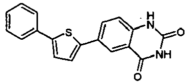 | 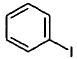 | 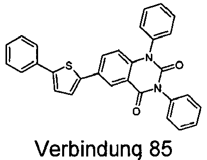 | **73** |
| d11 | 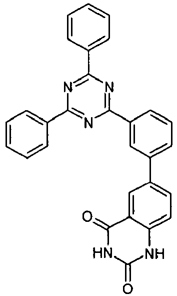 | 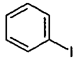 | 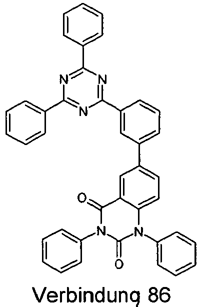 | **72** |
| d12 | 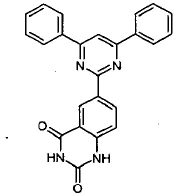 | 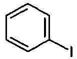 | 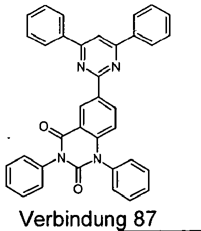 | **65** |
| d13 | 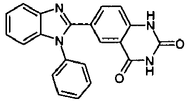 | 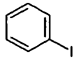 | 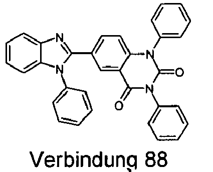 | **68** |
| d14 | 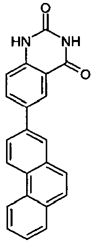 | 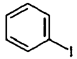 | 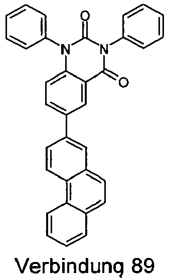 | **76** |
| d15 | 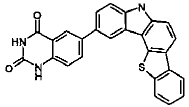 | 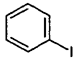 | 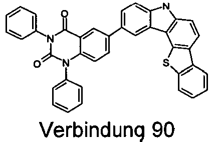 | **59** |
| d16 | 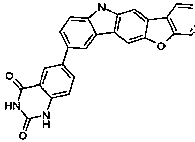 | 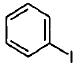 | 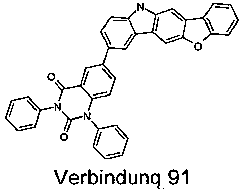 | **62** |
| d17 | 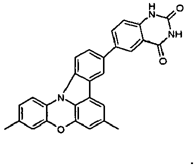 | 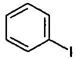 | 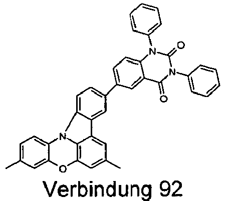 | **66** |
| d18 | 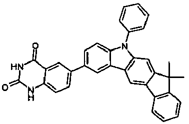 | 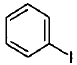 | 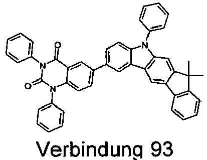 | **71** |
| d19 | 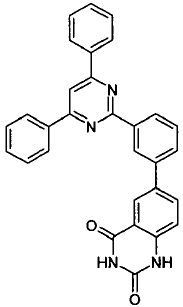 | 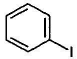 | 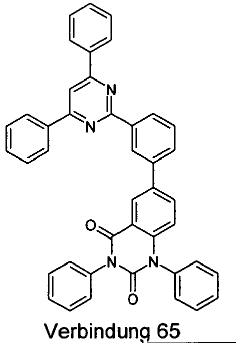 | **65** |
| d20 | 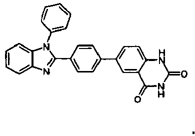 | 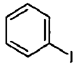 | 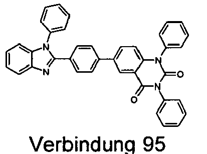 | **73** |
| d21 | 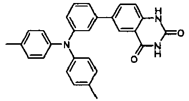 | 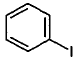 | 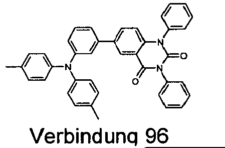 | **70** |
| d22 | 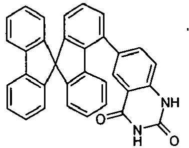 | 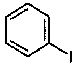 | 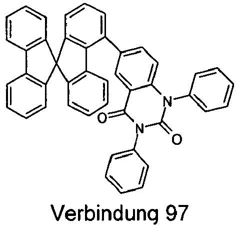 | **75** |
| d23 | 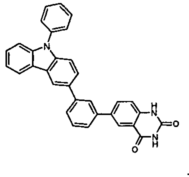 | 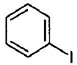 | 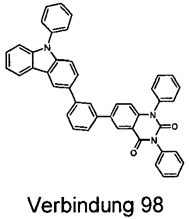 | **76** |
| d24 | 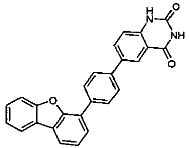 | 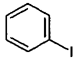 | 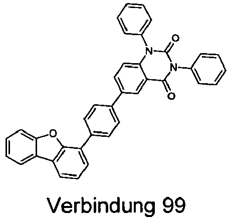 | **68** |
| d25 | 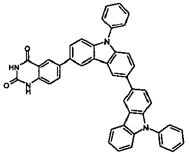 | 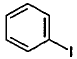 | 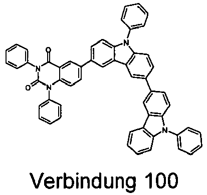 | **79** |
| d26 | 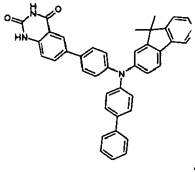 | 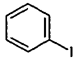 | 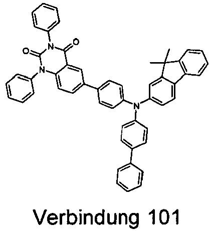 | **74** |
| d27 | 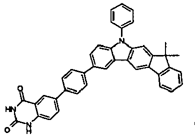 | 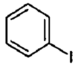 | 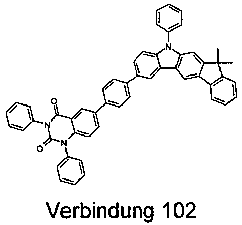 | **64** |
| d28 | 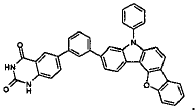 | 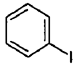 | 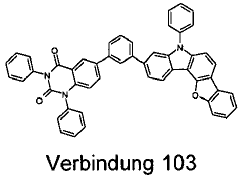 | **68** |
| d30 | 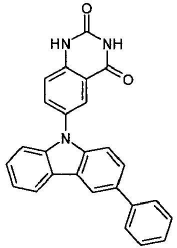 | 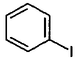 | 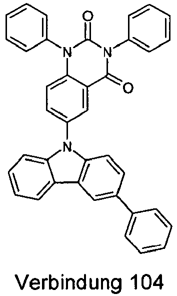 | **67** |
| d31 | 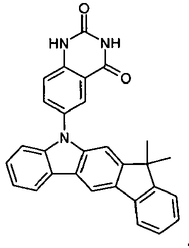 | 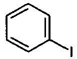 | 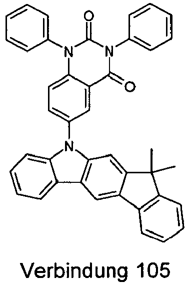 | **73** |
| d32 | 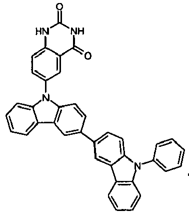 | 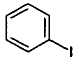 | 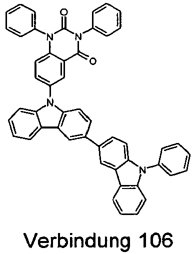 | **70** |
| d33 | 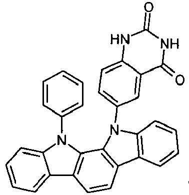 | 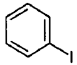 | 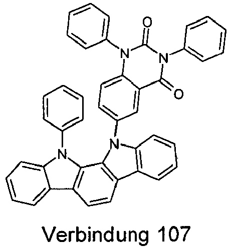 | **72** |
| d34 | 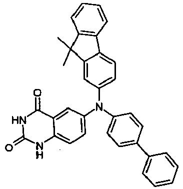 | 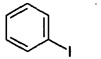 | 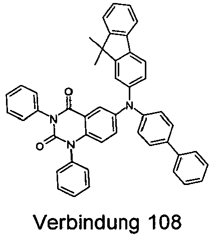 | **75** |
| d35 | 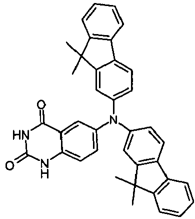 | 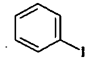 | 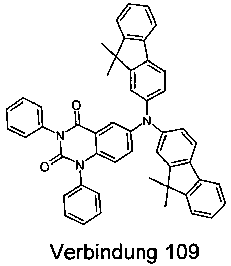 | **77** |
| d38 | 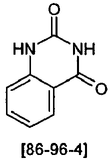 | 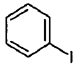 | 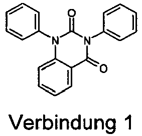 | **79** |
| **d39** | 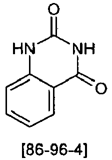 | 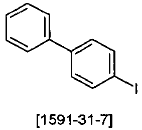 | 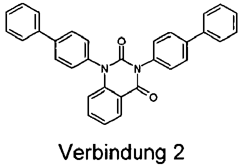 | **75** |
| **d40** | 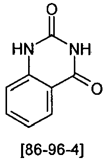 | 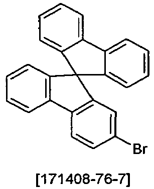 | 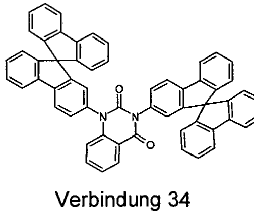 | **69** |
| **d41** | 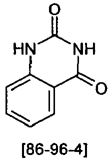 | 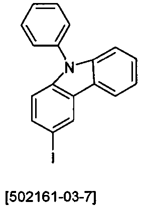 | 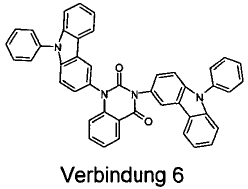 | **65** |
| **d42** | 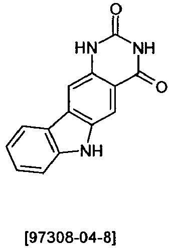 | 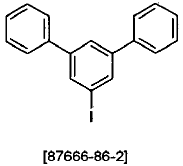 | 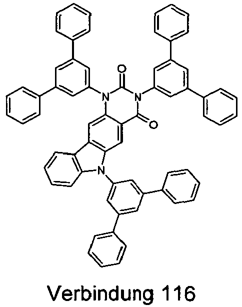 | **71** |

Analog können mit einem Äquivalent monosubstituierte Chinazolin-2,4-dion-Verbindungen hergestellt werden:

| **Bsp.** | **Edukt 1** | **Edukt 2** | **Produkt** | **Ausbeute [%]** |
|---|---|---|---|---|
| **d43** | 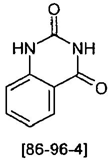 | 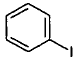 | 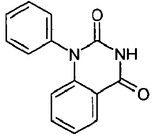 | **77** |
| **d44** | 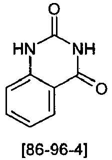 | 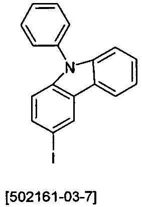 | 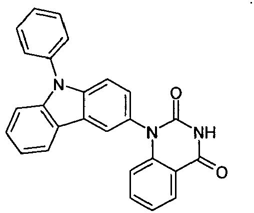 | **75** |
| **d45** | 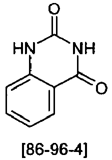 | 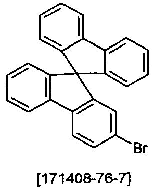 | 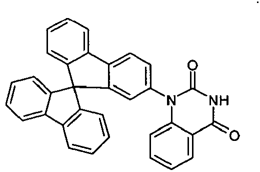 | **74** |
| **d46** | 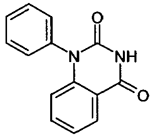 | 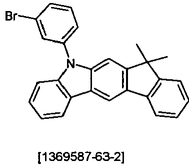 | 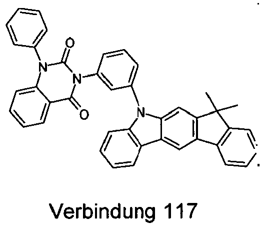 | **73** |
| **d47** | 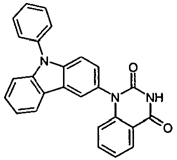 | 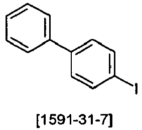 | 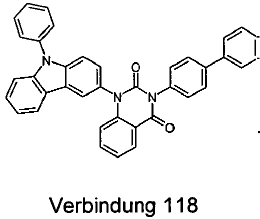 | **70** |
| **d48** | 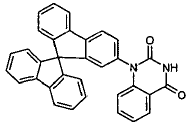 | 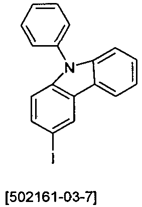 | 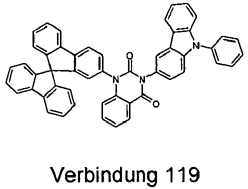 | **69** |

### Beispiel e: 1,4-Bis-[1,1';3',1"]terphenyl-5'-yl-1,4-dihydro-chinoxalin-2,3-dion

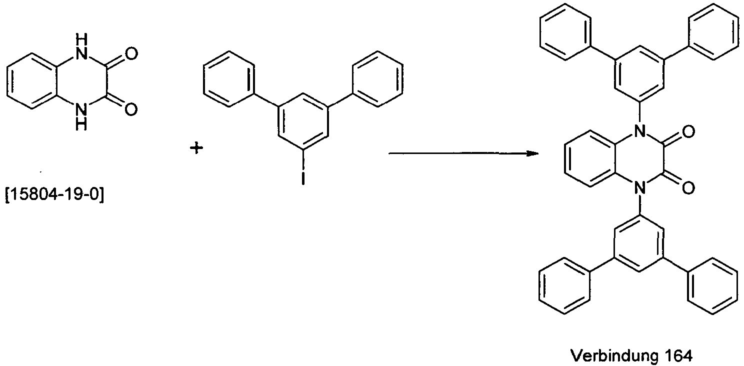

5,6g (40mmol) 6-Phenyl-1H-chinazolin-2,4-dion und 30 g (85 mmol) 4-lodbenzol und 44,7 g (320 mmol) Kaliumcarbonat, 3 g (16 mmol) Kupfer(I])-iodid und 3,6 g (16 mmol) 1,3-Di-(pyridin-2-yl)-propan-1,3-dion werden im 100 ml DMF bei 150 °C für 30 h gerührt. Die Lösung wird mit Wasser verdünnt und mit Essigester zweimal extrahiert, die vereinigten organischen Phasen über Na₂SO₄ getrocknet und einrotiert. Der Rückstand wird aus Toluol umkristallisiert und abschließend im Hochvakuum (p = 5 x 10⁻⁵ mbar) sublimiert. Die Reinheit beträgt 99.9%. Die Ausbeute beträgt 14 g (23 mmol) 61 % der Theorie.

Analog können folgende Verbindungen erhalten werden:

| **Bsp.** | **Edukt 1** | **Edukt 2** | **Produkt** | **Ausbeute [%]** |
|---|---|---|---|---|
| e1 | 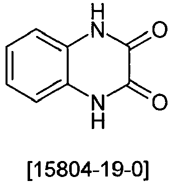 | 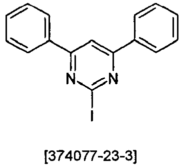 | 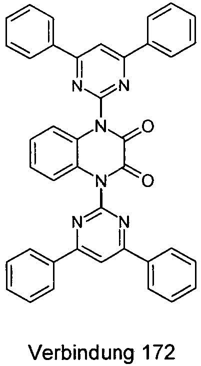 | 56 |
| e2 | 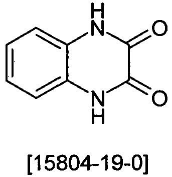 | 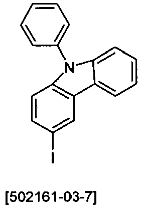 | 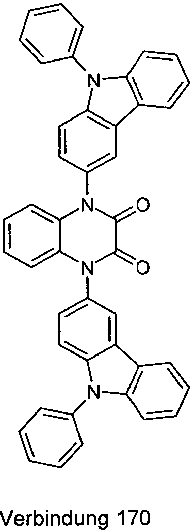 | **54** |
| e3 | 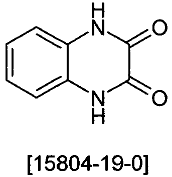 | 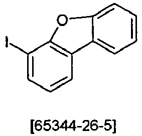 | 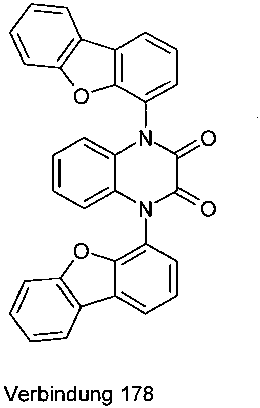 | **62** |
| e4 | 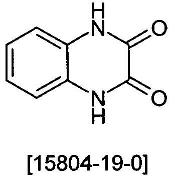 | 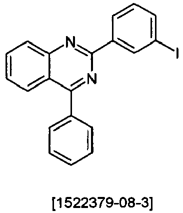 | 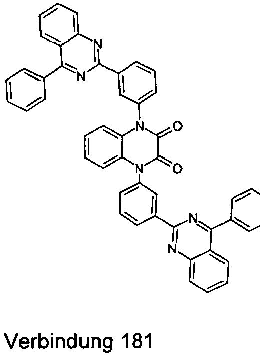 | **60** |
| e5 | 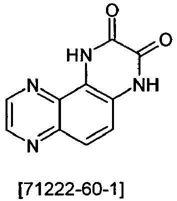 | 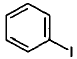 | 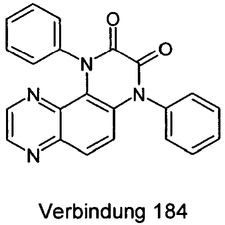 | **57** |
| e6 | 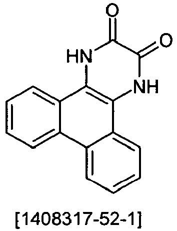 | 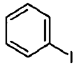 | 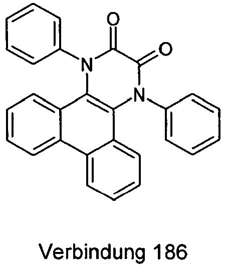 | **55** |
| e7 | 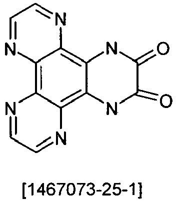 | 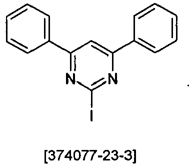 | 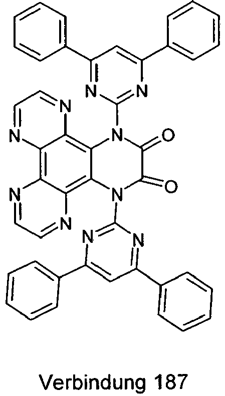 | **52** |
| e8 | 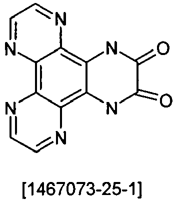 | 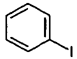 | 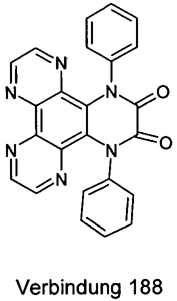 | **63** |
| e9 | 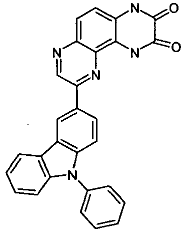 | 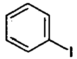 | 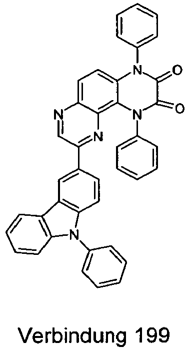 | **60** |
| e10 | 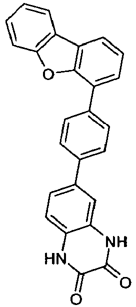 | 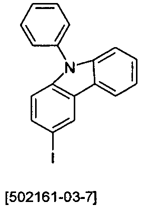 | 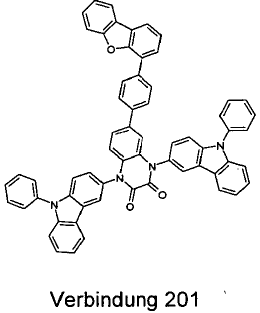 | **57** |
| e11 | 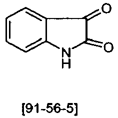 | 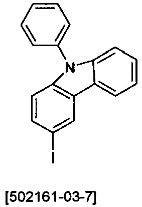 | 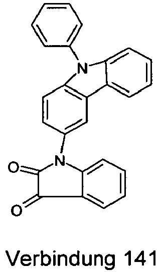 | **53** |
| e12 | 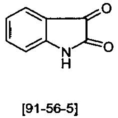 | 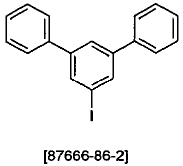 | 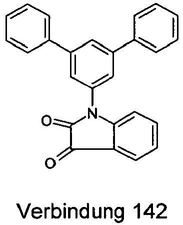 | **52** |
| e13 | 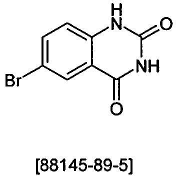 | 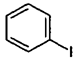 | 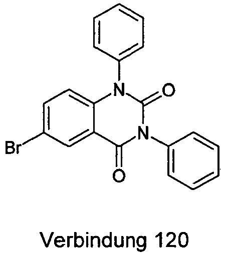 | **45** |

### Beispiel f: 1,3-Diphenyl-6-phenylamino-1H-quinazoline-2,4-dion

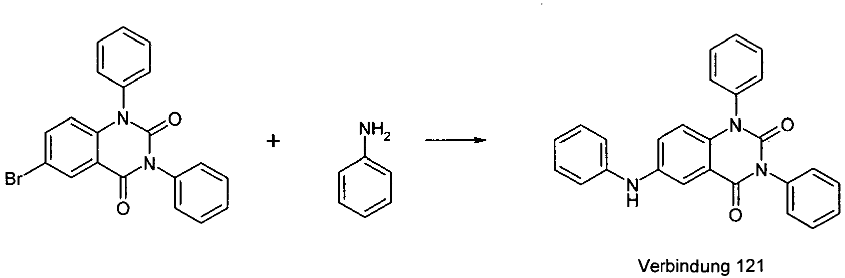

71,9 g (183 mmol) 6-Bromo-1,3-diphenyl-1H-quinazoline-2,4-dione, 20ml Anilin (220 mmol), 1,5 g DPPF (2,7 mmol), 0,5 g Palladium(II)acetat und 45 g Natrium-tert-butylat (486 mmol) werden in 1,5 I Toluol 18 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Der verbleibende Rückstand wird aus Heptan/Essigester umkristallisiert. Die Ausbeute beträgt 54 g (110 mmol, 57%).

### Beispiel g: 1,3-Diphenyl-1,6-dihydro-pyrimido[5,4-b]carbazole-2,4-dion (a) und 2,4-Diphenyl-4,7-dihydro-pyrimido[4,5-c]carbazole-1,3-dion (b)

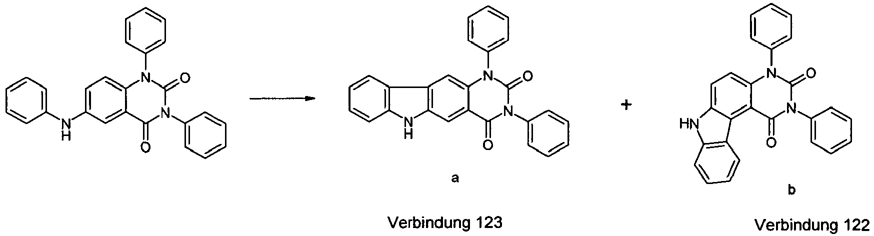

14 g (35 mmol)1,3-Diphenyl-6-phenylamino-1H-quinazoline-2,4-dion, 0,4 g Palladium(II)acetat (1,78 mmol) und 0,5 g Kaliumcarbonat (3,62 mmol) werden in 35 ml Pivalinsäure versetzt und das Gemisch bei 120 °C für 9 h gerührt. Nach dieser Zeit erfolgt die Zugabe von 0,4 g Palladium(II)acetat (1,78 mmol) und das Gemisch wird weiter bei 120 °C für 9 h gerührt. Dann werden 200 ml Dichlormethan und 0,1 M Na₂CO₃-Lösung zugegeben. Das Gemisch wird zwischen Wasser und Dichlormethan verteilt, die wässrige Phase dreimal mit Dichlormethan extrahiert, die vereinigten organische Phasen über Na₂SO₄ getrocknet und einrotiert. Der Rückstand wird chromatographisch getrennt. Die Ausbeute beträgt 3g (9,9 mmol) (a) und 9 g 29 mmol) (b).

Analog **Beispiel g** können folgende Verbindungen erhalten werden:

| **Bsp.** | **Edukt 1** | **Edukt 2** | **Produkt** | **Ausbeute [%]** |
|---|---|---|---|---|
| **g1** | 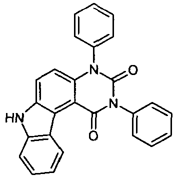 | 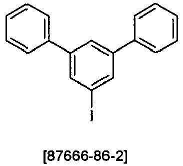 | 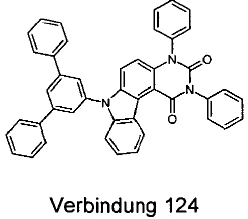 | **56** |
| **g2** | 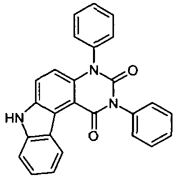 | 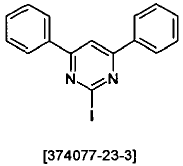 | 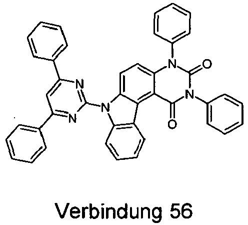 | **51** |
| **g3** | 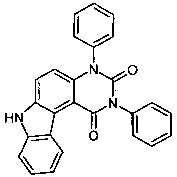 | 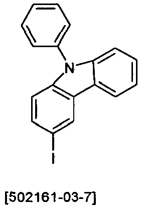 | 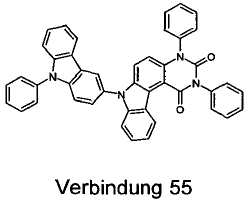 | **63** |
| **g4** | 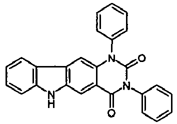 | 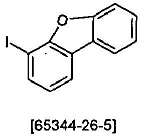 | 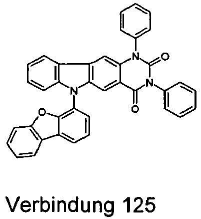 | **62** |

### Beispiel h: Allgemeine Synthese von N,N'-Diaryl-1,2-benzoldiamin

In 660 ml entgastes Toluol wird 1,06 g (4,75 mmol) Pd(OAc)₂ und 14,46 ml (14,46 mmol) Tri-*tert-*butylphosphin (1M Lösung in Toluol) zugegeben und 5 min. gerührt. Dann wird die Lösung mit 240 mmol des 1,2-Dibrombenzolderivats, 505 mmol des Arylamins und 67,22 g (700 mmol) Natrium-*tert-*butylat versetzt, nachentgast und 10 h bei 140 °C unter Schutzgasatmosphäre gerührt. Nach dem Abkühlen wird die Lösung mit 600 ml NH₄Cl-Lösung und 150 ml Essigsäureethylester versetzt, Phasen getrennt, mit Wasser gewaschen, über MgSO₄ getrocknet und eingeengt. Der Feststoff wird in Toluol gelöst und über Celite abfiltriert. Das Rohprodukt wird mit Heptan heiß ausgerührt.

### Beispiel h1: Synthese von N,N'-Bis(biphenyl-4-yl)-1,2-benzoldiamin

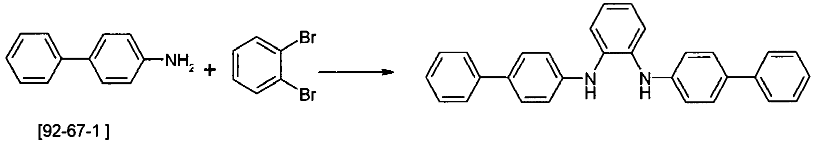

Die Synthese erfolgt nach der allgemeinen Vorschrift gemäß Beispiel h aus 56,6 g (240 mmol) 1,2-Dibrombenzol und 85,4g (505 mmol) 4-Aminobiphenyl. Der ausgefallene Feststoff wird aus Toluol/Acetonitril (5:1) umkristallisiert und der Rückstand mit MeOH gewaschen. Dabei erhält man 78 g (189 mmol) eines kristallinen Feststoffs. Die Gesamtausbeute beträgt 80 %.

Analog können folgende Verbindungen erhalten werden:

| **Bsp.** | **Edukt 1** | **Edukt 2** | **Produkt** | **Ausbeute** |
|---|---|---|---|---|
| h2 | 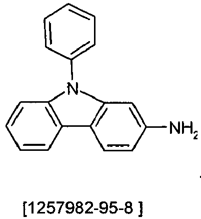 | 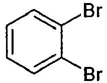 | 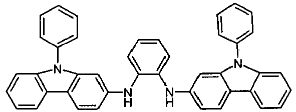 | 77% |
| h3 | 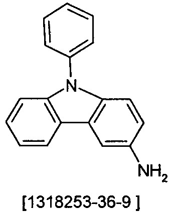 | 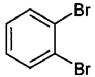 | 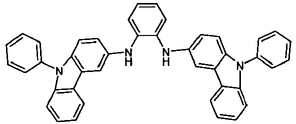 | 79% |

### Beispiel j: Synthese von Biphenyl-4-yl-(2-bromo-phenyl)-amin

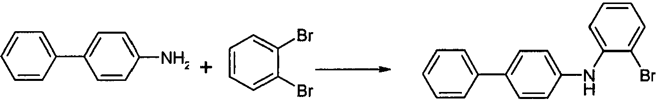

Die Synthese erfolgt nach der allgemeinen Vorschrift gemäß Beispiel h aus 118 g (700 mmol) 1,2-Dibrombenzol und 85,4g (505 mmol) 4-Aminobiphenyl. Der ausgefallene Feststoff wird aus Toluol/Acetonitril (5:1) umkristallisiert und der Rückstand mit MeOH gewaschen. Dabei erhält man 82 g (255 mmol) eines kristallinen Feststoffs. Die Gesamtausbeute beträgt 71 %.

Analog können folgende Verbindungen erhalten werden:

| **Bsp.** | **Edukt 1** | **Edukt 2** | **Produkt** | **Ausbeute** |
|---|---|---|---|---|
| j1 | 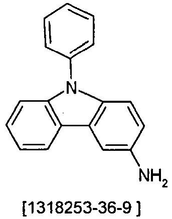 | 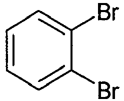 | 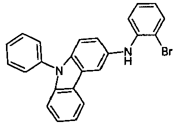 | 77% |
| j2 | 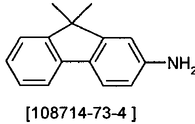 | 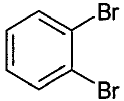 | 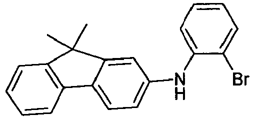 | 71% |

### Beispiel i: Synthese von Biphenyl-4-yl-(2-bromo-phenyl)-amin

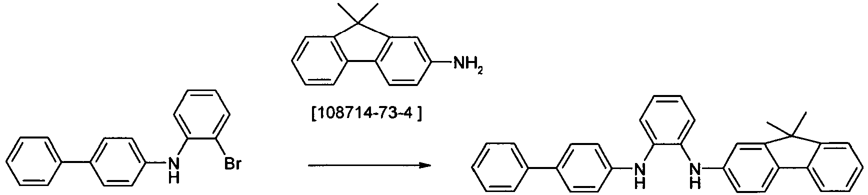

Die Synthese erfolgt nach der allgemeinen Vorschrift gemäß Beispiel h aus 105,5 g (505 mmol) Biphenyl-4-yl-(2-bromo-phenyl)-amin und 163 g (505 mmol) 9,9-Dimethyl-9H-fluoren-2-ylamine. Der ausgefallene Feststoff wird aus Toluol/Acetonitril (5:1) umkristallisiert und der Rückstand mit MeOH gewaschen. Dabei erhält man 146 g (324 mmol) eines kristallinen Feststoffs. Die Gesamtausbeute beträgt 87 %.

Analog können folgende Verbindungen erhalten werden:

| **Bsp.** | **Edukt 1** | **Edukt 2** | **Produkt** | **Ausbeute** |
|---|---|---|---|---|
| i1 | 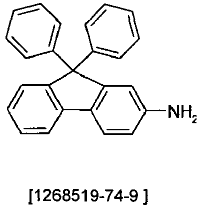 | 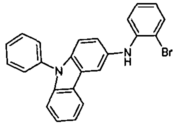 | 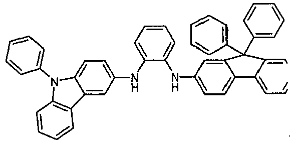 | 76% |
| i2 | 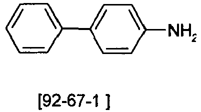 | 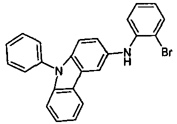 | 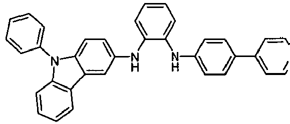 | 79% |
| i3 | 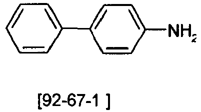 | 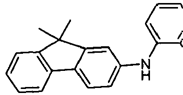 | 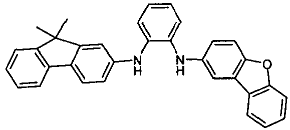 | 83% |

### Beispiel k: Synthese von N-Biphenyl-4-yl-N'-phenyl-1,2-benzoldiamin

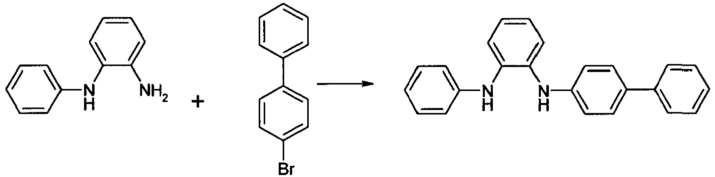

In 660 ml entgastes Toluol wird 0,35 g (1,58 mmol) Pd(OAc)₂ und 4,8 ml (4,86 mmol) Tri-*tert*-butylphosphin (1M Lösung in Toluol) zugegeben und 5 min. gerührt. Dann wird die Lösung mit 37,2 g (160 mmol) 4-Brombiphenyl, 29,4g (160 mmol) N-Phenyl-o-phenylendiamin und 22,4g (233 mmol) Natrium-*tert*-butylat versetzt, nachentgast und 10 h bei 140 °C unter Schutzgasatmosphäre gerührt. Nach dem Abkühlen wird die Lösung mit 200 ml NH₄Cl-Lösung und 50 ml Essigsäureethylester versetzt, Phasen getrennt, mit Wasser gewaschen, über MgSO₄ getrocknet und eingeengt. Der Feststoff wird in Toluol gelöst und über Celite abfiltriert. Das Rohprodukt wird mit Heptan heiß ausgerührt und mit MeOH gewaschen. Dabei erhält man 47 g (140 mmol) eines kristallinen Feststoffs. Die Gesamtausbeute beträgt 80 %.

Analog können folgende Verbindungen erhalten werden:

| **Bsp.** | **Edukt 1** | **Edukt 2** | **Produkt** | **Ausbeute** |
|---|---|---|---|---|
| ka | 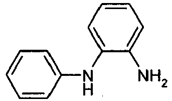 | 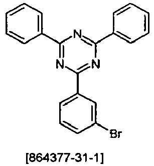 | 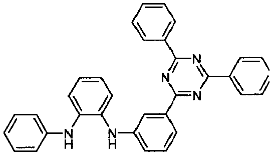 | 67% |

### Beispiel l: 1-Biphenyl-4-yl-4-{4-[(E)-((Z)-1-propenyl)-buta-1,3-dienyl]-phenyl}-1,4-dihydro-chinoxalin-2,3-dione

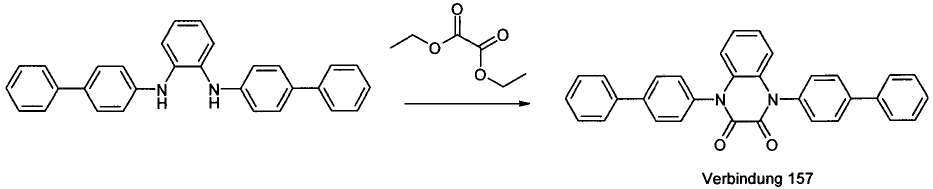

Eine Mischung aus 50 ml Oxalsäurediethylester und 78,2 g (190 mmol) N,N'-Bis-biphenyl-4-yl-benzene-1,2-diamin wird für 24 Stunden unter einer Argonatmosphäre auf 160°C erhitzt. Hierbei wird kontinuierlich der sich bildende Ethanol abdestilliert. Die Reaktionsmischung wird in Vakuum zur Trockne eingeengt und der verbleibende Rückstand zweimalig aus Ethanol umkristallisiert, Dabei erhält man 67 g (144 mmol) eines kristallinen Feststoffs. Die Gesamtausbeute beträgt 76 %

Analog können folgende Verbindungen erhalten werden:

| **Bsp.** | **Edukt 1** | **Produkt** | **Ausbeute** |
|---|---|---|---|
| I1 | 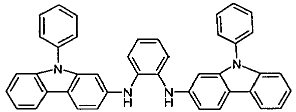 | 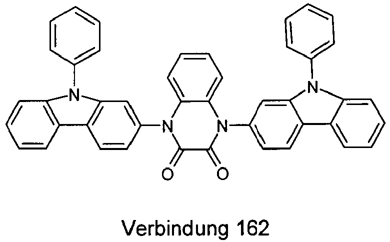 | 70% |
| I2 | 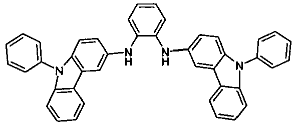 | 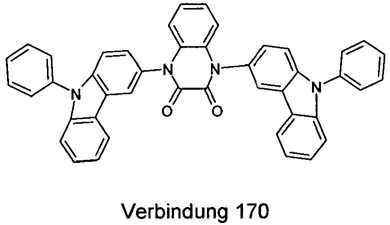 | 72% |
| I3 | 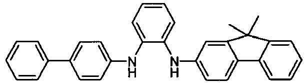 | 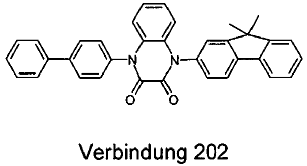 | 75% |
| I4 | 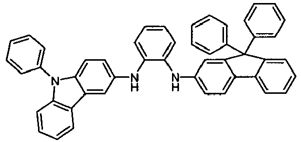 | 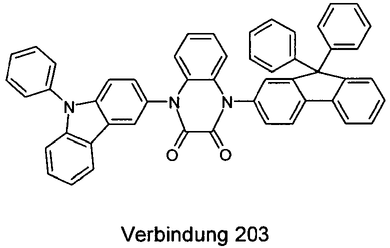 | 69% |
| I5 | 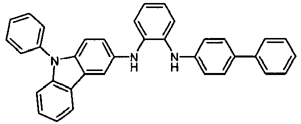 | 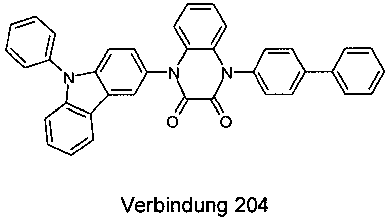 | 81% |
| I6 | 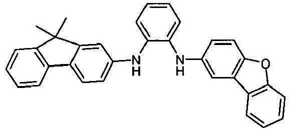 | 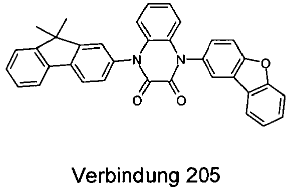 | 80% |
| I7 | 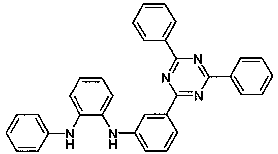 | 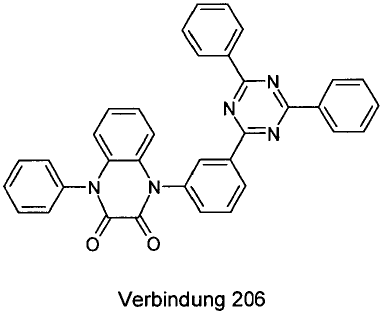 | 63% |

### Beispiel m: 1-(9,9'-Spiro[9H-flouren-2-yl)-1H-indole-2,3-dione

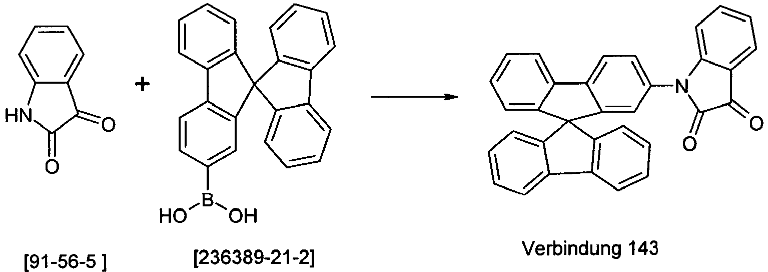

14,7g (100 mmol) Spiro-9,9'-biflour-2-boronsäure, 12 g (34 mmol) 1H-Indol-2,3-dion und 64 g (52 mmol) Kupferpulver, 12 ml (88 mmol) NEt₃ werden in 800 mL CH₂Cl₂ suspendiert und mit etwas Molekularsieb 4A° versetzt und kräftig 28h bei Raumtemperatur gerührt, und die Reaktionsmischung wird 16 h unter Rückfluss erhitzt. Danach wird die Mischung mit 40 ml MeOH versetzt und abfiltriert und eingeengt. Der Rückstand wird aus Toluol und aus Dichlormethan / umkristallisiert und abschließend im Hochvakuum sublimiert, Reinheit beträgt 99.9 %. Die Ausbeute beträgt 12,1 g (26 mmol), entsprechend 79% der Theorie.

Analog können folgende Verbindungen erhalten werden:

| **Bsp.** | **Edukt 1** | **Edukt 2** | **Produkt** | **Ausbeute** |
|---|---|---|---|---|
| m1 | 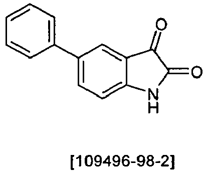 | 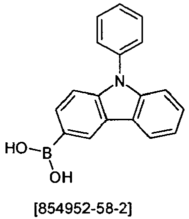 | 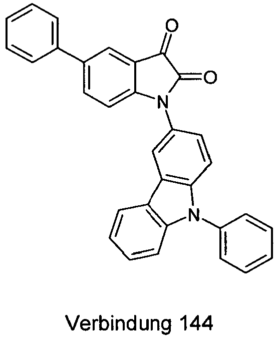 | 78% |
| m2 | 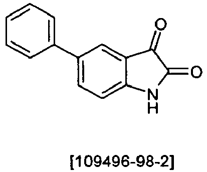 | 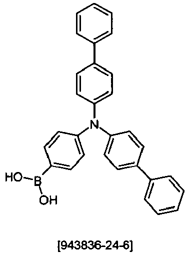 | 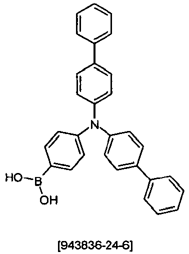 | 77% |
| m3 | 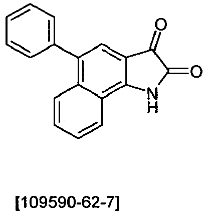 | 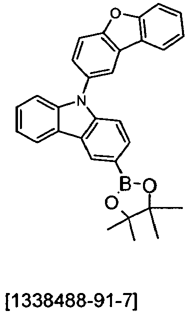 | 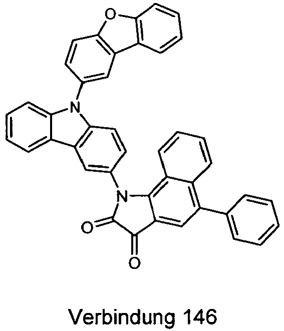 | 73% |
| m4 | 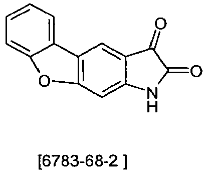 | 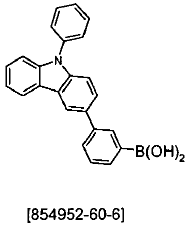 | 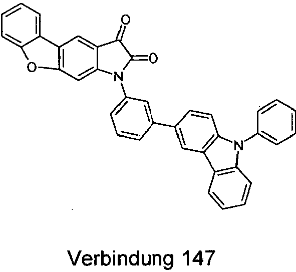 | 81% |
| m5 | 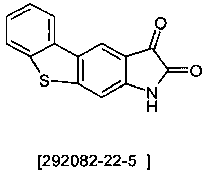 | 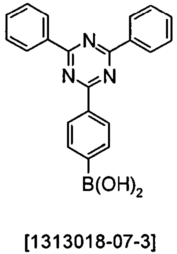 | 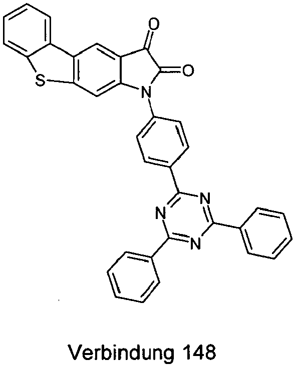 | 74% |
| m6 | 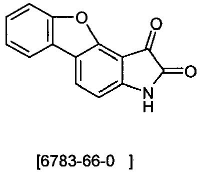 | 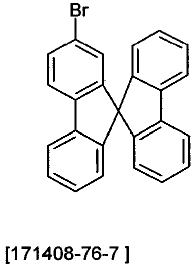 | 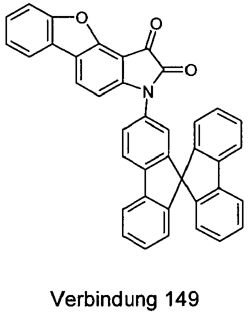 | 70% |

Analog können folgende Verbindungen erhalten werden nach der Vorschrift gemäß Beispiel m aus 250 mmol) Aryl-boronsäure und 20 mmol 1H-Indol-2,3-dion. Der ausgefallene Feststoff wird aus Toluol/Acetonitril (5:1) umkristallisiert und der Rückstand mit MeOH gewaschen. Der Rückstand wird aus Toluol und aus Dichlormethan umkristallisiert und abschließend im Hochvakuum sublimiert, Reinheit beträgt 99.9 %.

| **Bsp.** | **Edukt 1** | **Edukt 2** | **Produkt** | **Ausbeute** |
|---|---|---|---|---|
| m7 | 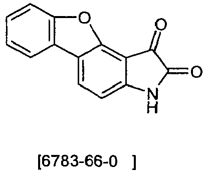 | 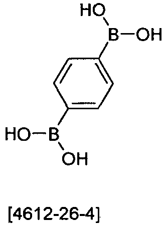 | 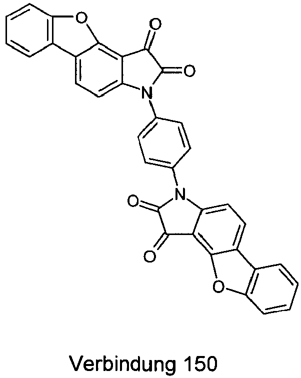 | 52% |
| m8 | 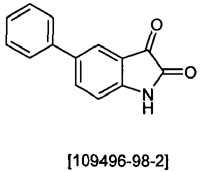 | 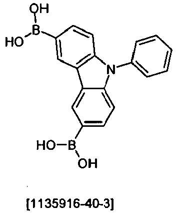 | 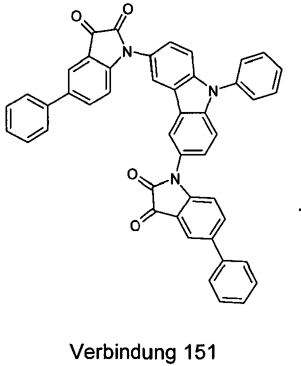 | 49% |

### Beispiel n: 1-(9,9'-Spiro[9H-flouren-2-yl)-1H-indole-2,3-dione

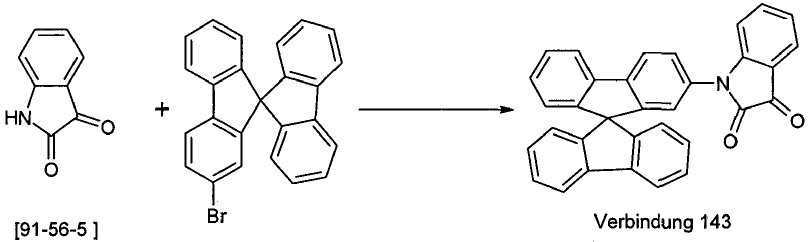

14,7g (102,4 mmol) 1H-Indol-2,3-dion, 44 g (112 mmol) Spiro-9,9'-biflour-2-boronsäure und 2,3 (10,2 mmol)1,3-Di[2-pyridyl]-1,3-propandion, 28,3 g (204 mmol) Kaliumcarbonat und 1,9 g (10,2) Kupferiodid werden im 1000 ml DMF unter Rückfluss 90h gerührt. Die Lösung wird mit Wasser verdünnt und mit Essigester zweimal extrahiert, die vereinigten organischen Phasen über Na₂SO₄ getrocknet und einrotiert und chromatographisch (EtOAc/Hexan: 2/3) gereinigt. Der Rückstand wird aus Toluol und aus Dichlormethan umkristallisiert und abschließend im Hochvakuum sublimiert, Reinheit beträgt 99.9 %. Die Ausbeute beträgt 36 g (79 mmol), entsprechend 80 % der Theorie.

Analog können folgende Verbindungen erhalten werden:

| **Bsp.** | **Edukt 1** | **Edukt 2** | **Produkt** | **Ausbeute** |
|---|---|---|---|---|
| n1 | 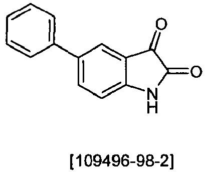 | 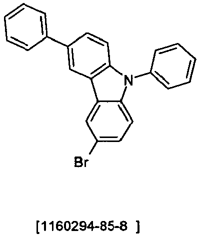 | 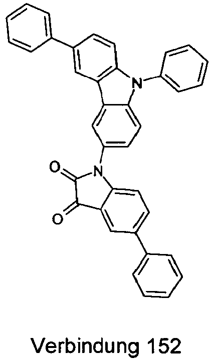 | 76% |
| n2 | 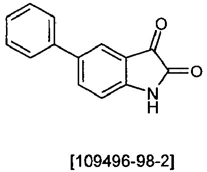 | 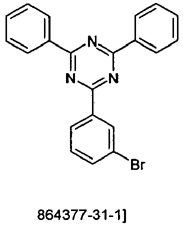 | 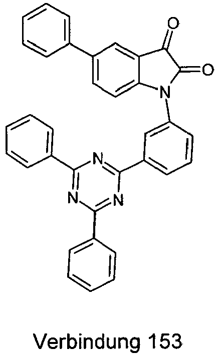 | 82% |

### Beispiel 1: Herstellung der OLEDs

In den folgenden Beispielen E1 bis E11 (siehe Tabellen 1.1 und 1.2) werden die Daten verschiedener OLEDs vorgestellt. Gereinigte Glasplättchen (Reinigung in Miele Laborspülmaschine, Reiniger Merck Extran), die mit strukturiertem ITO (Indium Zinn Oxid) der Dicke 50 nm beschichtet sind werden 25 Minuten mit UV-Ozon vorbehandelt (UV-Ozon Generator PR-100, Firma UVP) und innerhalb 30min zur verbesserten Prozessierung mit 20 nm PEDOT:PSS beschichtet (Poly(3,4-ethylenedioxythiophene) poly(styrenesulfonate), bezogen als CLEVIOS™ P VP AI 4083 von Heraeus Precious Metals GmbH Deutschland, aus wässriger Lösung aufgeschleudert) und anschließend bei 180°C 10min lang ausgeheizt. Diese beschichteten Glasplättchen bilden die Substrate, auf welche die OLEDs aufgebracht werden.

Die OLEDs haben prinzipiell folgenden Schichtaufbau: Substrat / Lochtransportschicht (HTL) / Zwischenschicht (IL) / Elektronenblockierschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1.1 zu entnehmen. Die weiteren zur Herstellung der OLEDs benötigten Materialien und der verwendeten Abkürzungen sind in Tabelle 1.3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie d46:BIC1:TEG1(50%:40%:10%) bedeutet hierbei, dass die Verbindung 117 in einem Volumenanteil von 50%, BIC1 in einem Anteil von 40% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 1.2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 1.2 zusammengefasst.

**Tabelle 1.1: Aufbau der OLEDs**

| Bsp. | HTL | IL | EBL | EML | HBL | ETL | EIL |
|---|---|---|---|---|---|---|---|
| | Dicke | Dicke | Dicke | Dicke | Dicke | Dicke | Dicke |
| E1 | SpA1 | HATCN | SpMA1 | IC2:d1 :TEG1 | ST1 | ST1:LiQ | --- |
| | 70nm | 5nm | 90nm | (60%:30%:10%) 30nm | 10nm | (50%:50%) 30nm | |
| E2 | SpA1 | HATCN | SpMA1 | IC2:d4:TEG1 | ST1 | ST1:LiQ | --- |
| | 70nm | 5nm | 90nm | (45%:45%:10%) 30nm | 10nm | (50%:50%) 30nm | |
| E3 | SpA1 | HATCN | SpMA1 | d11:TEG1 | --- | ST1:LiQ | --- |
| | 70nm | 5nm | 90nm | (85%:15%) 30nm | | (50%:50%) 40nm | |
| E4 | SpA1 | HATCN | SpMA1 | g2:TEG1 | --- | ST1:LiQ | --- |
| | 70nm | 5nm | 90nm | (85%:15%) 30nm | | (50%:50%) 40nm | |
| E5 | SpA1 | HATCN | SpMA1 | d46:TEG1 | ST1 | ST1:LiQ | --- |
| | 70nm | 5nm | 90nm | (85%:15%) 30nm | 10nm | (50%:50%) 30nm | |
| E6 | SpA1 | HATCN | SpMA1 | d46:BIC1 :TEG1 | ST1 | ST1:LiQ | --- |
| | 70nm | 5nm | 90nm | 50%:40%:10% 30nm | 10nm | 50%:50% 30nm | |
| E7 | SpA1 | HATCN | SpMA1 | IC2:e2:TEG1 | ST1 | ST1:LiQ | --- |
| | 70nm | 5nm | 90nm | (55%:35%:10%) 30nm | 10nm | (50%:50%) 30nm | |
| E8 | SpA1 | HATCN | SpMA1 | IC1TEG1 | --- | I7 | LiQ |
| | 70nm | 5nm | 90nm | (85%:15%) 30nm | | 40nm | 3nm |
| E9 | SpA1 | HATCN | SpMA1 | IC1:TEG1 | IC1 | n2 | LiQ |
| | 70nm | 5nm | 90nm | (85%:15%) 30nm | 10nm | 30nm | 3nm |
| E10 | SpA1 | HATCN | SpMA1 | I7:BIC1:TEG1 | ST1 | ST1:LiQ | --- |
| | 70nm | 5nm | 90nm | (60%:30%:10%) 30nm | 10nm | (50%:50% 30nm | |
| E11 | SpA1 | HATCN | SpMA1 | d46:TER1 | --- | ST1:LiQ | --- |
| | 90nm | 5nm | 130nm | 92%:8%) 40nm | | (50%:50%) 40nm | |

**Tabelle 1.2: Daten der OLEDs**

| Bsp. | U1000 (V) | SE1000 (cd/A) | LE1000 (Im/W) | EQE1000 | CIE x/y bei 1000 cd/m² |
|---|---|---|---|---|---|
| E1 | 3.3 | 49 | 47 | 13.6 % | 0.33/0.62 |
| E2 | 3.7 | 54 | 46 | 15.4 % | 0.35/0.61 |
| E3 | 3.6 | 53 | 45 | 15.0 % | 0.34/0.62 |
| E4 | 4.3 | 50 | 38 | 14.3 % | 0.36/0.61 |
| E5 | 3.3 | 58 | 54 | 16.1 % | 0.34/0.62 |
| E6 | 3.4 | 56 | 51 | 15.5 % | 0.34/0.62 |
| E7 | 3.3 | 47 | 45 | 13.3 % | 0.35/0.62 |
| E8 | 3.5 | 63 | 57 | 17.5 % | 0.34/0.62 |
| E9 | 4.7 | 56 | 38 | 15.7 % | 0.33/0.62 |
| E10 | 3.4 | 56 | 51 | 15.6 % | 0.34/0.62 |
| E11 | 4.8 | 10.3 | 6.7 | 11.1 % | 0.67/0.33 |

**Tabelle 1.3: Strukturformeln der Materialien für die OLEDs**

| | |
|---|---|
| 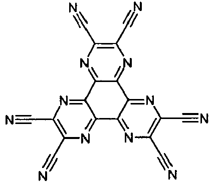 | 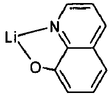 |
| HATCN | LiQ |
| 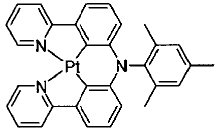 | 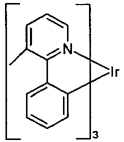 |
| TER1 | TEG1 |
| 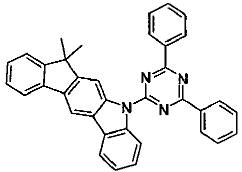 | 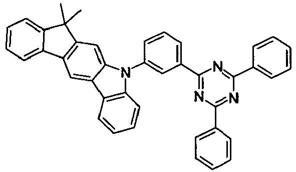 |
| IC1 | IC2 |
| 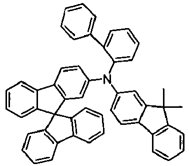 | 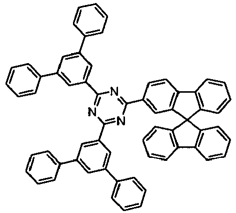 |
| SpMA1 | ST1 |
| 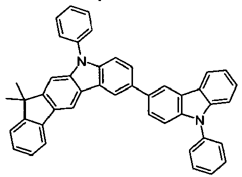 | |
| BIC1 | |
| 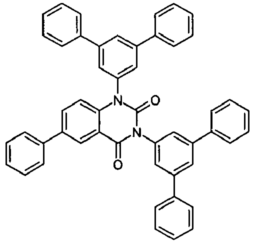 | 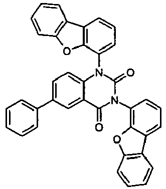 |
| d1 = Verbindung 111 | d4 = Verbindung 114 |
| 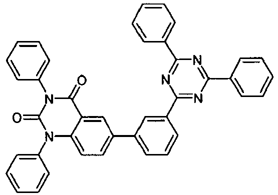 | 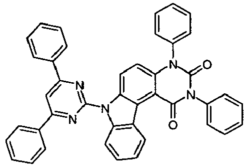 |
| d11 = Verbindung 86 | g2 = Verbindung 56 |
| 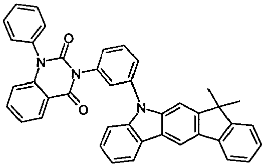 | 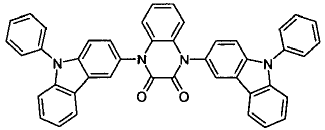 |
| d46 = Verbindung 117 | e2 = Verbindung 170 |
| 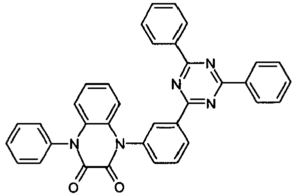 | 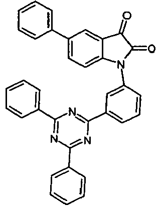 |
| I7 = Verbindung 206 | n2 = Verbindung 153 |

## Patentansprüche

1. Elektronische Vorrichtung enthaltend mindestens eine Verbindung der Formel (1) 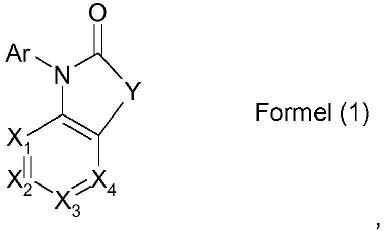 oder mindestens zwei Verbindungen der Formel (1), die über mindestens ein gemeinsames aromatisches oder heteroaromatisches Ringsystem Ar verbunden sind oder mindestens zwei Verbindungen der Formel (1), die eine gemeinsame Struktureinheit 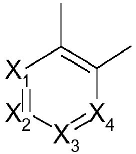 haben,
wobei für die verwendeten Symbole gilt:
X₁, X₂, X₃, X₄ sind jeweils unabhängig voneinander CR oder N;
Y ist bei jedem Auftreten 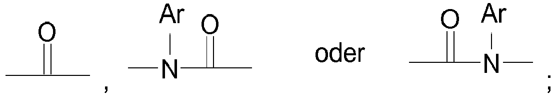
Ar ist bei jedem Auftreten gleich oder verschieden ein aromatisches oder heteroaromatisches Ringsystem mit 5-60 aromatischen Ringatomen, das mit einem oder mehreren Resten R¹ substituiert sein kann;
Ar¹ ist bei jedem Auftreten gleich oder verschieden ein aromatisches oder heteroaromatisches Ringsystem mit 5-30 aromatischen Ringatomen, das mit einem oder mehreren Resten R¹ substituiert sein kann; dabei können zwei Reste Ar¹, welche an dasselbe N-Atom oder P-Atom binden, auch durch eine Einfachbindung oder eine Brücke, ausgewählt aus N(R¹), C(R¹)₂ oder O, miteinander verbrückt sein;
R ist bei jedem Auftreten gleich oder verschieden ausgewählt aus der Gruppe bestehend aus H, D, F, Cl, Br, I, CN, CHO, NO₂, Si(R²)₃, B(OR²)₂, N(Ar¹)₂, N(R¹)₂, C(=O)Ar¹, C(=O)R¹, P(=O)(Ar¹)₂, S(=O)Ar¹, S(=O)(Ar¹)₂, CR²=CR²Ar¹, C=CAr¹, OSO₂R¹;
einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, wobei die genannten Kohlenwasserstoffgruppen jeweils mit einem oder mehreren Resten R¹ substituiert sein können und wobei eine oder mehrere nichtbenachbarte CH₂-Gruppen durch R¹C=CR¹, -C=C-, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können;
einem aromatischen oder heteroaromatischen Ringsystem mit 5 bis 60 aromatischen Ringatomen, das jeweils mit einem oder mehreren Resten R¹ substituiert sein kann,
einer Aryloxy- oder Heteroaryloxygruppe mit 5 bis 60 aromatischen Ringatomen, die mit einem oder mehreren Resten R¹ substituiert sein kann,
einer Aralkyl- oder Heteroaralkylgruppe mit 5 bis 60 aromatischen Ringatomen, die mit einem oder mehreren Resten R¹ substituiert sein kann,
oder einer Kombination dieser Systeme,
wobei zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann oder
wobei der Substituent R von X₁ und/oder der Substituent R von X₄ zusammen mit dem jeweils benachbarten N-Ar ein monocyclisches oder polycyclisches, aliphatisches, aromatisches oder heteroaromatisches Ringsystem bilden können, das mit einem oder mehreren Resten R¹ substituiert sein kann;
R¹ ist jeweils unabhängig ausgewählt aus der Gruppe bestehend aus H, D, F, CN, einer geradkettigen oder verzweigten Alkylgruppe mit 1 bis 20 C-Atomen, einer geradkettigen oder verzweigten Alkenylgruppe mit 2 bis 20 C-Atomen, einem aromatischem oder heteroaromatischem Ringsystem mit 5 bis 30 aromatischen Ringatomen, in dem ein oder mehrere H-Atome durch D, F, Cl, Br, I, CN oder eine geradkettige oder verzweigte Alkylgruppe mit 1 bis 10 C-Atomen oder eine geradkettige oder verzweigte Alkenylgruppe mit 2 bis 10 C-Atomen ersetzt sein können,
wobei zwei oder mehr benachbarte Substituenten R¹ miteinander ein mono- oder polycyclisches, aliphatisches, aromatisches oder heteroaromatisches Ringsystem bilden können und
R² ist jeweils unabhängig ausgewählt aus der Gruppe bestehend aus H, D oder ein aliphatischer, aromatischer und/oder heteroaromatischer Kohlenwasserstoffrest mit 1 bis 20 C-Atomen,
wobei auch zwei oder mehrere Reste R² miteinander ein Ringsystem bilden können.

2. Elektronische Vorrichtung nach Anspruch 1, ausgewählt aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoffsensibilisierten Solarzellen, Solarzellen enthaltend Perovskit, organischen optischen Detektoren, organischen Photorezeptoren, organischen Feld-Quench-Devices, lichtemittierenden elektrochemischen Zellen, organischen Laserdioden und Organic Plasmon Emitting Devices.

3. Elektronische Vorrichtung nach Anspruch 1 oder 2, **dadurch gekennzeichnet, dass** die Verbindung der Formel (1) als Matrixmaterial für einen fluoreszierenden oder phosphoreszierenden Emitter und/oder in einer Lochblockierschicht und/oder in einer Elektronentransportschicht und/oder in einer elektronenblockierenden bzw. exzitonenblockierenden Schicht und/oder in einer Lochtransportschicht eingesetzt wird.

4. Elektronische Vorrichtung nach einem oder mehreren der Ansprüche 1 bis 3, **dadurch gekennzeichnet, dass** Y in Formel (1) 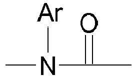 bedeutet und Ar jeweils unabhängig voneinander eine in Anspruch 1 genannte Bedeutung hat.

5. Elektronische Vorrichtung nach einem oder mehreren der Ansprüche 1 bis 3, **dadurch gekennzeichnet, dass** Y in Formel (1) 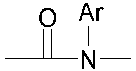 bedeutet und Ar jeweils unabhängig voneinander eine in Anspruch 1 genannte Bedeutung hat.

6. Elektronische Vorrichtung nach einem oder mehreren der Ansprüche 1 bis 3, **dadurch gekennzeichnet, dass** Y in Formel (1) 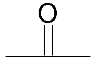 bedeutet.

7. Elektronische Vorrichtung nach einem oder mehreren der Ansprüche 1 bis 6, **dadurch gekennzeichnet, dass** mindestens eine Variable der Gruppe X₁, X₂, X₃ und X₄ N bedeutet und die restlichen Variablen CR bedeuten und R jeweils unabhängig voneinander eine der in Anspruch 1 genannten Bedeutungen hat.

8. Elektronische Vorrichtung nach einem oder mehreren der Ansprüche 1 bis 6, **dadurch gekennzeichnet, dass** die Variablen X₁, X₂, X₃ und X₄ CR bedeuten und R jeweils unabhängig voneinander eine der in Anspruch 1 genannten Bedeutungen hat.

9. Verbindungen der Formel (42), 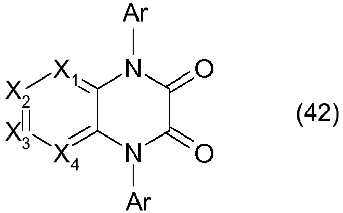 wobei X₁, X₂, X₃ und X₄ jeweils unabhängig voneinander CR bedeuten, wobei der Substituent R von X₁ und/oder der Substituent R von X₄ zusammen mit dem jeweils benachbarten N-Ar ein monocyclisches oder polycyclisches, aliphatisches, aromatisches oder heteroaromatisches Ringsystem bilden, das mit einem oder mehreren Resten R¹ substituiert sein kann,
und Ar, R, R¹ und R² jeweils unabhängig voneinander folgende Bedeutung haben:
Ar ist bei jedem Auftreten gleich oder verschieden ein aromatisches oder heteroaromatisches Ringsystem mit 5-60 aromatischen Ringatomen, das mit einem oder mehreren Resten R¹ substituiert sein kann; Ar¹ ist bei jedem Auftreten gleich oder verschieden ein aromatisches oder heteroaromatisches Ringsystem mit 5-30 aromatischen Ringatomen, das mit einem oder mehreren Resten R¹ substituiert sein kann; dabei können zwei Reste Ar¹, welche an dasselbe N-Atom oder P-Atom binden, auch durch eine Einfachbindung oder eine Brücke, ausgewählt aus N(R¹), C(R¹)₂ oder O, miteinander verbrückt sein; R ist bei jedem Auftreten gleich oder verschieden ausgewählt aus der Gruppe bestehend aus H, D, F, Cl, Br, I, CN, CHO, NO₂, Si(R²)₃, B(OR²)₂, N(Ar¹)₂, N(R¹)₂, C(=P)Ar¹, C(=O)R¹, P(=O)(Ar¹)₂, S(=O)Ar¹, S(=O)(Ar¹)₂, CR² =CR²Ar¹, C=CAr¹, OSO₂R¹; einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, wobei die genannten Kohlenwasserstoffgruppen jeweils mit einem oder mehreren Resten R¹ substituiert sein können und wobei eine oder mehrere nichtbenachbarte CH2-Gruppen durch R¹C=CR¹,-C=C-, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können; einem aromatischen oder heteroaromatischen Ringsystem mit 5 bis 60 aromatischen Ringatomen, das jeweils mit einem oder mehreren Resten R¹ substituiert sein kann, einer Aryloxy- oder Heteroaryloxygruppe mit 5 bis 60 aromatischen Ringatomen, die mit einem oder mehreren Resten R¹ substituiert sein kann, einer Aralkyl- oder Heteroaralkylgruppe mit 5 bis 60 aromatischen Ringatomen, die mit einem oder mehreren Resten R¹ substituiert sein kann, oder einer Kombination dieser Systeme, wobei zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann oder wobei der Substituent R von X₁ und/oder der Substituent R von X₄ zusammen mit dem jeweils benachbarten N-Ar ein monocyclisches oder polycyclisches, aliphatisches, aromatisches oder heteroaromatisches Ringsystem bilden können, das mit einem oder mehreren Resten R¹ substituiert sein kann; R¹ ist jeweils unabhängig ausgewählt aus der Gruppe bestehend aus H, D, F, CN, einer geradkettigen oder verzweigten Alkylgruppe mit 1 bis 20 C-Atomen, einer geradkettigen oder verzweigten Alkenylgruppe mit 2 bis 20 C-Atomen, einem aromatischem oder heteroaromatischem Ringsystem mit 5 bis 30 aromatischen Ringatomen, in dem ein oder mehrere H-Atome durch D, F, Cl, Br, I, CN oder eine geradkettige oder verzweigte Alkylgruppe mit 1 bis 10 C-Atomen oder eine geradkettige oder verzweigte Alkenylgruppe mit 2 bis 10 C-Atomen ersetzt sein können, wobei zwei oder mehr benachbarte Substituenten R¹ miteinander ein mono- oder polycyclisches, aliphatisches, aromatisches oder heteroaromatisches Ringsystem bilden können und R² ist jeweils unabhängig ausgewählt aus der Gruppe bestehend aus H, D oder ein aliphatischer, aromatischer und/oder heteroaromatischer Kohlenwasserstoffrest mit 1 bis 20 C-Atomen, wobei auch zwei oder mehrere Reste R² miteinander ein Ringsystem bilden können.

10. Verbindungen der Formeln (47) bis (51) 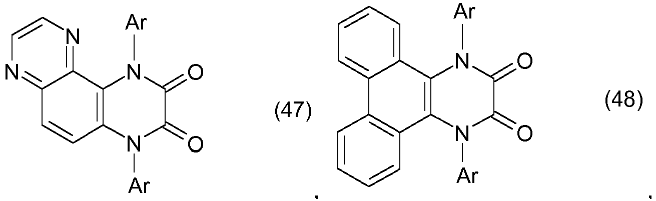 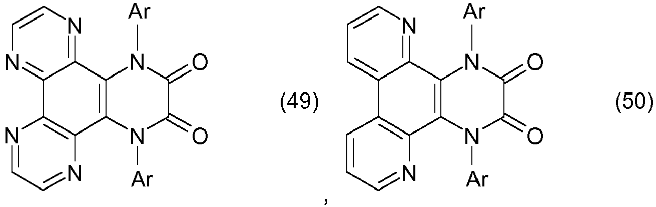 und 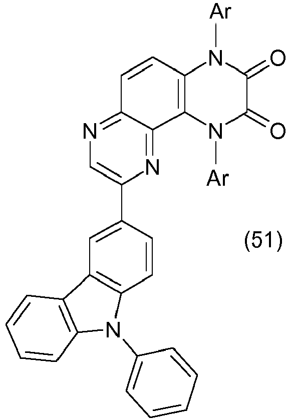 wobei Ar jeweils unabhängig voneinander folgende Bedeutung hat:
Ar ist bei jedem Auftreten gleich oder verschieden ein aromatisches oder heteroaromatisches Ringsystem mit 5-60 aromatischen Ringatomen, das mit einem oder mehreren Resten R¹ substituiert sein kann; und
R¹ ist jeweils unabhängig ausgewählt aus der Gruppe bestehend aus H, D, F, CN, einer geradkettigen oder verzweigten Alkylgruppe mit 1 bis 20 C-Atomen, einer geradkettigen oder verzweigten Alkenylgruppe mit 2 bis 20 C-Atomen, einem aromatischem oder heteroaromatischem Ringsystem mit 5 bis 30 aromatischen Ringatomen, in dem ein oder mehrere H-Atome durch D, F, Cl, Br, I, CN oder eine geradkettige oder verzweigte Alkylgruppe mit 1 bis 10 C-Atomen oder eine geradkettige oder verzweigte Alkenylgruppe mit 2 bis 10 C-Atomen ersetzt sein können, wobei zwei oder mehr benachbarte Substituenten R¹ miteinander ein mono- oder polycyclisches, aliphatisches, aromatisches oder heteroaromatisches Ringsystem bilden können.

11. Verbindungen der Formel (56) 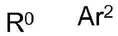 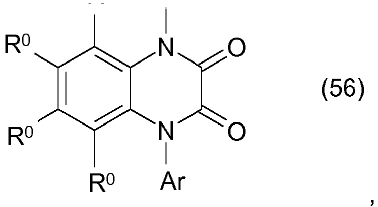 wobei
Ar ein aromatisches oder heteroaromatisches Ringsystem mit 5-60 aromatischen Ringatomen bedeutet, das mit einem oder mehreren nicht-aromatischen Resten R¹ substituiert sein kann,
Ar² ein aromatisches Ringsystem mit 13 bis 40 C-Atomen oder ein heteroaromatisches Ringsystem mit 4 bis 40 C-Atomen bedeutet, das mit einem oder mehreren nicht-aromatischen Resten R¹ substituiert sein kann,
R⁰ jeweils unabhängig voneinander aus der Gruppe bestehend aus H, D, F, CN, einer geradkettigen oder verzweigten Alkylgruppe mit 1 bis 20 C-Atomen, einer geradkettigen oder verzweigten Alkenylgruppe mit 2 bis 20 C-Atomen, einem aromatischem Ringsystem mit 5 bis 30 aromatischen Ringatomen, in dem ein oder mehrere H-Atome durch D, F, Cl, Br, I, CN oder eine geradkettige oder verzweigte Alkylgruppe mit 1 bis 10 C-Atomen oder eine geradkettige oder verzweigte Alkenylgruppe mit 2 bis 10 C-Atomen ersetzt sein können, ausgewählt wird und
wobei R¹ und R jeweils unabhängig voneinander folgende Bedeutung haben:
R ist bei jedem Auftreten gleich oder verschieden ausgewählt aus der Gruppe bestehend aus H, D, F, Cl, Br, I, CN, CHO, NO₂, Si(R²)₃, B(OR²)₂, N(Ar¹)₂, N(R¹)₂, C(=P)Ar¹, C(=O)R¹, P(=O)(Ar¹)₂, S(=O)Ar¹, S(=O)(Ar¹)₂, CR²=CR²Ar¹, C=CAr¹, OSO₂R¹; einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, wobei die genannten Kohlenwasserstoffgruppen jeweils mit einem oder mehreren Resten R¹ substituiert sein können und wobei eine oder mehrere nichtbenachbarte CH2-Gruppen durch R¹C=CR¹, -C=C-, Si(R¹)₂, Ge(R¹)₂, Sn(R¹)₂, C=O, C=S, C=Se, C=NR¹, P(=O(R¹), SO, SO₂, NR¹, O, S oder CONR¹ ersetzt sein können und wobei ein oder mehrere H-Atome durch D, F, Cl, Br, I, CN oder NO₂ ersetzt sein können; einem aromatischen oder heteroaromatischen Ringsystem mit 5 bis 60 aromatischen Ringatomen, das jeweils mit einem oder mehreren Resten R¹ substituiert sein kann, einer Aryloxy- oder Heteroaryloxygruppe mit 5 bis 60 aromatischen Ringatomen, die mit einem oder mehreren Resten R¹ substituiert sein kann, einer Aralkyl- oder Heteroaralkylgruppe mit 5 bis 60 aromatischen Ringatomen, die mit einem oder mehreren Resten R¹ substituiert sein kann, oder einer Kombination dieser Systeme, wobei zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann; R¹ ist jeweils unabhängig ausgewählt aus der Gruppe bestehend aus H, D, F, CN, einer geradkettigen oder verzweigten Alkylgruppe mit 1 bis 20 C-Atomen, einer geradkettigen oder verzweigten Alkenylgruppe mit 2 bis 20 C-Atomen, einem aromatischem oder heteroaromatischem Ringsystem mit 5 bis 30 aromatischen Ringatomen, in dem ein oder mehrere H-Atome durch D, F, Cl, Br, I, CN oder eine geradkettige oder verzweigte Alkylgruppe mit 1 bis 10 C-Atomen oder eine geradkettige oder verzweigte Alkenylgruppe mit 2 bis 10 C-Atomen ersetzt sein können, wobei zwei oder mehr benachbarte Substituenten R¹ miteinander ein mono- oder polycyclisches, aliphatisches, aromatisches oder heteroaromatisches Ringsystem bilden können und R² ist jeweils unabhängig ausgewählt aus der Gruppe bestehend aus H, D oder ein aliphatischer, aromatischer und/oder heteroaromatischer Kohlenwasserstoffrest mit 1 bis 20 C-Atomen, wobei auch zwei oder mehrere Reste R² miteinander ein Ringsystem bilden können.

12. Verbindungen der Formel (57), 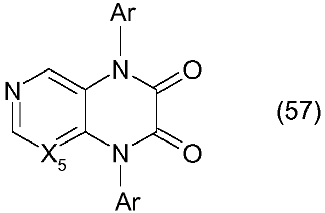 wobei
X₅ CR oder N entspricht und Ar und R folgende Bedeutung haben:
Ar ist bei jedem Auftreten gleich oder verschieden ein aromatisches oder heteroaromatisches Ringsystem mit 5-60 aromatischen Ringatomen, das mit einem oder mehreren Resten R¹ substituiert sein kann; Ar¹ ist bei jedem Auftreten gleich oder verschieden ein aromatisches oder heteroaromatisches Ringsystem mit 5-30 aromatischen Ringatomen, das mit einem oder mehreren Resten R¹ substituiert sein kann; dabei können zwei Reste Ar¹, welche an dasselbe N-Atom oder P-Atom binden, auch durch eine Einfachbindung oder eine Brücke, ausgewählt aus N(R¹), C(R¹)₂ oder O, miteinander verbrückt sein; R ist bei jedem Auftreten gleich oder verschieden ausgewählt aus der Gruppe bestehend aus H, D, F, Cl, Br, I, CN, CHO, NO₂, Si(R²)₃, B(OR²)₂, N(Ar¹)₂, N(R¹)₂, C(=P)Ar¹, C(=O)R¹, P(=O)(Ar¹)₂, S(=O)Ar¹, S(=O)(Ar¹)₂, CR²=CR²Ar¹, C=CAr¹, OSO₂R¹; einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen oder einer Alkenyl- oder Alkinylgruppe mit 2 bis 40 C-Atomen, wobei die genannten Kohlenwasserstoffgruppen jeweils mit einem oder mehreren Resten R¹ substituiert sein können und wobei eine oder mehrere nichtbenachbarte CH2-Gruppen durch R¹C=CR¹,-C=C-, Si(R¹)₂, Ge(R¹)₂, Sn(R¹)₂, C=O, C=S, C=Se, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können; einem aromatischen oder heteroaromatischen Ringsystem mit 5 bis 60 aromatischen Ringatomen, das jeweils mit einem oder mehreren Resten R¹ substituiert sein kann, einer Aryloxy- oder Heteroaryloxygruppe mit 5 bis 60 aromatischen Ringatomen, die mit einem oder mehreren Resten R¹ substituiert sein kann, einer Aralkyl- oder Heteroaralkylgruppe mit 5 bis 60 aromatischen Ringatomen, die mit einem oder mehreren Resten R¹ substituiert sein kann, oder einer Kombination dieser Systeme, wobei zwei oder mehr benachbarte Substituenten R ein monocyclisches oder polycyclisches, aliphatisches, aromatisches oder heteroaromatisches Ringsystem bilden können, das mit einem oder mehreren Resten R¹ substituiert sein kann oder wobei der Substituent R von X₅ zusammen mit dem benachbarten N-Ar ein monocyclisches oder polycyclisches, aliphatisches, aromatisches oder heteroaromatisches Ringsystem bilden können, das mit einem oder mehreren Resten R¹ substituiert sein kann; R¹ ist jeweils unabhängig ausgewählt aus der Gruppe bestehend aus H, D, F, CN, einer geradkettigen oder verzweigten Alkylgruppe mit 1 bis 20 C-Atomen, einer geradkettigen oder verzweigten Alkenylgruppe mit 2 bis 20 C-Atomen, einem aromatischem oder heteroaromatischem Ringsystem mit 5 bis 30 aromatischen Ringatomen, in dem ein oder mehrere H-Atome durch D, F, Cl, Br, I, CN oder eine geradkettige oder verzweigte Alkylgruppe mit 1 bis 10 C-Atomen oder eine geradkettige oder verzweigte Alkenylgruppe mit 2 bis 10 C-Atomen ersetzt sein können, wobei zwei oder mehr benachbarte Substituenten R¹ miteinander ein mono- oder polycyclisches, aliphatisches, aromatisches oder heteroaromatisches Ringsystem bilden können und R² ist jeweils unabhängig ausgewählt aus der Gruppe bestehend aus H, D oder ein aliphatischer, aromatischer und/oder heteroaromatischer Kohlenwasserstoffrest mit 1 bis 20 C-Atomen, wobei auch zwei oder mehrere Reste R² miteinander ein Ringsystem bilden können.

13. Formulierungen enthaltend mindestens eine Verbindung nach einem oder mehreren der Ansprüche 9 bis 12.

14. Verwendung von Verbindungen nach einem oder mehreren der Ansprüche 9 bis 12 in elektronischen Vorrichtungen.

## Claims

1. Electronic device containing at least one compound of the formula (1) 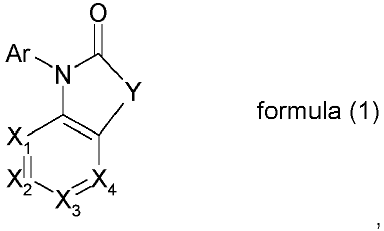 or at least two compounds of the formula (1) which are connected via at least one common aromatic or heteroaromatic ring system Ar or at least two compounds of the formula (1) which have a common structural unit 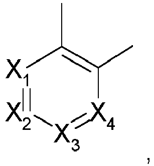 where the following applies to the symbols used:
X₁, X₂, X₃, X₄ are in each case, independently of one another, CR or N;
Y is on each occurrence 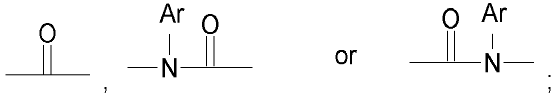
Ar is on each occurrence, identically or differently, an aromatic or heteroaromatic ring system having 5-60 aromatic ring atoms, which may be substituted by one or more radicals R¹;
Ar¹ is on each occurrence, identically or differently, an aromatic or heteroaromatic ring system having 5-30 aromatic ring atoms, which may be substituted by one or more radicals R¹; two radicals Ar¹ which are bonded to the same N atom or P atom may also be bridged to one another by a single bond or a bridge selected from N(R¹), C(R¹)₂ or O;
R is selected on each occurrence, identically or differently, from the group consisting of H, D, F, Cl, Br, I, CN, CHO, NO₂, Si(R²)₃, B(OR²)₂, N(Ar¹)₂, N(R¹)₂, C(=O)Ar¹, C(=O)R¹, P(=O)(Ar¹)₂, S(=O)Ar¹, S(=O)(Ar¹)₂, CR²=CR²Ar¹, C≡CAr¹, OSO₂R¹;
a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, where the said hydrocarbon groups may in each case be substituted by one or more radicals R¹ and where one or more non-adjacent CH₂ groups may be replaced by R¹C=CR¹, -C=C-, Si(R¹)₂, Ge(R¹)₂, Sn(R¹)₂, C=O, C=S, C=Se, C=NR¹, P(=O)(R¹), SO, SO₂, NR¹, O, S or CONR¹ and where one or more H atoms may be replaced by D, F, Cl, Br, I, CN or NO₂;
an aromatic or heteroaromatic ring system having 5 to 60 aromatic ring atoms, which may in each case be substituted by one or more radicals R¹,
an aryloxy or heteroaryloxy group having 5 to 60 aromatic ring atoms, which may be substituted by one or more radicals R¹,
an aralkyl or heteroaralkyl group having 5 to 60 aromatic ring atoms, which may be substituted by one or more radicals R¹,
or a combination of these systems,
where two or more adjacent substituents R may form a monocyclic or polycyclic, aliphatic, aromatic or heteroaromatic ring system, which may be substituted by one or more radicals R¹ or
where the substituent R of X₁ and/or the substituent R of X₄ together with the respectively adjacent N-Ar may form a monocyclic or polycyclic, aliphatic, aromatic or heteroaromatic ring system, which may be substituted by one or more radicals R¹;
R¹ is in each case independently selected from the group consisting of H, D, F, CN, a straight-chain or branched alkyl group having 1 to 20 C atoms, a straight-chain or branched alkenyl group having 2 to 20 C atoms, an aromatic or heteroaromatic ring system having 5 to 30 aromatic ring atoms, in which one or more H atoms may be replaced by D, F, Cl, Br, I, CN or a straight-chain or branched alkyl group having 1 to 10 C atoms or a straight-chain or branched alkenyl group having 2 to 10 C atoms,
where two or more adjacent substituents R¹ may form a mono- or polycyclic, aliphatic, aromatic or heteroaromatic ring system with one another and
R² is in each case independently selected from the group consisting of H, D or an aliphatic, aromatic and/or heteroaromatic hydrocarbon radical having 1 to 20 C atoms, where two or more radicals R² may also form a ring system with one another.

2. Electronic device according to Claim 1, selected from organic electroluminescent devices, organic integrated circuits, organic field-effect transistors, organic thin -film transistors, organic light-emitting transistors, organic solar cells, organic dye-sensitised solar cells, solar cells containing perovskite, organic optical detectors, organic photoreceptors, organic field-quench devices, light-emitting electrochemical cells, organic laser diodes and organic plasmon-emitting devices.

3. Electronic device according to Claim 1 or 2, **characterised in that** the compound of the formula (1) is employed as matrix material for a fluorescent or phosphorescent emitter and/or in a hole-blocking layer and/or in an electron-transport layer and/or in an electron-blocking or exciton-blocking layer and/or in a hole-transport layer.

4. Electronic device according to one or more of Claims 1 to 3, **characterised in that** Y in formula (1) denotes 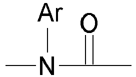 and Ar in each case, independently of one another, has a meaning given in Claim 1.

5. Electronic device according to one or more of Claims 1 to 3, **characterised in that** Y in formula (1) denotes 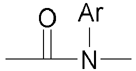 and Ar in each case, independently of one another, has a meaning given in Claim 1.

6. Electronic device according to one or more of Claims 1 to 3, **characterised in that** Y in formula (1) denotes

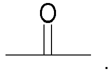

7. Electronic device according to one or more of Claims 1 to 6, **characterised in that** at least one variable of the group X₁, X₂, X₃ and X₄ denotes N and the remaining variables denote CR and R in each case, independently of one another, has one of the meanings given in Claim 1.

8. Electronic device according to one or more of Claims 1 to 6, **characterised in that** the variables X₁, X₂, X₃ and X₄ denote CR and R in each case, independently of one another, has one of the meanings given in Claim 1.

9. Compounds of the formula (42), 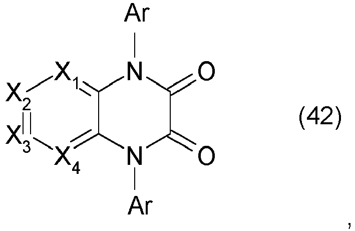 where X₁, X₂, X₃ and X₄ in each case, independently of one another, denote CR, where the substituent R of X₁ and/or the substituent R of X₄ together with the respectively adjacent N-Ar form a monocyclic or polycyclic, aliphatic, aromatic or heteroaromatic ring system, which may be substituted by one or more radicals R¹,
and Ar, R, R¹ and R² in each case, independently of one another, have the following meaning:
Ar is on each occurrence, identically or differently, an aromatic or heteroaromatic ring system having 5-60 aromatic ring atoms, which may be substituted by one or more radicals R¹; Ar¹ is on each occurrence, identically or differently, an aromatic or heteroaromatic ring system having 5-30 aromatic ring atoms, which may be substituted by one or more radicals R¹; two radicals Ar¹ which are bonded to the same N atom or P atom may also be bridged to one another by a single bond or a bridge selected from N(R¹), C(R¹)₂ or O;
R is selected on each occurrence, identically or differently, from the group consisting of H, D, F, Cl, Br, I, CN, CHO, NO₂, Si(R²)₃, B(OR²)₂, N(Ar¹)₂, N(R¹)₂, C(=O)Ar¹, C(=O)R¹, P(=O)(Ar¹)₂, S(=O)Ar¹, S(=O)(Ar¹)₂, CR²=CR²Ar¹, C≡CAr¹, OSO₂R¹; a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, where the said hydrocarbon groups may in each case be substituted by one or more radicals R¹ and where one or more non-adjacent CH₂ groups may be replaced by R¹C=CR¹, -C=C-, Si(R¹)₂, Ge(R¹)₂, Sn(R¹)₂, C=O, C=S, C=Se, C=NR¹, P(=O)(R¹), SO, SO₂, NR¹, O, S or CONR¹ and where one or more H atoms may be replaced by D, F, Cl, Br, I, CN or NO₂; an aromatic or heteroaromatic ring system having 5 to 60 aromatic ring atoms, which may in each case be substituted by one or more radicals R¹, an aryloxy or heteroaryloxy group having 5 to 60 aromatic ring atoms, which may be substituted by one or more radicals R¹, an aralkyl or heteroaralkyl group having 5 to 60 aromatic ring atoms, which may be substituted by one or more radicals R¹, or a combination of these systems, where two or more adjacent substituents R may form a monocyclic or polycyclic, aliphatic, aromatic or heteroaromatic ring system, which may be substituted by one or more radicals R¹ or where the substituent R of X₁ and/or the substituent R of X₄ together with the respectively adjacent N-Ar may form a monocyclic or polycyclic, aliphatic, aromatic or heteroaromatic ring system, which may be substituted by one or more radicals R¹;
R¹ is in each case independently selected from the group consisting of H, D, F, CN, a straight-chain or branched alkyl group having 1 to 20 C atoms, a straight-chain or branched alkenyl group having 2 to 20 C atoms, an aromatic or heteroaromatic ring system having 5 to 30 aromatic ring atoms, in which one or more H atoms may be replaced by D, F, Cl, Br, I, CN or a straight-chain or branched alkyl group having 1 to 10 C atoms or a straight-chain or branched alkenyl group having 2 to 10 C atoms, where two or more adjacent substituents R¹ may form a mono- or polycyclic, aliphatic, aromatic or heteroaromatic ring system with one another and R² is in each case independently selected from the group consisting of H, D or an aliphatic, aromatic and/or heteroaromatic hydrocarbon radical having 1 to 20 C atoms, where two or more radicals R² may also form a ring system with one another.

10. Compounds of the formulae (47) to (51) 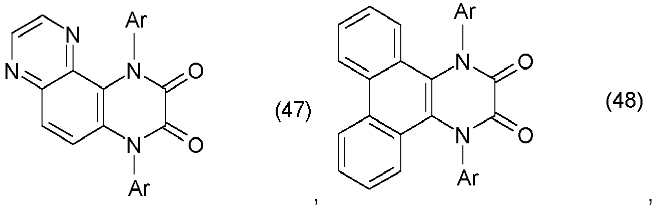 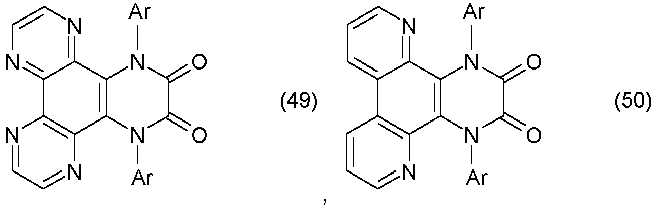 and 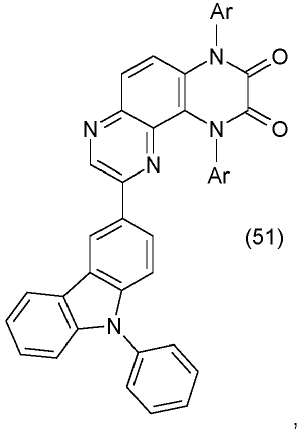 where Ar in each case, independently of one another, has the following meaning:
Ar is on each occurrence, identically or differently, an aromatic or heteroaromatic ring system having 5-60 aromatic ring atoms, which may be substituted by one or more radicals R¹; and
R¹ is in each case independently selected from the group consisting of H, D, F, CN, a straight-chain or branched alkyl group having 1 to 20 C atoms, a straight-chain or branched alkenyl group having 2 to 20 C atoms, an aromatic or heteroaromatic ring system having 5 to 30 aromatic ring atoms, in which one or more H atoms may be replaced by D, F, Cl, Br, I, CN or a straight-chain or branched alkyl group having 1 to 10 C atoms or a straight-chain or branched alkenyl group having 2 to 10 C atoms, where two or more adjacent substituents R¹ may form a mono- or polycyclic, aliphatic, aromatic or heteroaromatic ring system with one another.

11. Compounds of the formula (56) 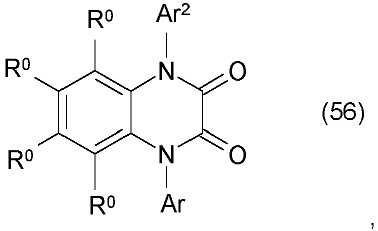 where
Ar denotes an aromatic or heteroaromatic ring system having 5-60 aromatic ring atoms, which may be substituted by one or more non-aromatic radicals R¹,
Ar² denotes an aromatic ring system having 13 to 40 C atoms or a heteroaromatic ring system having 4 to 40 C atoms, which may be substituted by one or more non-aromatic radicals R¹,
R⁰ in each case, independently of one another, is selected from the group consisting of H, D, F, CN, a straight-chain or branched alkyl group having 1 to 20 C atoms, a straight-chain or branched alkenyl group having 2 to 20 C atoms, an aromatic ring system having 5 to 30 aromatic ring atoms, in which one or more H atoms may be replaced by D, F, Cl, Br, I, CN or a straight-chain or branched alkyl group having 1 to 10 C atoms or a straight-chain or branched alkenyl group having 2 to 10 C atoms, and
where R¹ and R in each case, independently of one another, have the following meaning:
R is selected on each occurrence, identically or differently, from the group consisting of H, D, F, Cl, Br, I, CN, CHO, NO₂, Si(R²)₃, B(OR²)₂, N(Ar¹)₂, N(R¹)₂, C(=O)Ar¹, C(=O)R¹, P(=O)(Ar¹)₂, S(=O)Ar¹, S(=O)(Ar¹)₂, CR²=CR²Ar¹, C≡CAr¹, OSO₂R¹; a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, where the said hydrocarbon groups may in each case be substituted by one or more radicals R¹ and where one or more non-adjacent CH₂ groups may be replaced by R¹C=CR¹, -C=C-, Si(R¹)₂, Ge(R¹)₂, Sn(R¹)₂, C=O, C=S, C=Se, C=NR¹, P(=O)(R¹), SO, SO₂, NR¹, O, S or CONR¹ and where one or more H atoms may be replaced by D, F, Cl, Br, I, CN or NO₂; an aromatic or heteroaromatic ring system having 5 to 60 aromatic ring atoms, which may in each case be substituted by one or more radicals R¹, an aryloxy or heteroaryloxy group having 5 to 60 aromatic ring atoms, which may be substituted by one or more radicals R¹, an aralkyl or heteroaralkyl group having 5 to 60 aromatic ring atoms, which may be substituted by one or more radicals R¹, or a combination of these systems, where two or more adjacent substituents R may form a monocyclic or polycyclic, aliphatic, aromatic or heteroaromatic ring system, which may be substituted by one or more radicals R¹; R¹ is in each case independently selected from the group consisting of H, D, F, CN, a straight-chain or branched alkyl group having 1 to 20 C atoms, a straight-chain or branched alkenyl group having 2 to 20 C atoms, an aromatic or heteroaromatic ring system having 5 to 30 aromatic ring atoms, in which one or more H atoms may be replaced by D, F, Cl, Br, I, CN or a straight-chain or branched alkyl group having 1 to 10 C atoms or a straight-chain or branched alkenyl group having 2 to 10 C atoms, where two or more adjacent substituents R¹ may form a mono- or polycyclic, aliphatic, aromatic or heteroaromatic ring system with one another and R² is in each case independently selected from the group consisting of H, D or an aliphatic, aromatic and/or heteroaromatic hydrocarbon radical having 1 to 20 C atoms, where two or more radicals R² may also form a ring system with one another.

12. Compounds of the formula (57), 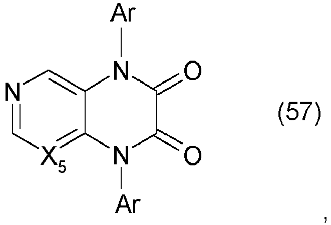 where
X₅ corresponds to CR or N and Ar and R have the following meaning:
Ar is on each occurrence, identically or differently, an aromatic or heteroaromatic ring system having 5-60 aromatic ring atoms, which may be substituted by one or more radicals R¹; Ar¹ is on each occurrence, identically or differently, an aromatic or heteroaromatic ring system having 5-30 aromatic ring atoms, which may be substituted by one or more radicals R¹; two radicals Ar¹ which are bonded to the same N atom or P atom may also be bridged to one another by a single bond or a bridge selected from N(R¹), C(R¹)₂ or O;
R is selected on each occurrence, identically or differently, from the group consisting of H, D, F, Cl, Br, I, CN, CHO, NO₂, Si(R²)₃, B(OR²)₂, N(Ar¹)₂, N(R¹)₂, C(=O)Ar¹, C(=O)R¹, P(=O)(Ar¹)₂, S(=O)Ar¹, S(=O)(Ar¹)₂, CR²=CR²Ar¹, C≡CAr¹, OSO₂R¹; a straight-chain alkyl, alkoxy or thioalkyl group having 1 to 40 C atoms or a branched or cyclic alkyl, alkoxy or thioalkyl group having 3 to 40 C atoms or an alkenyl or alkynyl group having 2 to 40 C atoms, where the said hydrocarbon groups may in each case be substituted by one or more radicals R¹ and where one or more non-adjacent CH₂ groups may be replaced by R¹C=CR¹, -C=C-, Si(R¹)₂, Ge(R¹)₂, Sn(R¹)₂, C=O, C=S, C=Se, C=NR¹, P(=O)(R¹), SO, SO₂, NR¹, O, S or CONR¹ and where one or more H atoms may be replaced by D, F, Cl, Br, I, CN or NO₂; an aromatic or heteroaromatic ring system having 5 to 60 aromatic ring atoms, which may in each case be substituted by one or more radicals R¹, an aryloxy or heteroaryloxy group having 5 to 60 aromatic ring atoms, which may be substituted by one or more radicals R¹, an aralkyl or heteroaralkyl group having 5 to 60 aromatic ring atoms, which may be substituted by one or more radicals R¹, or a combination of these systems, where two or more adjacent substituents R may form a monocyclic or polycyclic, aliphatic, aromatic or heteroaromatic ring system, which may be substituted by one or more radicals R¹ or where the substituent R of X₅ together with the adjacent N-Ar may form a monocyclic or polycyclic, aliphatic, aromatic or heteroaromatic ring system, which may be substituted by one or more radicals R¹;
R¹ is in each case independently selected from the group consisting of H, D, F, CN, a straight-chain or branched alkyl group having 1 to 20 C atoms, a straight-chain or branched alkenyl group having 2 to 20 C atoms, an aromatic or heteroaromatic ring system having 5 to 30 aromatic ring atoms, in which one or more H atoms may be replaced by D, F, Cl, Br, I, CN or a straight-chain or branched alkyl group having 1 to 10 C atoms or a straight-chain or branched alkenyl group having 2 to 10 C atoms, where two or more adjacent substituents R¹ may form a mono- or polycyclic, aliphatic, aromatic or heteroaromatic ring system with one another and R² is in each case independently selected from the group consisting of H, D or an aliphatic, aromatic and/or heteroaromatic hydrocarbon radical having 1 to 20 C atoms, where two or more radicals R² may also form a ring system with one another.

13. Formulations comprising at least one compound according to one or more of Claims 9 to 12.

14. Use of compounds according to one or more of Claims 9 to 12 in electronic devices.

## Revendications

1. Dispositif électronique contenant au moins un composé de la formule (1) 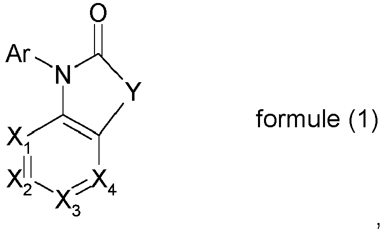 ou au moins deux composés de la formule (1) qui sont connectés via au moins un système de cycle aromatique ou hétéroaromatique commun Ar ou au moins deux composés de la formule (1) qui comportent une unité structurelle commune 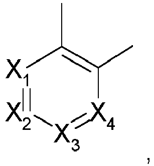 où ce qui suit s'applique aux symboles qui sont utilisés :
X₁, X₂, X₃, X₄ sont dans chaque cas, de manière indépendante les uns des autres, CR ou N ;
Y est pour chaque occurrence 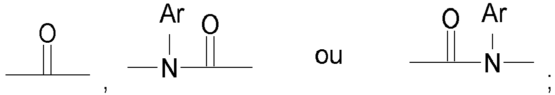
Ar est pour chaque occurrence, de manière identique ou différente, un système de cycle aromatique ou hétéroaromatique qui comporte de 5 à 60 atomes de cycle aromatique, lequel peut être substitué par un radical ou par plusieurs radicaux R¹ ;
Ar¹ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R¹ ; deux radicaux Ar¹ qui sont liés au même atome de N ou au même atome de P peuvent également être pontés l'un à l'autre au moyen d'une liaison simple ou d'un pont qui est sélectionné parmi N(R¹), C(R¹)₂ ou O ;
R est sélectionné pour chaque occurrence, de manière identique ou différente, parmi le groupe qui est constitué par H, D, F, Cl, Br, I, CN, CHO, NO₂, Si(R²)₃, B(OR²)₂, N(Ar¹)₂, N(R¹)₂, C(=O)Ar¹, C(=O)R¹, P(=O)(Ar¹)₂, S(=O)Ar¹, S(=O)(Ar¹)₂, CR²=CR²Ar¹, C≡CAr¹, OSO₂R¹ ; un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, où lesdits groupes hydrocarbone peuvent dans chaque cas être substitués par un radical ou par plusieurs radicaux R¹ et où un ou plusieurs groupe(s) CH₂ non adjacents peut/peuvent être remplacé(s) par R¹C=CR¹, -C=C-, Si(R¹)₂, Ge(R¹)₂, Sn(R¹)₂, C=O, C=S, C=Se, C=NR¹, P(=O)(R¹), SO, SO₂, NR¹, O, S ou CONR¹ et où un ou plusieurs atome(s) de H peut/peuvent être remplacé(s) par D, F, Cl, Br, I, CN ou NO₂;
un système de cycle aromatique ou hétéroaromatique qui comporte de 5 à 60 atomes de cycle aromatique, lequel peut dans chaque cas être substitué par un radical ou par plusieurs radicaux R¹,
un groupe aryloxy ou hétéroaryloxy qui comporte de 5 à 60 atomes de cycle aromatique, lequel peut être substitué par un radical ou par plusieurs radicaux R¹,
un groupe aralkyle ou hétéroaralkyle qui comporte de 5 à 60 atomes de cycle aromatique, lequel peut être substitué par un radical ou par plusieurs radicaux R¹,
ou une combinaison de ces systèmes,
où deux substituants R adjacents ou plus peuvent former un système de cycle aliphatique, aromatique ou hétéroaromatique monocyclique ou polycyclique, lequel peut être substitué par un radical ou par plusieurs radicaux R¹ ou
où le substituant R de X₁ et/ou le substituant R de X₄ en association avec le N-Ar respectivement adjacent peuvent/peut former un système de cycle aliphatique, aromatique ou hétéroaromatique monocyclique ou polycyclique, lequel peut être substitué par un radical ou par plusieurs radicaux R¹ ;
R¹ est, dans chaque cas de manière indépendante, sélectionné parmi le groupe qui est constitué par H, D, F, CN, un groupe alkyle en chaîne droite ou ramifié qui comporte de 1 à 20 atome(s) de C, un groupe alkényle en chaîne droite ou ramifié qui comporte de 2 à 20 atomes de C, un système de cycle aromatique ou hétéroaromatique qui comporte de 5 à 30 atomes de cycle aromatique, où un ou plusieurs atome(s) de H peut/peuvent être remplacé(s) par D, F, Cl, Br, I, CN ou un groupe alkyle en chaîne droite ou ramifié qui comporte de 1 à 10 atome(s) de C ou un groupe alkényle en chaîne droite ou ramifié qui comporte de 2 à 10 atomes de C,
où deux substituants R¹ adjacents ou plus peuvent former un système de cycle aliphatique, aromatique ou hétéroaromatique mono- ou polycyclique l'un avec l'autre ou les uns avec les autres et
R² est, dans chaque cas de manière indépendante, sélectionné parmi le groupe qui est constitué par H, D ou un radical hydrocarbone aliphatique, aromatique et/ou hétéroaromatique qui comporte de 1 à 20 atome(s) de C, où deux radicaux R² ou plus peuvent également former un système de cycle l'un avec l'autre ou les uns avec les autres.

2. Dispositif électronique selon la revendication 1, sélectionné parmi les dispositifs électroluminescents organiques, les circuits intégrés organiques, les transistors à effet de champ organiques, les transistors à film mince organiques, les transistors à émission de lumière ou électroluminescents organiques, les cellules solaires organiques, les cellules solaires sensibilisées par colorant organiques, les cellules solaires contenant de la pérovskite, les détecteurs optiques organiques, les photorécepteurs organiques, les dispositifs à extinction de champ organiques, les cellules électrochimiques à émission de lumière ou électroluminescentes, les diodes laser organiques et les dispositifs à émission de plasmons organiques.

3. Dispositif électronique selon la revendication 1 ou 2, **caractérisé en ce que** le composé de la formule (1) est utilisé en tant que matériau de matrice pour un émetteur fluorescent ou phosphorescent et/ou dans une couche de blocage de trous et/ou dans une couche de transport d'électrons et/ou dans une couche de blocage d'électrons ou une couche de blocage d'excitons et/ou dans une couche de transport de trous.

4. Dispositif électronique selon une ou plusieurs des revendications 1 à 3, **caractérisé en ce que** Y dans la formule (1) représente 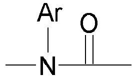 et Ar présente dans chaque cas, de manière indépendante les uns des autres, une signification qui a été donnée selon la revendication 1.

5. Dispositif électronique selon une ou plusieurs des revendications 1 à 3, **caractérisé en ce que** Y dans la formule (1) représente 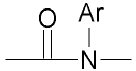 et Ar présente dans chaque cas, de manière indépendante les uns des autres, une signification qui a été donnée selon la revendication 1.

6. Dispositif électronique selon une ou plusieurs des revendications 1 à 3, **caractérisé en ce que** Y dans la formule (1) représente

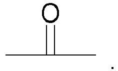

7. Dispositif électronique selon une ou plusieurs des revendications 1 à 6, **caractérisé en ce qu'**au moins une variable du groupe X₁, X₂, X₃ et X₄ représente N et les variables restantes représentent CR, et R présente dans chaque cas, de manière indépendante les uns des autres, l'une des significations qui ont été données selon la revendication 1.

8. Dispositif électronique selon une ou plusieurs des revendications 1 à 6, **caractérisé en ce que** les variables X₁, X₂, X₃ et X₄ représentent CR, et R présente dans chaque cas, de manière indépendante les uns des autres, l'une des significations qui ont été données selon la revendication 1.

9. Composés de la formule (42), 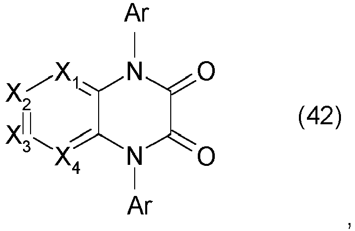 dans laquelle X₁, X₂, X₃ et X₄ représentent dans chaque cas, de manière indépendante les uns des autres, CR, où le substituant R de X₁ et/ou le substituant R de X₄ en association avec le N-Ar respectivement adjacent forme(nt) un système de cycle aliphatique, aromatique ou hétéroaromatique monocyclique ou polycyclique, lequel peut être substitué par un radical ou par plusieurs radicaux R¹,
et Ar, R, R¹ et R² présentent dans chaque cas, de manière indépendante les uns des autres, la signification qui suit :
Ar est pour chaque occurrence, de manière identique ou différente, un système de cycle aromatique ou hétéroaromatique qui comporte de 5 à 60 atomes de cycle aromatique, lequel peut être substitué par un radical ou par plusieurs radicaux R¹ ; Ar¹ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R¹ ; deux radicaux Ar¹ qui sont liés au même atome de N ou au même atome de P peuvent également être pontés l'un à l'autre au moyen d'une liaison simple ou d'un pont qui est sélectionné parmi N(R¹), C(R¹)₂ ou O ;
R est sélectionné pour chaque occurrence, de manière identique ou différente, parmi le groupe qui est constitué par H, D, F, Cl, Br, I, CN, CHO, NO₂, Si(R²)₃, B(OR²)₂, N(Ar¹)₂, N(R¹)₂, C(=O)Ar¹, C(=O)R¹, P(=O)(Ar¹)₂, S(=O)Ar¹, S(=O)(Ar¹)₂, CR²=CR²Ar¹, C≡CAr¹, OSO₂R¹ ; un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, où lesdits groupes hydrocarbone peuvent dans chaque cas être substitués par un radical ou par plusieurs radicaux R¹ et où un ou plusieurs groupe(s) CH₂ non adjacents peut/peuvent être remplacé(s) par O)(R¹), SO, SO₂, NR¹, O, S ou CONR¹ et où un ou plusieurs atome(s) de H peut/peuvent être remplacé(s) par D, F, Cl, Br, I, CN ou NO₂; un système de cycle aromatique ou hétéroaromatique qui comporte de 5 à 60 atomes de cycle aromatique, lequel peut dans chaque cas être substitué par un radical ou par plusieurs radicaux R¹, un groupe aryloxy ou hétéroaryloxy qui comporte de 5 à 60 atomes de cycle aromatique, lequel peut être substitué par un radical ou par plusieurs radicaux R¹,
un groupe aralkyle ou hétéroaralkyle qui comporte de 5 à 60 atomes de cycle aromatique, lequel peut être substitué par un radical ou par plusieurs radicaux R¹, ou une combinaison de ces systèmes, où deux substituants R adjacents ou plus peuvent former un système de cycle aliphatique, aromatique ou hétéroaromatique monocyclique ou polycyclique, lequel peut être substitué par un radical ou par plusieurs radicaux R¹ ou où le substituent R de X₁ et/ou le substituant R de X₄ en association avec le N-Ar respectivement adjacent peuvent/peut former un système de cycle aliphatique, aromatique ou hétéroaromatique monocyclique ou polycyclique, lequel peut être substitué par un radical ou par plusieurs radicaux R¹ ;
R¹ est, dans chaque cas de manière indépendante, sélectionné parmi le groupe qui est constitué par H, D, F, CN, un groupe alkyle en chaîne droite ou ramifié qui comporte de 1 à 20 atome(s) de C, un groupe alkényle en chaîne droite ou ramifié qui comporte de 2 à 20 atomes de C, un système de cycle aromatique ou hétéroaromatique qui comporte de 5 à 30 atomes de cycle aromatique, où un ou plusieurs atome(s) de H peut/peuvent être remplacé(s) par D, F, Cl, Br, I, CN ou un groupe alkyle en chaîne droite ou ramifié qui comporte de 1 à 10 atome(s) de C ou un groupe alkényle en chaîne droite ou ramifié qui comporte de 2 à 10 atomes de C, où deux substituants R¹ adjacents ou plus peuvent former un système de cycle aliphatique, aromatique ou hétéroaromatique mono- ou polycyclique l'un avec l'autre ou les uns avec les autres et R² est, dans chaque cas de manière indépendante, sélectionné parmi le groupe qui est constitué par H, D ou un radical hydrocarbone aliphatique, aromatique et/ou hétéroaromatique qui comporte de 1 à 20 atome(s) de C, où deux radicaux R² ou plus peuvent également former un système de cycle l'un avec l'autre ou les uns avec les autres.

10. Composés des formules (47) à (51) 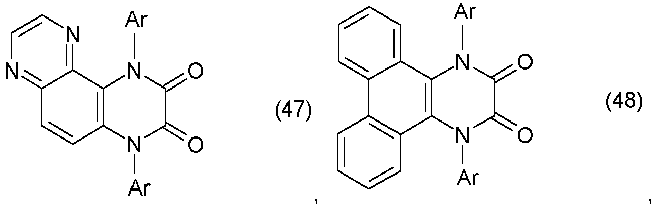 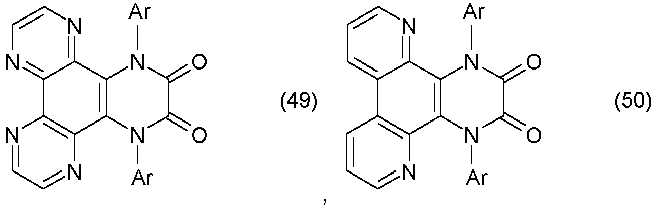 et 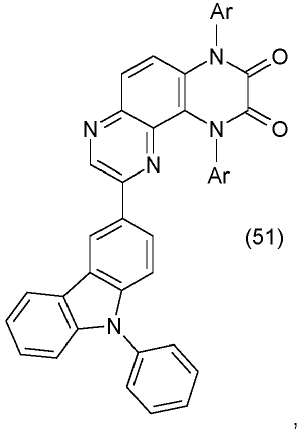 dans lesquelles Ar dans chaque cas, de manière indépendante les uns des autres, présente la signification qui suit :
Ar est pour chaque occurrence, de manière identique ou différente, un système de cycle aromatique ou hétéroaromatique qui comporte de 5 à 60 atomes de cycle aromatique, lequel peut être substitué par un radical ou par plusieurs radicaux R¹ ; et
R¹ est, dans chaque cas de manière indépendante, sélectionné parmi le groupe qui est constitué par H, D, F, CN, un groupe alkyle en chaîne droite ou ramifié qui comporte de 1 à 20 atome(s) de C, un groupe alkényle en chaîne droite ou ramifié qui comporte de 2 à 20 atomes de C, un système de cycle aromatique ou hétéroaromatique qui comporte de 5 à 30 atomes de cycle aromatique, où un ou plusieurs atome(s) de H peut/peuvent être remplacé(s) par D, F, Cl, Br, I, CN ou un groupe alkyle en chaîne droite ou ramifié qui comporte de 1 à 10 atome(s) de C ou un groupe alkényle en chaîne droite ou ramifié qui comporte de 2 à 10 atomes de C, où deux substituants R¹ adjacents ou plus peuvent former un système de cycle aliphatique, aromatique ou hétéroaromatique mono- ou polycyclique l'un avec l'autre ou les uns avec les autres.

11. Composés de la formule (56) 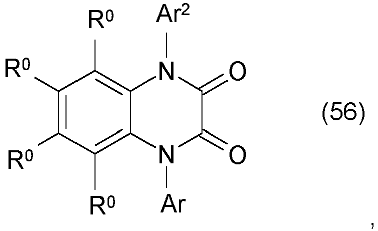 dans laquelle
Ar représente un système de cycle aromatique ou hétéroaromatique qui comporte de 5 à 60 atomes de cycle aromatique, lequel peut être substitué par un radical ou par plusieurs radicaux non aromatique(s) R¹,
Ar² représente un système de cycle aromatique qui comporte de 13 à 40 atomes de C ou un système de cycle hétéroaromatique qui comporte de 4 à 40 atomes de C, lequel peut être substitué par un radical ou plusieurs radicaux non aromatique(s) R¹,
R⁰ est sélectionné, pour chaque cas de manière indépendante les uns des autres, parmi le groupe qui est constitué par H, D, F, CN, un groupe alkyle en chaîne droite ou ramifié qui comporte de 1 à 20 atome(s) de C, un groupe alkényle en chaîne droite ou ramifié qui comporte de 2 à 20 atomes de C, un système de cycle aromatique qui comporte de 5 à 30 atomes de cycle aromatique, où un ou plusieurs atome(s) de H peut/peuvent être remplacé(s) par D, F, Cl, Br, I, CN ou un groupe alkyle en chaîne droite ou ramifié qui comporte de 1 à 10 atome(s) de C ou un groupe alkényle en chaîne droite ou ramifié qui comporte de 2 à 10 atomes de C, et
où R¹ et R présentent dans chaque cas, de manière indépendante l'un de l'autre, la signification qui suit :
R est sélectionné pour chaque occurrence, de manière identique ou différente, parmi le groupe qui est constitué par H, D, F, Cl, Br, I, CN, CHO, NO₂, Si(R²)₃, B(OR²)₂, N(Ar¹)₂, N(R¹)₂, C(=O)Ar¹, C(=O)R¹, P(=O)(Ar¹)₂, S(=O)Ar¹, S(=O)(Ar¹)₂, CR²=CR²Ar¹, C≡CAr¹, OSO₂R¹ ; un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, où lesdits groupes hydrocarbone peuvent dans chaque cas être substitués par un radical ou par plusieurs radicaux R¹ et où un ou plusieurs groupe(s) CH₂ non adjacents peut/peuvent être remplacé(s) par R¹C=CR¹, -C=C-, Si(R¹)₂, Ge(R¹)₂, Sn(R¹)₂, C=O, C=S, C=Se, C=NR¹, P(=O)(R¹), SO, SO₂, NR¹, O, S ou CONR¹ et où un ou plusieurs atome(s) de H peut/peuvent être remplacé(s) par D, F, Cl, Br, I, CN ou NO₂;
un système de cycle aromatique ou hétéroaromatique qui comporte de 5 à 60 atomes de cycle aromatique, lequel peut dans chaque cas être substitué par un radical ou par plusieurs radicaux R¹, un groupe aryloxy ou hétéroaryloxy qui comporte de 5 à 60 atomes de cycle aromatique, lequel peut être substitué par un radical ou par plusieurs radicaux R¹,
un groupe aralkyle ou hétéroaralkyle qui comporte de 5 à 60 atomes de cycle aromatique, lequel peut être substitué par un radical ou par plusieurs radicaux R¹, ou une combinaison de ces systèmes, où deux substituants R adjacents ou plus peuvent former un système de cycle aliphatique, aromatique ou hétéroaromatique monocyclique ou polycyclique, lequel peut être substitué par un radical ou par plusieurs radicaux R¹ ; R¹ est, dans chaque cas de manière indépendante, sélectionné parmi le groupe qui est constitué par H, D, F, CN, un groupe alkyle en chaîne droite ou ramifié qui comporte de 1 à 20 atome(s) de C, un groupe alkényle en chaîne droite ou ramifié qui comporte de 2 à 20 atomes de C, un système de cycle aromatique ou hétéroaromatique qui comporte de 5 à 30 atomes de cycle aromatique, où un ou plusieurs atome(s) de H peut/peuvent être remplacé(s) par D, F, Cl, Br, I, CN ou un groupe alkyle en chaîne droite ou ramifié qui comporte de 1 à 10 atome(s) de C ou un groupe alkényle en chaîne droite ou ramifié qui comporte de 2 à 10 atomes de C, où deux substituants R¹ adjacents ou plus peuvent former un système de cycle aliphatique, aromatique ou hétéroaromatique mono- ou polycyclique l'un avec l'autre ou les uns avec les autres et R² est, dans chaque cas de manière indépendante, sélectionné parmi le groupe qui est constitué par H, D ou un radical hydrocarbone aliphatique, aromatique et/ou hétéroaromatique qui comporte de 1 à 20 atome(s) de C, où deux radicaux R² ou plus peuvent également former un système de cycle l'un avec l'autre ou les uns avec les autres.

12. Composés de la formule (57), 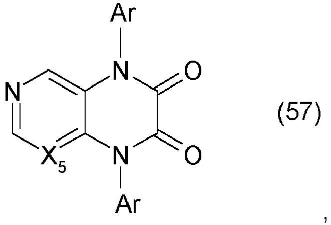 dans laquelle
X₅ correspond à CR ou N et Ar et R présentent la signification qui suit :
Ar est pour chaque occurrence, de manière identique ou différente, un système de cycle aromatique ou hétéroaromatique qui comporte de 5 à 60 atomes de cycle aromatique, lequel peut être substitué par un radical ou par plusieurs radicaux R¹ ; Ar¹ est pour chaque occurrence, de manière identique ou différente, un système de cycle aromatique ou hétéroaromatique qui comporte de 5 à 30 atomes de cycle aromatique, lequel peut être substitué par un radical ou par plusieurs radicaux R¹ ; deux radicaux Ar¹ qui sont liés au même atome de N ou au même atome de P peuvent également être pontés l'un à l'autre au moyen d'une liaison simple ou d'un pont qui est sélectionné parmi N(R¹), C(R¹)₂ ou O ;
R est sélectionné pour chaque occurrence, de manière identique ou différente, parmi le groupe qui est constitué par H, D, F, Cl, Br, I, CN, CHO, NO₂, Si(R²)₃, B(OR²)₂, N(Ar¹)₂, N(R¹)₂, C(=O)Ar¹, C(=O)R¹, P(=O)(Ar¹)₂, S(=O)Ar¹, S(=O)(Ar¹)₂, CR²=CR²Ar¹, C≡CAr¹, OSO₂R¹ ; un groupe alkyle, alcoxy ou thioalkyle en chaîne droite qui comporte de 1 à 40 atome(s) de C ou un groupe alkyle, alcoxy ou thioalkyle ramifié ou cyclique qui comporte de 3 à 40 atomes de C ou un groupe alkényle ou alkynyle qui comporte de 2 à 40 atomes de C, où lesdits groupes hydrocarbone peuvent dans chaque cas être substitués par un radical ou par plusieurs radicaux R¹ et où un ou plusieurs groupe(s) CH₂ non adjacents peut/peuvent être remplacé(s) par R¹C=CR¹, -C=C-, Si(R¹)₂, Ge(R¹)₂, Sn(R¹)₂, C=O, C=S, C=Se, C=NR¹, P(=O)(R¹), SO, SO₂, NR¹, O, S ou CONR¹ et où un ou plusieurs atome(s) de H peut/peuvent être remplacé(s) par D, F, Cl, Br, I, CN ou NO₂;
un système de cycle aromatique ou hétéroaromatique qui comporte de 5 à 60 atomes de cycle aromatique, lequel peut dans chaque cas être substitué par un radical ou par plusieurs radicaux R¹, un groupe aryloxy ou hétéroaryloxy qui comporte de 5 à 60 atomes de cycle aromatique, lequel peut être substitué par un radical ou par plusieurs radicaux R¹,
un groupe aralkyle ou hétéroaralkyle qui comporte de 5 à 60 atomes de cycle aromatique, lequel peut être substitué par un radical ou par plusieurs radicaux R¹, ou une combinaison de ces systèmes, où deux substituants R adjacents ou plus peuvent former un système de cycle aliphatique, aromatique ou hétéroaromatique monocyclique ou polycyclique, lequel peut être substitué par un radical ou par plusieurs radicaux R¹ ou où le substituant R de X₅ en association avec le N-Ar adjacent peut former un système de cycle aliphatique, aromatique ou hétéroaromatique monocyclique ou polycyclique, lequel peut être substitué par un radical ou par plusieurs radicaux R¹ ;
R¹ est, dans chaque cas de manière indépendante, sélectionné parmi le groupe qui est constitué par H, D, F, CN, un groupe alkyle en chaîne droite ou ramifié qui comporte de 1 à 20 atome(s) de C, un groupe alkényle en chaîne droite ou ramifié qui comporte de 2 à 20 atomes de C, un système de cycle aromatique ou hétéroaromatique qui comporte de 5 à 30 atomes de cycle aromatique, où un ou plusieurs atome(s) de H peut/peuvent être remplacé(s) par D, F, Cl, Br, I, CN ou un groupe alkyle en chaîne droite ou ramifié qui comporte de 1 à 10 atome(s) de C ou un groupe alkényle en chaîne droite ou ramifié qui comporte de 2 à 10 atomes de C, où deux substituants R¹ adjacents ou plus peuvent former un système de cycle aliphatique, aromatique ou hétéroaromatique mono- ou polycyclique l'un avec l'autre ou les uns avec les autres et R² est, dans chaque cas de manière indépendante, sélectionné parmi le groupe qui est constitué par H, D ou un radical hydrocarbone aliphatique, aromatique et/ou hétéroaromatique qui comporte de 1 à 20 atome(s) de C, où deux radicaux R² ou plus peuvent également former un système de cycle l'un avec l'autre ou les uns avec les autres.

13. Formulations comprenant au moins un composé selon une ou plusieurs des revendications 9 à 12.

14. Utilisation de composés selon une ou plusieurs des revendications 9 à 12 dans les dispositifs électroniques.
